# EUROPEAN PATENT SPECIFICATION

(11) **EP 1 799 823 B1**
(45) Date of publication and mention of the grant of the patent: **25.11.2009**
(21) Application number: 05850658.5
(22) Date of filing: 19.09.2005
(51) Int. Cl.: C12N 15/10, C12N 15/01, C12N 15/53, C12N 9/06, C40B 10/00

(54) **METHOD FOR MODULATING THE EVOLUTION OF A POLYPEPTIDE ENCODED BY A NUCLEIC ACID SEQUENCE**
VERFAHREN ZUR MODULATION DER ENTWICKLUNG EINES DURCH EINE NUKLEINSÄURESEQUENZ KODIERTEN POLYPEPTIDS
PROCÉDÉ PERMETTANT DE MODULER L'ÉVOLUTION D'UN POLYPEPTIDE CODÉ PAR UNE SÉQUENCE D'ACIDES NUCLÉIQUES

(30) Priority: 17.09.2004 US 610597 P
(43) Date of publication of application: 27.06.2007
(73) Proprietor: INSTITUT PASTEUR, 75015 Paris (FR)
(72) Inventor: MAZEL, Didier, F-92150 Suresnes (FR); CAMBRAY, Guillaume, 78690 Les Essarts le Roi (FR)
(74) Representative: Texier, Christian
(86) International application number: PCT/IB2005/003317
(87) International publication number: WO 2006/046132

(56) References cited:
- WO-A-02/16944
- WO-A-97/33988
- WO-A-02/098443
- PLOTKIN JOSHUA B ET AL: "Codon bias and frequency-dependent selection on the hemagglutinin epitopes of influenza A virus." PROCEEDINGS OF THE NATIONAL ACADEMY OF SCIENCES OF THE UNITED STATES OF AMERICA, vol. 100, no. 12, 10 June 2003 (2003-06-10), pages 7152-7157, XP002382909 ISSN: 0027-8424
- SANO GEN-ICHIRO ET AL: "Purification and characterization of dihydrofolate reductase of Plasmodium falciparum expressed by a synthetic gene in Escherichia coli" MOLECULAR AND BIOCHEMICAL PARASITOLOGY, vol. 63, no. 2, 1994, pages 265-273, XP002382910 ISSN: 0166-6851
- FLENSBURG J ET AL: "MASSIVE OVERPRODUCTION OF DIHYDROFOLATE REDUCTASE IN BACTERIA AS A RESPONSE TO THE USE OF TRIMETHOPRIM" EUROPEAN JOURNAL OF BIOCHEMISTRY, vol. 162, no. 3, 1987, pages 473-476, XP002382911 ISSN: 0014-2956
- PLOTKIN JOSHUA B ET AL: "Detecting selection using a single genome sequence of M. tuberculosis and P. falciparum." NATURE. 29 APR 2004, vol. 428, no. 6986, 29 April 2004 (2004-04-29), pages 942-945, XP002382912 ISSN: 1476-4687 cited in the application
- PLOTKIN JOSHUA B ET AL: "Tissue-specific codon usage and the expression of human genes." PROCEEDINGS OF THE NATIONAL ACADEMY OF SCIENCES OF THE UNITED STATES OF AMERICA. 24 AUG 2004, vol. 101, no. 34, 24 August 2004 (2004-08-24), pages 12588-12591, XP002382913 ISSN: 0027-8424
- KADLEC KRISTINA ET AL: "Molecular basis of resistance to trimethoprim, chloramphenicol and sulphonamides in Bordetella bronchiseptica." THE JOURNAL OF ANTIMICROBIAL CHEMOTHERAPY. SEP 2005, vol. 56, no. 3, September 2005 (2005-09), pages 485-490, XP002382914 ISSN: 0305-7453

## Description

### BACKGROUND OF THE INVENTION

### Field of the Invention

A method for making a mutant polypeptide by analyzing codon usage within a gene and selecting a synonymous nucleotide sequence with a higher, lower or different capacity to mutate. A computer-implemented method for analyzing and selecting nucleotide sequences with an altered ability to mutate. Mutate is here defined at the level of amino-acid sequence. Mutation then does not refer to nucleotide as usual but to amino-acid changes. Consequently silent or neutral mutations of a codon must not to be considered.

### Description of the Related Art

The genetic code is known. This code is redundant. That is, for most polypeptides, there are many different nucleic acid sequences that encode the same amino acid sequence forming a polypeptide or protein.

The table below shows the genetic code and which codons encode which amino acids. The codons UAA, UGA and UAG are stop codons in the standard genetic code and do not ordinarily encode an amino acid. The table below shows each codon and the amino acid it encodes. For example: UUU encodes phenylalanine (Phe, F) and UCU encodes serine (Ser, S).

| | | **Second Position of Codon** | | | | | |
|---|---|---|---|---|---|---|---|
| | | **U** | **C** | **A** | **G** | | |
| **F** **i** **r** **s** **t** **P** **o** **s** **i** **t** **i** **o** **n** | **U** | UUU | UCU | UAU | UGU | **U** **C** **A** **G** | **T** **h** **i** **r** **d** **P** **o** **s** **i** **t** **i** **o** **n** |
| | | Phe | Ser | Tyr | Cys | | |
| | | [F] | [S] | [Y] | [C] | | |
| | | | | | | | |
| | | UUC | UCC | UAC | UGC | | |
| | | Phe | Ser | Tyr | Cys | | |
| | | [F] | [S] | [Y] | [C] | | |
| | | | | | | | |
| | | UUA | UCA | UAA | UGA | | |
| | | Leu | Ser | *Ter* | *Ter* | | |
| | | [L] | [S] | [end] | [end] | | |
| | | | | | | | |
| | | UUG | UCG | UAG | UGG | | |
| | | Leu | Ser | *Ter* | Trp | | |
| | | [L] | [S] | [end] | [W] | | |
| | **C** | CUU | CCU | CAU | CGU | **U** **C** **A** **G** | |
| | | Leu | Pro | His | Arg | | |
| | | [L] | [P] | [H] | [R] | | |
| | | | | | | | |
| | | CUC | CCC | CAC | CGC | | |
| | | Leu | Pro | His | Arg | | |
| | | [L] | [P] | [H] | [R] | | |
| | | | | | | | |
| | | CUA | CCA | CAA | CGA | | |
| | | Leu | Pro | Gln | Arg | | |
| | | [L] | [P] | [Q] | [R] | | |
| | | | | | | | |
| | | CUG | CCG | CAG | CGG | | |
| | | Leu | Pro | Gln | Arg | | |
| | | [L] | [P] | [Q] | [R] | | |
| | **A** | AUU | ACU | AAU | AGU | **U** **C** **A** **G** | |
| | | Ile [I] | Thr [T] | Asn [N] | Ser [S] | | |
| | | | | | | | |
| | | AUC | ACC | AAC | AGC | | |
| | | Ile | Thr | Asn | Ser | | |
| | | [I] | [T] | [N] | [S] | | |
| | | | | | | | |
| | | AUA | ACA | AAA | AGA | | |
| | | Ile | Thr | Lys | Arg | | |
| | | [I] | [T] | [K] | [R] | | |
| | | | | | | | |
| | | AUG | ACG | AAG | AGG | | |
| | | Met | Thr | Lys | Arg | | |
| | | [M] | [T] | [K] | [R] | | |
| | **G** | GUU | GCU | GAU | GGU | **U** **C** **A** **G** | |
| | | Val | Ala | Asp | Gly | | |
| | | [V] | [A] | [D] | [G] | | |
| | | | | | | | |
| | | GUC | GCC | GAC | GGC | | |
| | | Val | Ala | Asp | Gly | | |
| | | [V] | [A] | [D] | [G] | | |
| | | | | | | | |
| | | GUA | GCA | GAA | GGA | | |
| | | Val | Ala | Glu | Gly | | |
| | | [V] | [A] | [E] | [G] | | |
| | | | | | | | |
| | | GUG | GCG | GAG | GGG | | |
| | | Val | Ala | Glu | Gly | | |
| | | [V] | [A] | [E] | [G] | | |

As shown above, different codons may encode the same amino acid. For example, in the standard genetic code there are six codons which encode leucine (Leu, L). These codons are known as synonymous codons, because they each encode the same amino acid. While synonymous codons encode the same amino acid residue, each organism has a preference for particular synonymous codons over others. This preference is known as codon bias. For example, according to Source: www.tigr.org *Escherichia coli*, strain K-12 exhibits the following codon usage:

**Escherichia coli K12 [gbbct]: 5095 CDS's (1609357 codons)**

| [AA] | [codon] | [Triplet Frequency for corresponding AA] | | | | |
|---|---|---|---|---|---|---|
| Ala | GCA | 21.32% | | Leu | CTG | 49.52% |
| Ala | GCT | 16.14% | | Leu | TTG | 12.88% |
| Ala | GCG | 35.56% | | Leu | CTC | 10.44% |
| Ala | GCC | 26.98% | | Leu | CTA | 3.68% |
| | | | | Leu | TTA | 13.10% |
| Arg | CGG | 9.85% | | Leu | CTT | 10.38% |
| Arg | CGA | 6.47% | | | | |
| Arg | AGA | 3.85% | | Lys | AAA | 76.51% |
| Arg | CGT | 37.78% | | Lys | AAG | 23.49% |
| Arg | AGG | 2.25% | | | | |
| Arg | CGC | 39.80% | | Met | ATG | 100.00% |
| | | | | | | |
| Asn | AAC | 54.88% | | Phe | TTC | 42.58% |
| Asn | AAT | 45.12% | | Phe | TTT | 57.42% |
| | | | | Pro | CCG | 52.50% |
| Asp | GAT | 62.78% | | Pro | CCC | 12.47% |
| Asp | GAC | 37.22% | | Pro | CCA | 19.11% |
| | | | | Pro | CCT | 15.92% |
| Cys | TGT | 44.43% | | | | |
| Cys | TGC | 55.57% | | Ser | TCA | 12.38% |
| | | | | Ser | TCC | 14.84% |
| End | TAA | 63.08% | | Ser | AGT | 15.15% |
| End | TAG | 7.61% | | Ser | TCT | 14.55% |
| End | TGA | 29.31% | | Ser | TCG | 15.40% |
| | | | | Ser | AGC | 27.67% |
| Gln | CAA | 34.77% | | | | |
| Gln | CAG | 65.23% | | Thr | ACC | 43.39% |
| | | | | Thr | ACA | 13.19% |
| | | | | Thr | ACT | 16.64% |
| Glu | GAG | 31.14% | | Thr | ACG | 26.78% |
| Glu | GAA | 68.86% | | | | |
| | | | | Trp | TGG | 100.00% |
| Gly | GGG | 15.11% | | | | |
| Gly | GGA | 10.90% | | Tyr | TAT | 56.99% |
| Gly | GGC | 40.33% | | Tyr | TAC | 43.01% |
| Gly | GGT | 33.66% | | | | |
| | | | | Val | GTC | 21.54% |
| His | CAT | 57.11% | | Val | GTG | 37.28% |
| His | CAC | 42.89% | | Val | GTT | 25.80% |
| | | | | Val | GTA | 15.38% |
| Ile | ATA | 7.33% | | | | |
| Ile | ATT | 50.71% | | | | |
| Ile | ATC | 41.96% | | | | |

In the same manner codon (triplet) frequency for corresponding amino acids for humans or other organisms can be easily obtained from their correspondent codon bias.

A native gene will generally tend to exhibit the codon usage or preference of the particular organism from which it is derived. However, the codons of a native or original gene sequence are limited to the sequence space that they can explore and then to the amino acid they can reach. Thus, said original codons are not necessarily the codons with the highest or broadest capacity to mutate.

By "sequence space" of a defined nucleotide sequence, we intend all possible nucleotide sequences derived by a single point mutation of one single codon of the original sequence.

As disclosed below, however, not all codons encoding the same amino acid residue are equivalent. Some synonymous codons allow for a greater frequency or range of mutation than others. The present invention is based in part on replacing the codons in a native protein-coding sequence with synonymous codons with a higher, broader or different capacity to mutate.

Codon usage and bias has been studied for frequency-dependent selection of epitopes in pathogens such as influenza virus, Plotkin et al., Proc Natl Acad Sci U S A. 2003 June 10; 100(12):7152-7. Epub 2003 May 14. Codon volatility has been used to measure selective pressures on proteins, Plotkin et al. Nature vol 428 29 April 2004.

Plotkin Joshua B et al. PNAS vol. 100, no. 12, 10 June 2003, pages 7152-7157, analysed the sequence evolution of three influenza A genes over the past two decades. They studied codon usage as a discriminating signature of gene- and even residue-specific diversifying and purifying selection. Non-random codon choice can increase or decrease the effective local substitution rate. Plotkin Joshua B et al. demonstrated that the codons of hemagglutinin, particularly those in the antibody-combining regions, are significantly biased toward substitutional point mutations relative to the codons of other influenza virus genes. Plotkin et al. stated that both AGG and CGG encode arginine, but using the Hamilton matric v-H-(AGG)=7, whereas v·H·(CGG)=5. Hence, with a constant per-base mutation rate, AGG is 1.4 times more likely to undergo a substitution than in CGG. Thus, even for a fixed amino acid sequence, codon usage may bias a sequence toward, or away from, future substitutional changes.

WO 02/098443 discloses a process for determining the sequence of a modified mRNA, using a computer program that modifies the nucleotide sequence of an arbitrary mRNA in such a way as to maximise the G/C content of the nucleic acid, and maximise the presence of codons recognized by abundant tRNAs present in a particular cell(s). The computer program is based on an understanding of the genetic code and exploits the degenerative nature of the genetic code. By this means a modified mRNA having desirable properties is obtained, wherein the amino acid sequence encoded by the modified mRNA is identical to that of the unmodified mRNA sequence. Moreover, an RNA construct with a sequence of the lac-Z gene from E. coli optimised with regard to stabilisation and translational efficiency was produced with the aid of said computer program. A G/C content of 69% (compared to the wild type sequence of 51%) was achieved in this manner. Through the synthesis of overlapping oligonucleotides that comprise the modified sequence, the optimised sequence was produced. The modified lacZ sequence was incorporated into the plasmid pT7Ts and was propagated in bacteria and purified. Furthermore, the gene for the influenza matrix protein was optimised with the aid of said computer program. The G/C-rich sequence variant was thereby formed.

WO 02/16944 describes a method to prepare a synthetic nucleic acid molecule comprising an open reading frame, comprising altering greater than 25% of the codons in the synthetic nucleic acid sequence which has a decreased number of transcription regulatory sequences to yield a further synthetic nucleic acid molecule, wherein the codons which are altered do not result in an increased number of transcription regulatory sequences, wherein the further synthetic nucleic acid molecule encodes a polypeptide with at least 85%amino acid sequence identity to the polypeptide encoded by the parent nucleic acid sequence, wherein the codons which are altered encode the same amino acid as the corresponding codons in the parent nucleic acid sequence.

Sano Gen-Ichiro et al. Molecular and Biochemical Parasitology, 63, no. 2, 1994, pages 265-273, disclose the design and construction of a gene encoding Plasmodium falciparum dihydrofolate reductase by changing the codon usage, based on the hypothesis that the P. falciparum sequence contains impediments for its expression in E. coli. Flensburg J et al. European Journal of Biochemistry, vol. 162, no. 3, 1987, pages 473-476, describe the massive overproduction of dihydrofolate reductase in bacteria as a response to the use of trimethoprim. The structural gene for the overproduced dihydrofolate reductase was found to be identical to that of E. coli K12, with nine exceptions, of which seven resulted in synonymous codon usage. Of the seven base changes that do not alter the amino acid sequence of the overproduced enzyme in comparison to that of E. coli K12, five resulted in codons that are more commonly in highly expressed proteins and which correspond to tRNA species that are more abundant in the cell. WO 97/33988 describes human dihydrofolate reductase (DHFR) mutant differs from the wild type enzyme by having an amino acid with a bulkier side chain than leucine at position 22, and an amino acid with a less bulky but more hydrophilic side chain than phenylalanine at position 31. Codon usage and bias have been used to passively analyze known gene sequences or construct phylogenetic trees, in order to analyze past history of the sequence. However, methods of using such information to engineer new nucleotide sequences having a modified capacity to mutate have not previously been suggested. In other words, manipulation of a given gene's codon usage has never been proposed to alter its subsequent evolution.

The present invention is based on the discovery that by replacing one or more codons in a native or original polypeptide-encoding nucleic acid sequence (gene) by a synonymous codon, the subsequent evolution of the polypeptide-encoding nucleic acid sequence can be controlled. Indeed some amino acids that were unreachable by way of a single point mutation can be reached from an alternative synonymous sequence. Hence, the method renders certain mutations evolutionary accessible. Some protein mutants, which were virtually unobtainable (evolutionarily inaccessible) using the wild-type or original nucleic acid sequence, become possible when an appropriate synonymous nucleic acid sequence is used.

The method of the present invention can be used to increase, decrease, stabilize or change the ability of a native gene to mutate. Increasing the mutational frequency or altering the range of mutations that can occur in a polypeptide-encoding nucleic acid sequence is beneficial when further selecting for functional variants of the protein encoded by the original or native nucleic sequence.

The method may also be used to reduce the mutational frequency of a nucleic acid sequence or gene, when a high mutation rate is undesirable, such as when a sequence is used to encode biologically useful proteins or vaccines.

### BRIEF SUMMARY OF THE INVENTION

One aspect of the invention is a method for making a mutant polypeptide comprising:
- identifying an original nucleotide sequence which encodes a polypeptide;
- determining at least one synonymous nucleotide sequence encoding the same protein, which comprises at least one synonymous codon different from the corresponding codon in the original nucleotide sequence.
- synthesizing said synonymous polynucleotide sequence,
- transforming said synonymous polynucleotide sequence into a host cell,
- culturing said host cell under culture conditions specifically designed to positively link features of interest to cell fitness, e.g. in the presence of chemicals or antibiotics, thereby introducing at least one point mutation into said synonymous nucleotide sequence,
- isolating a mutant cell expressing a mutant polypeptide, and
- recovering said mutant polypeptide.

Said method can comprise the selection of a synonymous nucleic acid sequence which encodes the same polypeptide as an original (e.g., native, wild-type) gene or nucleic acid sequence, but which has an altered capacity to mutate. Selection may be based on increasing, diversifying, or decreasing the mutation rate of the synonymous gene sequence. As explained below, this method may be used to select a synonymous nucleic acid sequence exhibiting the maximal relative evolutionary power or, alternatively, a sequence having the maximal intrinsic evolutionary power.

A sequence may also be selected based on its ability to undergo particular mutations, such as increasing or decreasing the mutation rate of one or more codons to mutant codons encoding a particular amino acid.

A second aspect of the invention is computer-implemented method for selecting a nucleotide sequence which is synonymous to a known polynucleotide sequence, using the Evolutionary Lanscape Painter program (ELP), and comprising:
obtaining an original nucleotide sequence which encodes a polypeptide ;
determining synonymous nucleotides for each codon of the sequence;
determining the intrinsic evolutionary power of each synonymous codon;
selecting a synonymous nucleotide sequence having a higher or lower intrinsic evolutionary power than the original nucleotide sequence.

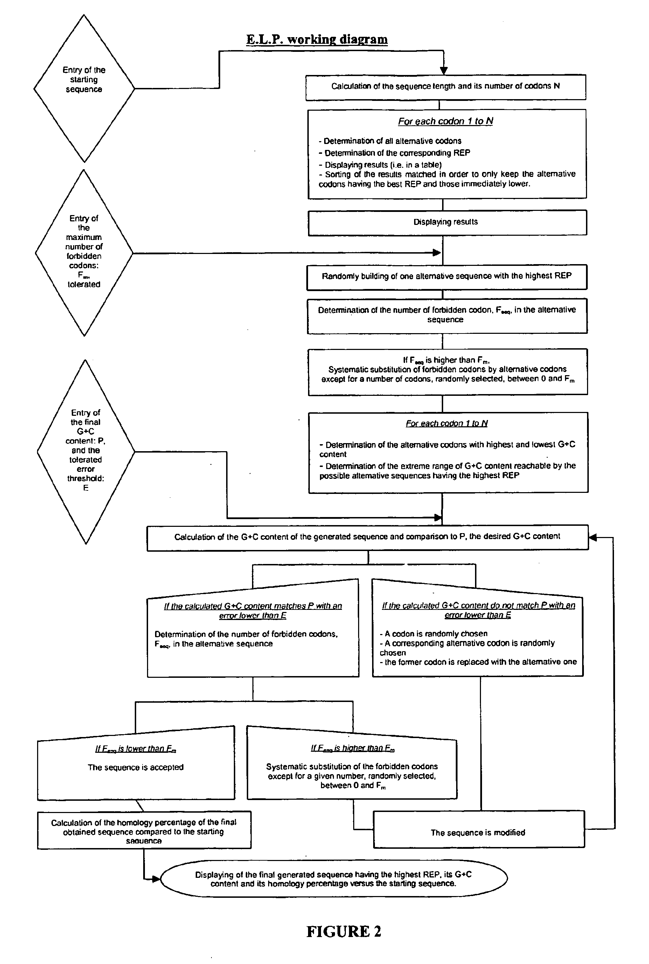

One example of computer software suitable for this purpose is the ELP software as described for example in Fig. 2.

Other aspects of the invention will be apparent from the following disclosure.

### BRIEF DESCRIPTION OF THE DRAWINGS

Figure 1 shows the evolutive (evolutionary) landscape for the UUG and CUC codons.
Figure 2 shows an ELP (Evolutionary Landscape Painter) working diagram.
   Index of abbreviation used:
   - E =: error threshold tolerated for G+C content
   - Fm =: maximum number of forbidden codons tolerated in the sequence
   - Fseq =: number of forbidden codons in the generated sequence
   - N =: number of codons in the sequence
   - P =: final G+C content
   - REP =: Relative Evolutionary Potential
   See the E.L.P. readme for a definition of the forbidden codons.

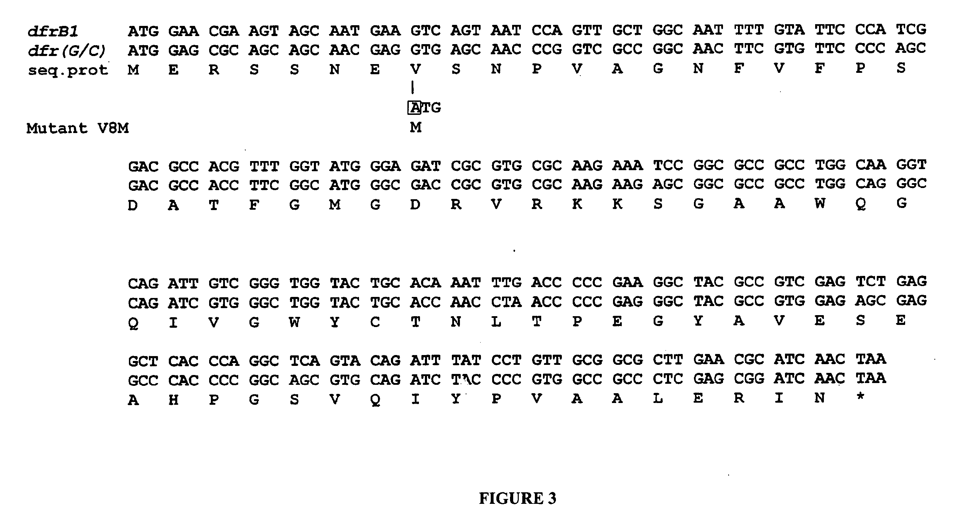

Figure 3 depicts the *dfrB1* wild type (low GC content) and *dfrB1_{GC}* (high GC content) nucleic acid sequences. Both nucleic acid sequences encode the same amino acid sequence (blue). Modifications to the original *dfrB1* nucleotide sequence are shown in red.

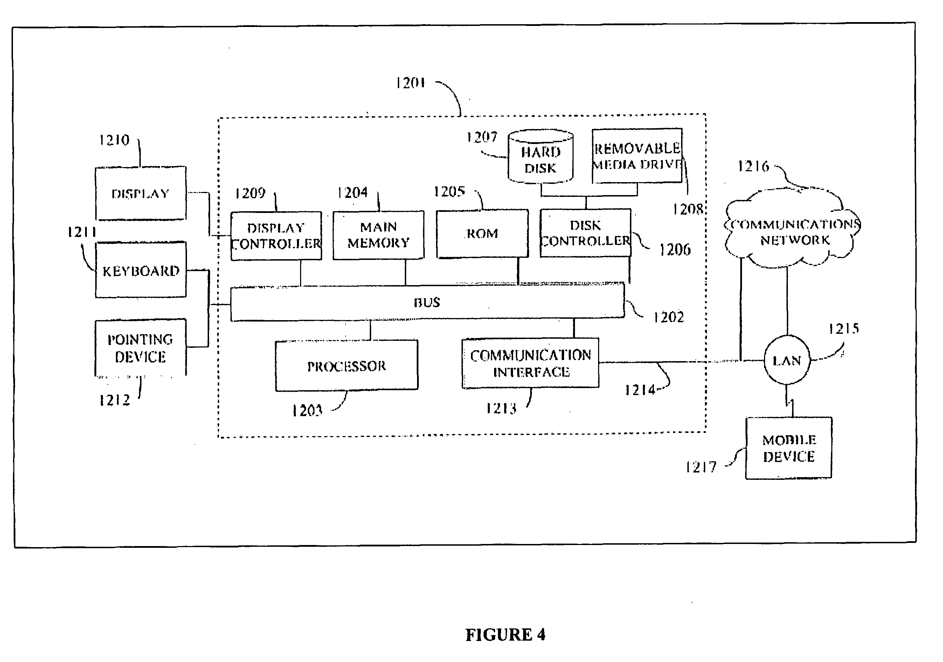

Figure 4 illustrates a computer system 1201 upon which an embodiment of the present invention may be implemented.

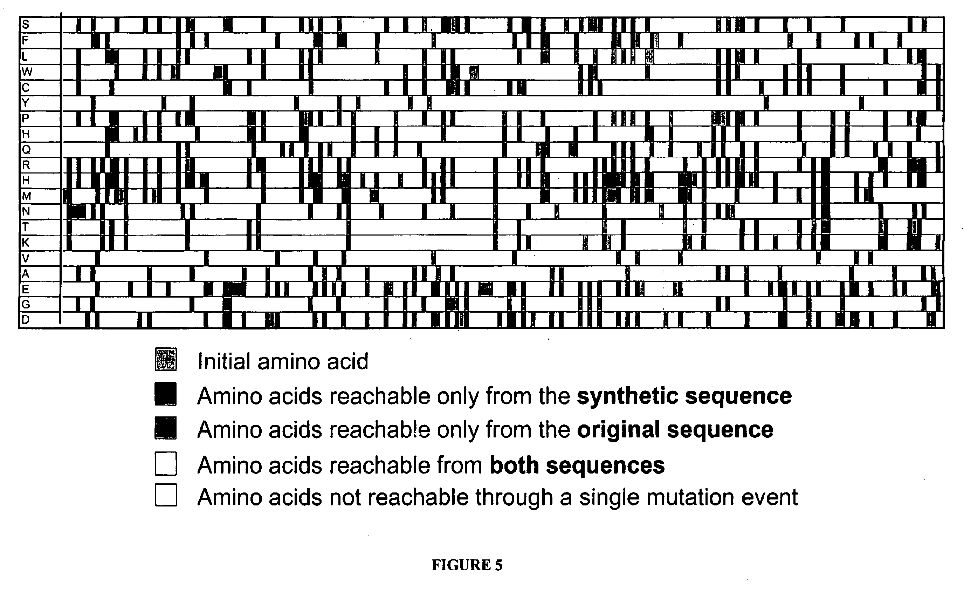

Fig. 5 (color) depicts an evolutionary landscape. Original amino acid residues are shown in pink. Residues accessible by mutation of the original (red), synthetic (blue), both original and synthetic (yellow) or not accessible by a single mutation event (white) are shown.

### DETAILED DESCRIPTION OF THE INVENTION

An original nucleic acid sequence may be isolated and sequenced based on methods well-known in the art as described, for example, by Current Protocols in Molecular Biology, (April, 2004, through supplement 66), see e.g., Chapter 2 "Preparation and Analysis of DNA" and Chapter 7 "DNA Sequencing". Alternatively, the nucleotide sequence for a particular gene and the actual or deduced amino acid sequence encoded by that gene may have already been published or be available from a sequence database. Numerous nucleotide sequences of both prokaryotic and eukaryotic organisms are known. For example, GenBank^{®} is the NIH genetic sequence database, an annotated collection of all publicly available DNA sequences (Nucleic Acids Research 2004 Jan 1;32(1):23-6). There are approximately 37,893,844,733 bases in 32,549,400 sequence records as of February 2004. Other sequence databases are Current Protocols in Molecular Biology (April, 2004, through supplement 66), Chapter 19 "Informatics for Molecular Biologists".

Once a nucleotide sequence of interest has been identified, if the corresponding amino acid sequence is not already known, it may be easily deduced based on the structure of the nucleotide sequence referring to the genetic code. Computer programs suitable for this purpose are well-known and are Current Protocols in Molecular Biology (April, 2004, through supplement 66), Chapter 19 "Informatics for Molecular Biologists". Alternatively the ELP program can be used.

As discussed above, an original nucleotide sequence will show a particular codon usage and codon bias generally corresponding to the organism from which it was derived. The original or wild-type nucleotide sequence does not necessarily have a high capacity to accumulate point mutations which change the identity of the amino acid sequence it encodes. However, the evolutionary ability of this native sequence may be optimized by the method of the present invention.

There are numerous synonymous nucleotide sequences encoding most polypeptides and proteins. Each particular synonymous nucleotide sequence has a particular capacity to accumulate point mutations in its codons. The present inventors have discovered a method for identifying and selecting the synonymous nucleotide sequences with a higher, lower, or simply different, capacity to mutate. For example, point mutations sustained by these engineered synonymous polynucleotide sequences provide a wider range of polypeptide mutants than would the unmodified native sequence.

Each synonymous nucleotide sequence has a potential mutation frequency based on the identity of the specific codon used to encode amino-acid at each codon position. Point mutations may be made to some synonymous codons without affecting the amino acid encoded by that codon. For example, a point mutation of the third nucleotide of the CUU leucine codon will have not affect the amino acid encoded by the mutant because CUU, CUC, CUG and CUA all encode leucine. On the other hand, other point mutations, such as to nucleotides 1 and 2 of the CUU leucine codon will cause the mutant codon to encode a different amino acid than leucine. Depending on the identity of the particular leucine codon, single point mutations will allow the resulting mutant codon to encode a range of different amino acids.

The **evolutionary landscape** (evolutive landscape, EL) of a particular codon refers to all the different amino acids accessible by a single point mutation of the original codon. Since different synonymous codons may have different evolutionary landscapes, each codon has a particular mutational capacity and frequency. For example, a single base mutation of leucine codon UUG could alter this codon to a codon for Phe (UUU, UUC), Leu (UUA, CUG), Met (AUG), Val (GUG), Ser (UCG), or Trp (UGG). The evolutionary landscape of the UUG codon would encompass Phe, Leu, Met, Val, Ser and Trp. Similarly, the evolutionary landscape of the adjacent UUA (Leu codon) would encompass Phe, Leu, Ile, Val, and Ser. The stop codons (UAA, UGA and UAG) are not considered as part of the evolutionary landscape because they rather stand as an evolutionary dead end.

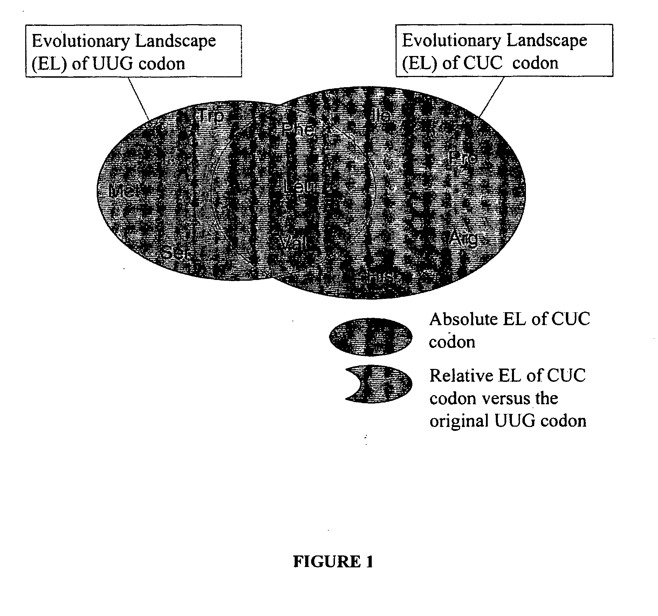

The **"intrinsic evolutionary power"** (IEP) of a codon is defined as the whole number of amino acids present in the evolutionary landscape of the considered codon, that is, it is equal to the cardinal number of this set of accessible amino acids. For the UUG codon the AEL is 6 (Phe, Leu, Val, Met, Ser and Trp). For the CUC codon the AEL is 7 (Phe, Leu, Val, His, Arg, Pro, Ile)--see Fig. 1 The intrinsic evolutionary power of the UUG (Leu) codon described above is six (6), because a single base mutation in this codon would allow the mutated codon to encode any one of six different amino acids. The intrinsic evolutionary power of the adjacent UUA (Leu) codon is five (5).

The **"relative evolutionary power"** (REP) of a codon is defined as the number of amino acids that are part of the evolutionary landscape of the alternative codon but do not form part of the evolutionary landscape of the original codon, that is, it is equal to the cardinal number IEP minus the cardinal number of the intersection between the evolutionary landscapes of the original codon and the considered codon. This intersection represents the amino acids which are part of the landscapes of both the original codon and the considered codon, in Fig. 1 these amino acids are Phe, Leu and Val.

The REP of the CUC codon would thus be +4, because a single point mutation of the CUC codon could cause it to encode four amino acids (Ile, Pro, Arg, His) not encodable by a single point mutation of the UUC codon.

The evolutionary landscape (EL) of a codon is the number of different amino acids that said codon could encode if it sustained a point mutation to a single base. For example, the evolutionary landscapes of the original codon UUG and alternates codons UUA, CUU, CUC, CUA and CUG encoding Leu are shown below.

| **Codon** | **AA** | **AA** | **AA** | **AA** | **AA** | **AA** | **AA** | **AA** | **AA** | **AA** | **AA** |
|---|---|---|---|---|---|---|---|---|---|---|---|
| **UUA** | Leu | Ser | Ile | | | | | | | Val | Phe |
| **UUG** | Leu | Ser | | Trp | | Met | | | | Val | Phe |
| **CUU** | Leu | | Ile | | | | Pro | His | Arg | Val | Phe |
| **CUC** | Leu | | Ile | | | | Pro | His | Arg | Val | Phe |
| **CUA** | Leu | | Ile | | Glu | | Pro | | Arg | Val | |
| **CUG** | Leu | | | | Glu | Met | Pro | | Arg | Val | |

The intrinsic evolutionary power (IEP) is the number of amino acids within the evolutionary landscape of a codon, e.g., for UAA there are five amino acids within the evolutionary landscape shown in the table above (Leu, Ser, Ile, Val and Phe).

The relative evolutionary power (REP) is the number of amino acids in the evolutionary landscape of a substitute codon that are not part of the evolutionary landscape of the original codon. If the codon in the original polynucleotide sequence is UUG, then the relative evolutionary power of the other five leucine codons compared to UUG is:

| ***UUG (Native codon)*** | **REP** | **IEP** |
|---|---|---|
| **UUA** | +1 | 5 |
| **UUG** | 0 | 6 |
| **CUU** | +4 | 7 |
| **CUC** | +4 | 7 |
| **CUA** | +4 | 6 |
| **CUG** | +3 | 6 |

The algorithm developed by the inventors allows selection of the codons having the highest relative evolutionary power. The proposed method allows the selection of mutant codons that would need at least two mutations to be selected naturally. It thus modify the evolutionary landscape at a given codon position encoding a particular amino acid. Indeed, for an original UUA codon to mutate to a Met codon (AUG) it must undergo two mutations, i.e., UUA to AUA or from UUA to UUG, and then AUA to AUG or from UUG to AUG. However, by replacing the original UUA codon with the UUG codon, only a single mutation would be required to produce the AUG (Met) codon. Since double point mutations in a single codon are infrequent during mutagenesis, the present method facilitates mutation of such a sequence.

The relative evolutionary power (REP) parameter allows one to easily substitute an original codon by a synonymous codon in order to maximize the ability to explore the evolutionary landscape for that codon position. For example, if the native codon is UUG (leucine), one might replace this native codon with either UUA or CUU which are both synonymous codons for leucine. However, selection of CUU would maximize the evolutionary landscape available because CUU has a REP of +4 while UUA only has a REP of +1. That is selection of CUU would allow the possibility of point mutations to codons encoding four amino acids inaccessible by point mutations of the original UUG codon, while selection of UUA would only allows reaching one amino acid inaccessible by point mutation of the original UUG codon. The introduction of the "relative evolutionary power" parameter allows a designer to determine an alternative codon that change as most as possible the evolutionary landscape explorable at a given codon position.

A process, by means of PERL based software, can calculate values of the "relative evolutionary power" parameter for each alternative codon and then replace each original codon by one alternative codon, in order to obtain two alternative sequences based either on having maximal intrinsic evolutionary power or having maximal relative evolutionary power.

The "evolutionary powers" described so far can be considered as quantitative ones because they rely on the mere counting of reachable amino-acids. However, "qualitative evolutionary power" may also be envisaged. For instance, a specific evolutionary power can be attributed to each synonymous codon according to the needs of the designer. This way a synonymous codon may also be selected based on its absolute ability to mutate to a codon encoding any amino acid different from that of the original codon.

Alternatively a synonymous codon may be selected on the basis of its specific ability to mutate to a codon encoding one of a specific class of amino acids, such as positively-charged (basic: lysine, arginine, histidine), negatively-charged (acidic: aspartate, glutamate), non-polar (hydrophobic: glycine, alanine, valine, leucine, isoleucine, methionine, phenylalanine, tryptophan, proline) or nonionizable polar (serine, threonine, asparagine, glutamine, cysteine, selenocysteine, tyrosine). Then, a designer can define a specific table of qualitative evolutionary power that would depend on the nature of native codons in order to force selection of alternative codon of same or different nature as the native one. For example, one can decide to attribute higher evolutionary power to alternative codon leading to basic amino-acid if the native codon encodes itself a basic amino acid. In such a case, if the native codon were CGA (Arg, basic) then more power would be attributed to CGC because CAC (which encodes His, another basic amino-acid) is reachable from CGC.

Also, one can decide to attribute a less evolutionary power to some codons leading to a limited usage of particular codons, to avoid for example the use of codons that are rarely used by the host or to avoid sequences having two consecutive or contiguous "rare" codons.

Selection of a synonymous codon may also be based on its ability to mutate into a codon encoding a specific amino acid, such as to a codon encoding an amino acid with an ability to form crosslinks (cysteine), ability to form kinks (proline) in a protein, or by its capacity for post-translational modification. For example, a double point mutation of a UCU or UCG serine codon in a wild-type nucleic acid sequence would be required to convert the Ser codon to a Cys codon. However, only a single point mutation would be required to make this change in a synonymous nucleotide sequence which uses a UCU or UCC Ser codon.

Alternatively, a synonymous nucleotide sequence may be selected to reduce its capacity or frequency of mutation by selecting one or more codons with a reduced capacity to change to another amino acid or by reducing the range of amino acids encoded by a mutant codon resulting from a single base mutation of the original codon. Such a method would be advantageous for stabilizing nucleic acid sequences used to produce biologically active polypeptides or vaccines.

The relative or intrinsic evolutionary power of an original sequence may be increased (or decreased) by modifying a number of codons ranging from one codon up to all the codons of the sequence. The percentage of codons modified may be expressed as either the number of modified codons divided by the total number of codons in the original sequence, or the number of modified codons divided by the number of codons having synonymous codons within the original sequence. For example, at least 0.01, 0.1, 0.25, 0.5, 1, 2, 5, 10, 20, 30, 40, 50, 60, 70, 80, 90, 95, 99 or even 100% of the codons of a given sequence may be modified. This range includes all intermediate values and subranges and the percentage values take into account the number of codons in the original polynucleotide, e.g., the minimal percent modification for a polynucleotide having only 100 codons (300 nucleotides) would be 1%. For example, the minimal modification to be made to a polynucleotide sequence would be the replacement of a single codon, where the substituted codon has a higher or lower intrinsic or relative evolutionary power than the codon in the corresponding wild-type or native polynucleotide sequence. The maximal number of codons of a polynucleotide which may be modified would be all the codons having at least one synonymous codon encoding the same amino acid. The range of modification contemplated by the present invention is from a single codon to all the synonymous codons or any intermediate percentage of modifiable codons, where the minimal percentage is expressed as 1 over the total number of codons in the polynucleotide sequence or 1 over the total number of modifiable codons (codons having at least one synonymous codon).

Selection of a synonymous nucleotide sequence can be performed using the computer-implemented method of the invention. This method analyzes or determines synonymous nucleic acid sequences of a given original gene sequence which have a modified capacity to mutate. This aspect also includes computer programs or software suitable for determining or selecting the desired synonymous nucleic acid sequence, as well as a computer system which executes or implements the software or computer program. One example of computer software suitable for this purpose is the ELP software (ELP for Evolutionary Landscape Painter), a PERL based Software developed by the inventors. A brief description of the steps included in the ELP software is described below.

The invention is not limited to the standard genetic code, but may also be applied to genes encoded by non-standard genetic codes, such as those found in vertebrate, invertebrate, yeast, or protist mitochondria, or in the nuclear nucleic acids of certain bacteria, yeasts and ciliates. It may also be applied to nucleic acids conforming to an artificial genetic code. For example, it may be used in conjunction with the use of a nonsense mutation suppression method, which incorporates non-standard amino acids into a polypeptide.

Once a synonymous nucleotide sequence has been identified, it may be synthesized by methods well-known in the art, such as by chemical or biochemical synthesis. Methods for synthesizing nucleotide sequences are described by Current Protocols in Molecular Biology (April, 2004, through supplement 66). For example, once the alternative sequence of the first mutated gene is obtained, the designed synthetic nucleic acid is prepared by synthesis of fragments of about 70 bp. Said fragments are 5' end phosphorylated, consecutive, correspond to the two strands of the gene and overlap the junctions of the complementary strand. These fragments are ligated to form the longer sequence desired.

When the synonymous nucleic acid sequence has been obtained, it may be subjected to mutation. Generally, the selected synonymous nucleic acid sequence will have a higher, greater or different capacity to mutate than the original nucleic acid sequence. The selected synonymous sequence is subjected to mutagenesis, mutant sequences (which encode amino acid sequences different than the original gene) are obtained, expressed and selected or screened on the basis of a factor of interest, often a biological property such as enzymatic activity or form immunogenic or antigenic activity.

Methods, vectors and host cells for expressing nucleic acid sequences are well-known and the methods described by Current Protocols in Molecular Biology, (April, 2004, supplement 66), see e.g., Chapters 1-3, 5 and 6. For example, a nucleic acid sequence may be expressed by inserting it into a vector, transforming the vector into a prokaryotic or eukaryotic host cell under conditions suitable for protein expression. For example, the synthetic synonymous nucleic acid may be cloned into a low copy number vector such as *ori* VpSC101 and then expressed in a bacterium such as *Escherichia coli*.

Alternatively, the mutated nucleotide sequence may be expressed using various cell-free protocols which are known in the art.

Methods for screening polypeptides encoded by mutated synonymous nucleic acid sequences involve selection on the basis of a genetic or phenotypic characteristic of the mutated polypeptide. For example, selection may be based on the biological activity of the mutant polypeptide, such as its enzymatic activity, substrate-binding activity, or immunological activity. A mutant enzyme may be tested for its absolute or relative enzymatic activity, and a mutated immunogen or antigen for its absolute or relative immunogencity or antigenicity.

Natural selection is based on the ability of a cell transformed with the mutant protein to survive under particular culture conditions (for example presence of particular chemicals or antibiotics) specifically designed to positively link features of interest to cell fitness. This selection could be made by spreading out the bacteria in a selective medium or by competition in liquid cultures containing antibiotic concentrations near the limit of resistance. The phenotype and nucleotide sequence of selected mutant can be confirmed and biochemical properties of the encoded proteins further evaluated.

Methods for analyzing the biological activity and structural characteristics are well-known in the art. Many screening methods are known to those of skill in the art. Specific reference is made to such methods as disclosed by Current Protocols in Molecular Biology (April, 2004, through supplement 66).

Once a mutant nucleic acid encoding a polypeptide mutant of interest is identified, the mutant nucleic acid sequence may be further modified by iterations of the above method. Once identified mutation of interest can also be put together on a sequence either synthesized or obtained by DNA shuffling in order to evaluate their interactions.

Mutant polypeptide sequences encoded by mutant or modified polynucleotides produced by the method of the present invention will generally have at least 90, 95 or 99% sequence similarity with the original polypeptide and will generally be encoded by polynucleotides which are at least 90, 95 or 99% similar to the polynucleotide sequence encoding the original polypeptide or a polynucleotide which is synonymous with that encoding the original polypeptide. Such mutant polypeptides may also be encoded by polynucleotide sequences which hybridize under stringent conditions to the original polynucleotide sequence or to a polynucleotide sequence synonymous with that of the original polynucleotide sequence determined by the methods of the present invention.

Such similarity may be determined by an algorithm, such as those described by Current Protocols in Molecular Biology, vol. 4, chapter 19 (1987-2004) or by using known software or computer programs such as the *BestFit* or *Gap* pairwise comparison programs (GCG Wisconsin Package, Genetics Computer Group, 575 Science Drive, Madison, Wisconsin 53711). *BestFit* uses the local homology algorithm of Smith and Waterman, Advances in Applied Mathematics 2: 482-489 (1981), to find the best segment of identity or similarity between two sequences. *Gap* performs global alignments: all of one sequence with all of another similar sequence using the method of Needleman and Wunsch, J. Mol. Biol. 48:443-453 (1970). When using a sequence alignment program such as *BestFit,* to determine the degree of sequence homology, similarity or identity, the default setting may be used, or an appropriate scoring matrix may be selected to optimize identity, similarity or homology scores. Similarly, when using a program such as *BestFit* to determine sequence identity, similarity or homology between two different amino acid sequences, the default settings may be used, or an appropriate scoring matrix, such as *blosum45* or *blosum80*, may be selected to optimize identity, similarity or homology scores.

Such variants may also be **characterized in that** a nucleic acid sequence encoding such a variant will hybridize under stringent conditions with the original or synonymous polynucleotide sequence. Such hybridization conditions may comprise hybridization at 5x SSC at a temperature of about 50 to 68° C. Washing may be performed using 2x SSC and optionally followed by washing using 0.5x SSC. For even higher stringency, the hybridization temperature may be raised to 68° C or washing may be performed in a solution of 0.1 x SSC. Other conventional hybridization procedures and conditions may also be used as described by Current Protocols in Molecular Biology, (1987-2004), see e.g. Chapter 2.

### EXAMPLES

*aac(6')-Ib* encodes an acetyltransferase which confer resistance to several widely used aminoglycosides antibiotics. Mutational properties of the wild-type and of a synthetic sequence derived from this gene are described below. It was established from the very start of years 1960 that nucleotidic composition of the genome of a given organism is directly reflected in its amino acid composition of its proteins (Sueoka N (1961) P.N.A.S. (USA) 47;1141-1149). We observed that this imprint influences the evolutionary landscape which can be explored by simple change starting from a given gene, i.e., to constrain the range of amino acids accessible by simple change from a codon. We thus propose a principle of systematic handling of any gene, founded on the redundancy of the code genetic and allowing determining the sequence of genes coding for identical proteins but offering a different evolutionary landscape.

This principle allows, for example, the identification of, nucleotide sequences the most different as possible from that of the initial gene. For each codon of a given gene, one can indeed determine to it alternate codons that code for the same amino acid but which will have an altered evolutionary power, that is to say either higher, smaller or merely different. The definition of the evolutionary power depends on the constraints that one want to impose on the sequence evolutionary landscape. It can correspond to the number of amino acids accessible by simple change from a codon ("intrinsic evolutionary power"), to be defined in a more restrictive way as the number of amino acids present in the evolutionary landscape of the alternate codon which did not form part of that of the initial codon ("relative evolutionary power") or even be calculated following a specific table set up by the designer according to his needs ("qualitative evolutionary power"). This change of coding theoretically makes possible to reach mutants which would normally require at least two changes in the same codon of the wild type gene to be able to be selected. Such double mutants of the same codon are obtained at very weak frequencies, whatever is the protocol of mutagenesis used and this even if iterative mutagenesis protocols starting from the mutants obtained are envisaged. Indeed, that would imply that the first change in the codon is at least neutral and as well as possible advantageous in term of fitness in order not to be eliminated by selection, which is absolutely not predictable. However, as this first change can be deleterious for the host, certain combinations cannot be explored by selection. One embodiment of this invention relates to a method that permits to increase specifically the number of double or triple mutations affecting some codons.

Two models have been successively developed in order to demonstrate the validity of this method. First, a synthetic gene was derived from the gene of the dehydrofolate reductase coded by gene dfrB1, which provides resistance to the antibiotic trimethoprim. The wild-type dfrB1 gene (further referred to as dfrBlWT) contains 52% G +C, however, the corresponding synthetic gene constructed dfrB1GC, contains 69% G+C. Both genes encode the same polypeptide sequence.

Experiences have been made starting from the dfrB1 WT gene having 52.7%GC and coding for a dehydrofolate reductase of 78 amino acids, conferring resistance to the trimethoprim (MIC 512 micg/ml).

A synthetic gene was then designed with a different evolutionary potential by imposing a %GC from 69 + 0.2, and the avoidance of E. coli rare codons, with a tolerance for rare codon (codon use less than 5% for the codons of a given amino acid) and a codon use optimized when compared to the codon use of Deinococcus radiodurans (a bacteria with a high %GC content).

The DfrB1GC gene was then assembled by hybridization of the six synthetic nucleotides hereafter:

| | | | |
|---|---|---|---|
| DfrC1 | TATGGAGCGCAGCAGCAACGAGGTGAGCAACCCGGTCG CCGGCAACTTCGTGTTCCCCAGCGACGCCACCTTCGGCA TGGGCGACCG | 0.2 | Phosphorylation 5' |
| DfrC2 | CGTGCGCAAGAAGAGCGGCGCCGCCTGGCAGGGCCAGA TCGTGGGCTGGTACTGCACCAACCTGACCCCCGAGGGCT ACGCCGTGGA | 0.2 | Phosphorylation 5' |
| DfrC3 | GAGCGAGGCCCACCCCGGCAGCGTGCAGATCTACCCCG TGGCCGCCCTCGAGCGGATCAACTAA | 0.2 | Phosphorylation 5' |
| DfrC4 | CGTCGCTGGGGAACACGAAGTTGCCGGCGACCGGGTTG CTCACCTCGTTGCTGCTGCGCTCCA | 0.2 | Phosphorylation 5' |
| DfrC5 | TCAGGTTGGTGCAGTACCAGCCCACGATCTGGCCCTGCC AGGCGGCGCCGCTCTTCTTGCGCACGCGGTCGCCCATGC CGAAGGTGG | 0.2 | Phosphorylation 5' |
| DfrC6 | CGCGTTAGTTGATCCGCTCGAGGGCGGCCACGGGGTAG ATCTGCACGCTGCCGGGGTGGCCTCGCTCTCCACGGCG TAGCCCTCGGGGG | 0.2 | Phosphorylation 5' |

Then a ligation in a pTZ18R plasmid bearing a synthetic promoter Ptac, clonage sites NdeI-MluI for inserting the synthetic gene, previously digested by these enzymes.

The *dfrB1wt* gene has been cloned in the same sites and in an identical environment.

Both constructions have been inserted as a unique copy at *metA* locus of the *E. coli* chromosome by allelic exchange. This locus codes for an unrelated homoserine transsuccinylase, which is a very good locus to reach integration in *E. coli* chromosome, because it is quite stable.

Both bacterial strains *dfrB1*_{*W*T} and *dfrB1_{GC}* which were isogenic except for the *dfrB1* alleles, were then submitted to continuous growth in selective medium (Mueller-Hinton + Trimethoprim at 37°C) by serial transfer of 10⁹ cells, for 350 generations as described by Lenski and Travisano (1994).

Briefly, one milliliter of media containing 10⁹ cells issued from each culture cycle is inoculated with 63 ml of culture medium.

Maximal growth in such conditions allows six generations to be made (2⁶=64).

This high cell density in the inoculum warrants the presence of at least 10 mutated versions of the targeted gene and the conservation of the mutations. About 20 generations per day have been hen established.

This protocol allows the competitive selection of cells showing the best fitness in a given population. The populations obtained at the end of the 350 generations, in both allelic population were then submitted to competition by co -cultivation for 20 generations with either their own progenitor, the evolved population, or between evolved population (*dfrB1*WT + *dfrB1*GCevolved; *dfrB1*GC + *dfrB1*GC evolved: *dfrB1*WT + *dfr1*WTevolved; *dfrB1*WTevolved et *dfrB1*GCevolved in mixes 1:1) as exemplified in the review of Elena and Lenski (2003, Nature reviews, 4(6): 457-469). Whatever could be the co-cultivation considered, we found that the *dfrB1*GCevolved population took over all other populations by far ( ≥99.9%). Sequencing showed that the *dfrB1*GCevolved population was homogeneous and constituted of only a single clone carrying a mutation in the 8th codon of *dfrB1*GC, leading to a substitution of the valine residue into a methionine (V8M). PI transduction of the *dfrB1*GC(V8M) allele in the WT strain MG1655, i.e., in an unselected genome context, and repetition of the co-cultivation experiments confirmed that the V8M mutation was uniquely and unambiguously responsible of the selective advantage.

The analysis of both cultures shows effectively unique mutation in the complete sequence gene + promoter, a change G into A of the first base of the codon 8 a to a substitution Val into Met in position 8 (GTG into ATG).

This mutation has been placed in its initial context by translation and the same results in co-culture experiences have been obtained. This last observation confirms that this mutation is effectively at the origin of the selective advantage.

To obtain this mutation from the original gene sequence, two point mutations would have been required: GTC into ATG. This example clearly illustrates the possible applications of this principle, which enables a considerable modulation of the evolutionary landscape that can be explored from a given gene coding for a functional protein.

Another model has been developed to further assess the efficiency of the principle. A synthetic gene was derived from the gene of the aminoglycoside acetyltransferase coded by *aac(6')-Ib*, which typically provides resistance to the antibiotics tobramycin and amikacin. The wild-type *aac(6')-Ib* gene (further referred to as *aac(6')-Ib* _{WT}) contains 54% G +C. The corresponding synthetic gene constructed, *aac(6')-Ib_{SYN}"* contains 51 % G+C, in harmony with *E. coli* genome composition. Both genes encode the same polypeptide sequence. However, the two sequences share only 61% similarity at the nucleic acid level. On average, each codon of *aac(6')-Ib_{SYN}* can lead to 1.6 amino acids that were not reachable by *aac(6')-Ib*_{WT}*.*

The *aac(6')-Ib_{SYN}* gene was then assembled by hybridization of the 16 synthetic nucleotides hereafter:

| N° | Name | Sequence | Phosphorylation |
|---|---|---|---|
| 1. | AAClt1 | AATTCATATGACGGAACACGATTTGGCCATGTTGTAC | Phosphorylation 5' |
| 2. | AAClt2 | GAATGGTTGAACAGAAGTCACATTGTGGAATGGTGGGGGGT GAGGAGGCTAGACCCACTTTGGCAGATG | Phosphorylation 5' |
| 3. | AAClt3 | TCCAAGAGCAATATCTTCCCTCGGTGCTGGCCCAGGAAAGTGT GACGCCCTATATCGCTATGCTTAACGG | Phosphorylation 5' |
| 4. | AAClt4 | TGAACCCATCGGTTACGCACAAAGTTATGTGGCATTGGGTTCG GGTGATGGTTGGTGGGAGGAGGAGACG | Phosphorylation 5' |
| 5. | AAClt5 | GACCCCGGTGTCAGAGGTATTGATCAACTGCTTGCCAGGTTCG GGTGATGGTTGGTGGGAGGAGGAGACG | Phosphorylation 5' |
| 6. | AAClt6 | GACCCCGGTGTCAGAGGTATTGATCAACTGCTTGCCACCCAGA AGTGACGAAAATTCAGACTGATCCCAG | Phosphorylation 5' |
| 7. | AAClt7 | TCCCTCGAATCTTAGAGCCATTAGATGTTATGAAAAGGCCGGT TTCGAACGTCAGGGGACGGTCACGACG | Phosphorylation 5' |
| 8. | AAClt8 | CCCGACGGGCCCGCAGTTTATATGGTGCAGACTAGACAAGCTT TTGAAAGAACTAGATCGGACGCATGAG | Phosphorylation 5' |
| 9. | AAClb1 | CCCACCATTCCACAATGTGACTTCTGTTCAACCATTCGTACAAC ATGGCCAAATCGTGTTCCGTCATATG | Phosphorylation 5' |
| 10. | AAClb2 | TCCTGGGCCAGCACCGAGGGAAGATATTGCTCTTGGACATCTG CCAAAGTGGGTCTAGCCTCCTCACCCC | Phosphorylation 5' |
| 11. | AAClb3 | CAATGCCACATAACTTTGTGCGTAACCGATGGGTTCACCGTTA AGCATAGCGATATAGGGCGTCACACTT | Phosphorylation 5' |
| 12. | AAClb4 | TGGCAAGCAGTTGATCAATACCTCTGACACCGGGGTCCGTCTC CTCCTCCCACCAACCATCACCCGAACC | Phosphorylation 5' |
| 13. | AAClb5 | TGGCAAGCAGTTGATCAATACCTCTGACACCGGGGTCCGTCTC CTCCTCCCACCAACCATCACCCGAACC | Phosphorylation 5' |
| 14. | AAClb6 | CTTTTCATAACATCTAATGGCTCTAAGATTCGAGGGACTGGGA TCAGTCTGAATTTTCGTCACTTCTGGG | Phosphorylation 5' |
| 15. | AAClb7 | GTCTAGTCTGCACCATATAAACTGCGGGCCCGTCGGGCGTCGT GACCGTCCCCTGACGTTCGAAACCGGC | Phosphorylation 5' |
| 16. | AAClb8 | GATCCTCATGCGTCCGATCTAGTTCTTTCAAAAGCTT | Phosphorylation 5' |

The assembly product was then ligated in a low copy number plasmid derived from pAM238 by partial deletion of polylinker and introduction of EcoRI cloning site. This plasmid carries a Plac promoter controlled by LacI, upstream of the BamHI-EcoRI cloning sites, in which the synthetic gene is inserted. This system allows a controlled gene expression, in conditions related to those of a chromosomal gene.

The *aac(6')-Ib*_{WT} gene has been cloned in the same sites and in an identical environment.

Both sequences *aac(6')-Ib*_{WT} and *aac(6)-Ib*_{SYN} were subjected to mutagenesis using error-prone PCR (mutazyme II^{©} kit, stratagene). The resulting alleles were cloned into the previously described plasmid and then transformed into *E.coli*. Two independent libraries exhibiting different mutation rates (around 1 mutation and 5 mutations per gene) were created for each sequence. Within a given library, each individuals were isogenic except for the *aac(6')-Ib* alleles. Libraries were then screened in structured medium (Luria Broth + Agar + IPTG) in presence of an antibiotic gradient. The following aminoglycosides were used to create independent gradients: Tobramycine, Amikacine, Neomycin, Gentamicin, Isepamicin.

Enhanced resistance phenotypes are identified as a isolated colony at antibiotic concentration higher than the original MIC. Such colonies are purified. These *aac(6')*-Ib alleles are then re-isolated, cloned and transformed in a naïve genetic environment in order to eliminate false positive candidates. Once confirmed, resistance profiles on all five aminoglycosides and sequence of the corresponding alleles are determined.

**Table 1: Mutation isolated are represented according to the antibiotic they have been selected on and the version of the genes from which they are derived. The figures into brackets refers to the increase in MIC compared to wild type versions. Codons implicated are presented into parenthesis.**

| | **Tob** | **Neo** | **Amk** | **Gm** | **Isp** |
|---|---|---|---|---|---|
| ***Aa_ini*** | **∅** | **∅** | **∅** (101 : CAA) | **L102S** (102 : TTA → TCA) **[ x 5 ]** | **∅** (55: TTA) |
| ***aac_syn*** | **∅** | **∅** | **Q101L** (101 : CAG → CTG) **[ x 3 ]** | **∅** (102 : CTG) | **L55Q** (55: CTG → CAG) **[ x 8 ]** |

| | | | | | |
|---|---|---|---|---|---|
| aac_ini : initial sequence ; aac_syn : synthetic sequence ; Tob : tobramycin ; Neo : neomycin ; Amk : amikacin ; Gm : gentamicin ; Isp : isepamicin ;0 : no advantageous mutant identified | | | | | |

The results are represented in Table 1 above. Few mutations have been isolated, in spite of the enhanced exploration of the local sequence space by *aac(6')-Ib_{WT} and aac(6')-Ib_{SYN}.* This can be interpreted as a proof of the limited evolutionary perspectives of the protein, particularly on Tobramycin and Neomycin. On Amikacin, Gentamicin and Isepamicin, mutations that improved the level of resistance have been isolated. However, the two versions of the genes did not lead to the same set of variants. The *aac(6')-Ib_{WT}* gene only led to isolation of a L102S mutation on gentamicin. This substitution have been widely described in clinical strains bearing the *aac(6')-Ib* gene (ref). Indeed a simple transition from T to C allows TTA, encoding leucine in the wild type gene to reach TCA, encoding serine. This substitution has not been isolated from libraries of the synthetic gene. Indeed, in *aac(6')-Ib_{SYN}* TTA has been changed to the synonymous codon CTG, because REP_{CTG/TTA} = 4. The change from leucine to serine would then have required two mutations from CTG to TCG.

The other identified mutations have only been isolated from synthetic gene mutant libraries. The mutation Q101L induces a threefold increase of MIC on amikacin. This substitution is due to a transition from CAG to CTG. Such a substitution is possible from *aac(6')-Ib_{WT}*: in this sequence glutamine is represented by CAA which can lead to leucine CTA. However, the codon CTA is weakly used in several γ-proteobacteria species where the gene *aac(6')-Ib* is commonly found. Weakly used codons are known to reduce translation efficiency (accuracy and speed). CTA is then likely to be counter selected in nature, even if Q101L is otherwise advantageous. Indeed this mutation has only been described once, in association with the mutation L102S (ref).

The substitution L55Q has been isolated on isepamicin. It correspond to a direct CTG to CAG transversion in the *aac(6')-Ib_{SYN}* gene. The leucine is encoded by TTA in *aac(6')-Ib_{WT}*. Reaching a glutamine codon from TTA require TAA or CTA as intermediates. CTA is likely to be counter selected due to weak usage. TAA correspond to STOP in the genetic code. As a 185 amino-acids long protein is not likely to be functional when restricted to its first 55 amino-acids, STOP codon must be counter selected at position 55. The only way to access glutamine from TTA would then be through the sequence TTA→TTG→CTG→CAG, which is highly susceptible to genetic drift in large population of bacteria. The L55Q substitution has never been described so far, which might be taken as a proof of non accessibility in nature.

Two advantageous substitutions out of three would not has been isolated without inclusion of the *aac(6')-Ib_{SYN}* gene into the directed evolution protocol developed. The rational design of an alternative sequence permits to broaden exploration of the sequence space, and hence to enhance directed evolution protocol efficiency.

### Use of ELP software to select oligonucleotide sequences

A systematic principle of handling of any gene was proposed by the inventors, based on the redundancy of the code genetic and allowing to determine alternative sequences, coding for identical proteins but offering a potential landscape evolutionary different, even possibly most different possible from that from initial gene. Such alternative sequences give access by simple substitution to inaccessible amino acids since the native sequence. This protocol thus makes it possible to pass goatskin bottles certain constraints selective or stochastic in order to explore in a more extensive way the universe of the possible ones.

An algorithm was implemented, called Evolutionary Landscape Painter, able for any gene to determine alternative sequences of better Relative Evolutionary Potential (REP) compared to the wild version, even of better REP when one compared to the other in reference to the savage.

The Relative Evolutionary Potential of a codon X compared to a synonymous codon Y is defined like the cardinal of the whole of the acids amino accessible by a simple change from the codon X which is not accessible since Y. This program was used to build synthetic versions of the gene: aac(6')-Ib, a bacterial gene of resistance to the aminoglycosides.

### Directed evolution of the gene aac(6')-Ib

A synthetic version of the gene aac(6')-Ib was assembled. This gene codes for N-acetyl transferase pertaining to the super family of GNATs (GCN5-related N-acetyl transferase (Neuwald and Landsman, 1997). GNATs constitute a super-family of enzymes which catalyse the transfer of an acetyl group starting from the acetyl-CoA on primary amines carried by a large variety of acceptant molecules.

More precisely, AAC(6')-Ib is an acetylase modifying some aminoglycosides (tobramycin, netilmicin, kanamycin and amikacin) but not of others (gentamicin, isepamycin). This gene has 185 codons (555 NT, G+C 54%). These characteristics make of it an ideal candidate to test the model, by widening it to obtain mutants recognizing new substrates.

Indeed, it is possible to select the mutants having an increased acetylating activity with respect to its natural substrates, but also to select mutants presenting a new acetylating spectrum. These last mutants present a much broader potential in term of industrial and search application that a simple increase in activity.

Four banks were built presenting increasing rates of changes starting from the synthetic gene. Four similar banks were established starting from the wild gene aac(6')-Ib. These banks are screened on tobramycin, neomycin, kanamycin and amikacin, natural substrates of the enzyme, for an increase in activity. The screen is also carried out on gentamycin and isepamicin, in order to isolate variants having modified spectra of resistance.

No mutant with the increased capacities of resistance was identified on tobramycin, amikacin, kanamycin or neomycin. We conclude that the gene aac(6')-Ib reached its evolutionary limits for the acetylating of its natural substrates. This result is supported by the results of a study carried out on the gene aac(6')-Iaa (Salipante & Hall, Mol. Biol. Evol, 2003; 20(4): 653-659).

Several works mention the spontaneous appearance in clinical stocks of a variant gene, called aac(6')-Ib', allowing the acetylating of gentamicin instead of amikacin. By doing this the protein acquires the characteristics of an AAC of type II instead of type I.

This event is due to a single punctual mutation. It concerns a transition from T towards C which results in the replacement of a leucine by a serine into position 102.

This mutant was found in all the banks of aac(6')-Ib wild gene. On the other hand, none of the banks of synthetic gene allowed the isolation of said genotype, nor of any other genotype suggesting the existence of other variants able to resist to gentamycin.

A mutant was isolated whose capacities of resistance to isepamycin are increased (CMI X 10). The mutation consists of the substitution of a leucine by a glutamine in position 55. This variant was only isolated starting from the banks resulting from synthetic gene. Such substitution is not reachable starting from initial gene.

Leucine is encoded there by codon TTA, but the glutamine corresponds to code CAA and CAG. On the other hand in synthetic gene, this leucine is represented by codon CTG. A conversion of T towards A thus carries out to obtaining a glutamine.

Other mutants are in the course of characterization. The screen procedure proves being hard because it is difficult to isolate a genotype. Indeed the resistance conferred by the gene aac(6')-Ib corresponds to a strategy of inactivation of antibiotic. Thus concentration in functional amynoglycosides decreases locally during time around colonies allowing the less resistant phenotypes to grow in their turn. The coexistence of several genotypes within the same colony in structured medium were observed. This phenomenon prohibits the development of a screen based on the natural selection in medium not structured, weighing down as much handling necessary.

The results obtained until now consolidate this observation. The synthetic gene gave access to a variant showing increased resistance to isepamycin. This mutant was not obtained starting from wild gene. Moreover any natural or synthetic variant of the gene aac(6')-Ib presenting this variation was not described in the data bases. On a deeper phylogenetic level, none AACs correlated with AAC(6')Ib carries the described variation. Thus it seems that in nature, as at the laboratory, the L55Q mutation cannot emerge starting from wild gene.

In addition the mutation L102S was obtained driving to the replacement of a resistance to the amikacin by a resistance to gentamicin only starting from wild gene. That shows that the synthetic sequence in spite of the protocol of mutagenesis which is imposed to him cannot reach serine any more. The constraints weighing on this sequence are quite different from those being exerted on the initial sequence. From this point of view, it is possible to handle a gene in order to block its natural evolution towards variant which one wishes to avoid.

In conclusion, the application of the principle of widening the evolutionary landscape of a gene, shows the interest of the alternate gene synthesis for obtaining of new variant out of evolutionary possibilities starting from merely native genes.

### COMPUTER-IMPLEMENTED ASPECTS OF THE INVENTION

The invention encompasses computer-implemented selection of a synonymous nucleotide sequence containing at least one synonymous codon from among a multitude of such synonymous codons and includes the attribution to each codon of some structural parameters that when combined allow the selection of the best mutation depending on the evolutionary power required.

The following table shows aspects of the evolutionary landscape painter program.

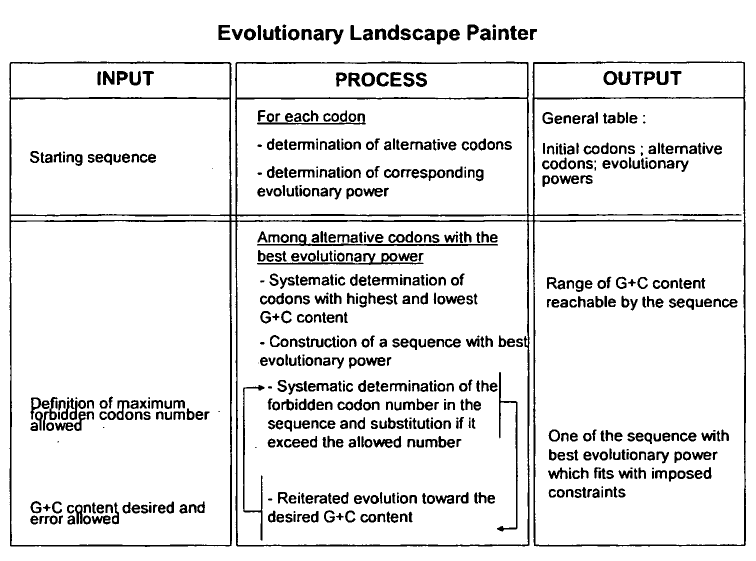

The Evolutionary Landscape Painter computer program allows the determination of alternative sequences having the best relative evolutionary power (REP) for any DNA sequence written in A/T/C/G language. It is possible to select the GC content of the final sequence as well as to control the number of codons infrequently used in the final sequence.

The GC content of the genome of a particular organism is reflective of global constrains at the molecular level. It is preferable to be constrained to the GC content of the host organism in order to avoid the action of any parasitic evolutionary pressure. The computer program calculates the GC global contents of the entire sequence. Consequently, locally, the generated alternative sequences do not present a constant GC content.

Inside a genome, the use of codons is not randomly permitted. Thus, for a given amino acid, some correspondent (synonymous) codons are poorly represented. The excessive presence of such codons within a sequence could give rise to an early termination of the protein translation. Therefore, it is preferable to limit the content of such codons within the alternative sequence.

A forbidden codon is defined by the following rule. For a given amino acid, a coefficient is calculated as follows: frequency of the most used codon/frequency of the less used codon. If the value of this coefficient is higher than 6, then the codon having the slighter frequency is arbitrarily considered as having too slight a usage and is forbidden.

The ELP Program is written in PERL language. To execute it, it is necessary to have activeperl. PERL software is freely accessible at the following URL: http://www.perl.org/get.html. To use the ELP program enter the Windows command, search the file containing the ELP file and select the text file "sequence.txt". This file corresponds to the original DNA sequence. Then type, >perl E.L.P. sequence.txt (1). The program will prompt the entry of the following data:
1. the number "N" of the forbidden codons tolerated in the final sequence;
2. the GC content "P" searched in the final sequence and
3. the threshold or error "E" tolerated for the GC content.

The output may be printed as a text file by typing: >output text" at the end of the command line (1) before executing the program.

Figure 4 illustrates a computer system 1201 upon which an embodiment of the present invention may be implemented. The computer system 1201 includes a bus 1202 or other communication mechanism for communicating information, and a processor 1203 coupled with the bus 1202 for processing the information. The computer system 1201 also includes a main memory 1204, such as a random access memory (RAM) or other dynamic storage device (e.g., dynamic RAM (DRAM), static RAM (SRAM), and synchronous DRAM (SDRAM)), coupled to the bus 1202 for storing information and instructions to be executed by processor 1203. In addition, the main memory 1204 may be used for storing temporary variables or other intermediate information during the execution of instructions by the processor 1203. The computer system 1201 further includes a read only memory (ROM) 1205 or other static storage device (e.g., programmable ROM (PROM), erasable PROM (EPROM), and electrically erasable PROM (EEPROM)) coupled to the bus 1202 for storing static information and instructions for the processor 1203.

The computer system 1201 also includes a disk controller 1206 coupled to the bus 1202 to control one or more storage devices for storing information and instructions, such as a magnetic hard disk 1207, and a removable media drive 1208 (e.g., floppy disk drive, read-only compact disc drive, read/write compact disc drive, compact disc jukebox, tape drive, and removable magneto-optical drive). The storage devices may be added to the computer system 1201 using an appropriate device interface (e.g., small computer system interface (SCSI), integrated device electronics (IDE), enhanced-IDE (E-IDE), direct memory access (DMA), or ultra-DMA).

The computer system 1201 may also include special purpose logic devices (e.g., application specific integrated circuits (ASICs)) or configurable logic devices (e.g., simple programmable logic devices (SPLDs), complex programmable logic devices (CPLDs), and field programmable gate arrays (FPGAs)).

The computer system 1201 may also include a display controller 1209 coupled to the bus 1202 to control a display 1210, such as a cathode ray tube (CRT), for displaying information to a computer user. The computer system includes input devices, such as a keyboard 1211 and a pointing device 1212, for interacting with a computer user and providing information to the processor 1203. The pointing device 1212, for example, may be a mouse, a trackball, or a pointing stick for communicating direction information and command selections to the processor 1203 and for controlling cursor movement on the display 1210. In addition, a printer may provide printed listings of data stored and/or generated by the computer system 1201.

The computer system 1201 performs a portion or all of the processing steps of the invention in response to the processor 1203 executing one or more sequences of one or more instructions contained in a memory, such as the main memory 1204. Such instructions may be read into the main memory 1204 from another computer readable medium, such as a hard disk 1207 or a removable media drive 1208. One or more processors in a multi-processing arrangement may also be employed to execute the sequences of instructions contained in main memory 1204. In alternative embodiments, hard-wired circuitry may be used in place of or in combination with software instructions. Thus, embodiments are not limited to any specific combination of hardware circuitry and software.

As stated above, the computer system 1201 includes at least one computer readable medium or memory for holding instructions programmed according to the teachings of the invention and for containing data structures, tables, records, or other data described herein. Examples of computer readable media are compact discs, hard disks, floppy disks, tape, magneto-optical disks, PROMs (EPROM, EEPROM, flash EPROM), DRAM, SRAM, SDRAM, or any other magnetic medium, compact discs (e.g., CD-ROM), or any other optical medium, punch cards, paper tape, or other physical medium with patterns of holes, a carrier wave (described below), or any other medium from which a computer can read.

Stored on any one or on a combination of computer readable media, the present invention includes software for controlling the computer system 1201, for driving a device or devices for implementing the invention, and for enabling the computer system 1201 to interact with a human user (e.g., print production personnel). Such software may include, but is not limited to, device drivers, operating systems, development tools, and applications software. Such computer readable media further includes the computer program product of the present invention for performing all or a portion (if processing is distributed) of the processing performed in implementing the invention.

The computer code devices of the present invention may be any interpretable or executable code mechanism, including but not limited to scripts, interpretable programs, dynamic link libraries (DLLs), Java classes, and complete executable programs. Moreover, parts of the processing of the present invention may be distributed for better performance, reliability, and/or cost.

The term "computer readable medium" as used herein refers to any medium that participates in providing instructions to the processor 1203 for execution. A computer readable medium may take many forms, including but not limited to, non-volatile media, volatile media, and transmission media. Non-volatile media includes, for example, optical, magnetic disks, and magneto-optical disks, such as the hard disk 1207 or the removable media drive 1208. Volatile media includes dynamic memory, such as the main memory 1204. Transmission media includes coaxial cables, copper wire and fiber optics, including the wires that make up the bus 1202. Transmission media also may also take the form of acoustic or light waves, such as those generated during radio wave and infrared data communications.

Various forms of computer readable media may be involved in carrying out one or more sequences of one or more instructions to processor 1203 for execution. For example, the instructions may initially be carried on a magnetic disk of a remote computer. The remote computer can load the instructions for implementing all or a portion of the present invention remotely into a dynamic memory and send the instructions over a telephone line using a modem. A modem local to the computer system 1201 may receive the data on the telephone line and use an infrared transmitter to convert the data to an infrared signal. An infrared detector coupled to the bus 1202 can receive the data carried in the infrared signal and place the data on the bus 1202. The bus 1202 carries the data to the main memory 1204, from which the processor 1203 retrieves and executes the instructions. The instructions received by the main memory 1204 may optionally be stored on storage device 1207 or 1208 either before or after execution by processor 1203.

The computer system 1201 also includes a communication interface 1213 coupled to the bus 1202. The communication interface 1213 provides a two-way data communication coupling to a network link 1214 that is connected to, for example, a local area network (LAN) 1215, or to another communications network 1216 such as the Internet. For example, the communication interface 1213 may be a network interface card to attach to any packet switched LAN. As another example, the communication interface 1213 may be an asymmetrical digital subscriber line (ADSL) card, an integrated services digital network (ISDN) card or a modem to provide a data communication connection to a corresponding type of communications line. Wireless links may also be implemented. In any such implementation, the communication interface 1213 sends and receives electrical, electromagnetic or optical signals that carry digital data streams representing various types of information.

The network link 1214 typically provides data communication through one or more networks to other data devices. For example, the network link 1214 may provide a connection to another computer through a local network 1215 (e.g., a LAN) or through equipment operated by a service provider, which provides communication services through a communications network 1216. The local network 1214 and the communications network 1216 use, for example, electrical, electromagnetic, or optical signals that carry digital data streams, and the associated physical layer (e.g., CAT 5 cable, coaxial cable, optical fiber, etc.). The signals through the various networks and the signals on the network link 1214 and through the communication interface 1213, which carry the digital data to and from the computer system 1201 maybe implemented in baseband signals, or carrier wave based signals. The baseband signals convey the digital data as unmodulated electrical pulses that are descriptive of a stream of digital data bits, where the term "bits" is to be construed broadly to mean symbol, where each symbol conveys at least one or more information bits. The digital data may also be used to modulate a carrier wave, such as with amplitude, phase and/or frequency shift keyed signals that are propagated over a conductive media, or transmitted as electromagnetic waves through a propagation medium. Thus, the digital data may be sent as unmodulated baseband data through a "wired" communication channel and/or sent within a predetermined frequency band, different than baseband, by modulating a carrier wave. The computer system 1201 can transmit and receive data, including program code, through the network(s) 1215 and 1216, the network link 1214 and the communication interface 1213. Moreover, the network link 1214 may provide a connection through a LAN 1215 to a mobile device 1217 such as a personal digital assistant (PDA) laptop computer, or cellular telephone.

The computer system 1201 may also include special purpose logic devices (e.g., application specific integrated circuits (ASICs)) or configurable logic devices (e.g., simple programmable logic devices (SPLDs), complex programmable logic devices (CPLDs), and field programmable gate arrays (FPGAs)).

The computer system 1201 may also include a display controller 1209 coupled to the bus 1202 to control a display 1210, such as a cathode ray tube (CRT), for displaying information to a computer user. The computer system includes input devices, such as a keyboard 1211 and a pointing device 1212, for interacting with a computer user and providing information to the processor 1203. The pointing device 1212, for example, may be a mouse, a trackball, or a pointing stick for communicating direction information and command selections to the processor 1203 and for controlling cursor movement on the display 1210. In addition, a printer may provide printed listings of data stored and/or generated by the computer system 1201.

The computer system 1201 performs a portion or all of the processing steps of the invention in response to the processor 1203 executing one or more sequences of one or more instructions contained in a memory, such as the main memory 1204. Such instructions may be read into the main memory 1204 from another computer readable medium, such as a hard disk 1207 or a removable media drive 1208. One or more processors in a multi-processing arrangement may also be employed to execute the sequences of instructions contained in main memory 1204. In alternative embodiments, hard-wired circuitry may be used in place of or in combination with software instructions. Thus, embodiments are not limited to any specific combination of hardware circuitry and software.

As stated above, the computer system 1201 includes at least one computer readable medium or memory for holding instructions programmed according to the teachings of the invention and for containing data structures, tables, records, or other data described herein. Examples of computer readable media are compact discs, hard disks, floppy disks, tape, magneto-optical disks, PROMS (EPROM, EEPROM, flash EPROM), DRAM, SRAM, SDRAM, or any other magnetic medium, compact discs (e.g., CD-ROM), or any other optical medium, punch cards, paper tape, or other physical medium with patterns of holes, a carrier wave (described below), or any other medium from which a computer can read.

Stored on any one or on a combination of computer readable media, the present invention includes software for controlling the computer system 1201, for driving a device or devices for implementing the invention, and for enabling the computer system 1201 to interact with a human user (e.g., print production personnel). Such software may include, but is not limited to, device drivers, operating systems, development tools, and applications software. Such computer readable media further includes the computer program product of the present invention for performing all or a portion (if processing is distributed) of the processing performed in implementing the invention.

The computer code devices of the present invention may be any interpretable or executable code mechanism, including but not limited to scripts, interpretable programs, dynamic link libraries (DLLs), Java classes, and complete executable programs. Moreover, parts of the processing of the present invention may be distributed for better performance, reliability, and/or cost.

The term "computer readable medium" as used herein refers to any medium that participates in providing instructions to the processor 1203 for execution. A computer readable medium may take many forms, including but not limited to, non-volatile media, volatile media, and transmission media. Non-volatile media includes, for example, optical, magnetic disks, and magneto-optical disks, such as the hard disk 1207 or the removable media drive 1208. Volatile media includes dynamic memory, such as the main memory 1204. Transmission media includes coaxial cables, copper wire and fiber optics, including the wires that make up the bus 1202. Transmission media also may also take the form of acoustic or light waves, such as those generated during radio wave and infrared data communications.

Various forms of computer readable media may be involved in carrying out one or more sequences of one or more instructions to processor 1203 for execution. For example, the instructions may initially be carried on a magnetic disk of a remote computer. The remote computer can load the instructions for implementing all or a portion of the present invention remotely into a dynamic memory and send the instructions over a telephone line using a modem. A modem local to the computer system 1201 may receive the data on the telephone line and use an infrared transmitter to convert the data to an infrared signal. An infrared detector coupled to the bus 1202 can receive the data carried in the infrared signal and place the data on the bus 1202. The bus 1202 carries the data to the main memory 1204, from which the processor 1203 retrieves and executes the instructions. The instructions received by the main memory 1204 may optionally be stored on storage device 1207 or 1208 either before or after execution by processor 1203.

The computer system 1201 also includes a communication interface 1213 coupled to the bus 1202. The communication interface 1213 provides a two-way data communication coupling to a network link 1214 that is connected to, for example, a local area network (LAN) 1215, or to another communications network 1216 such as the Internet. For example, the communication interface 1213 may be a network interface card to attach to any packet switched LAN. As another example, the communication interface 1213 may be an asymmetrical digital subscriber line (ADSL) card, an integrated services digital network (ISDN) card or a modem to provide a data communication connection to a corresponding type of communications line. Wireless links may also be implemented. In any such implementation, the communication interface 1213 sends and receives electrical, electromagnetic or optical signals that carry digital data streams representing various types of information.

The network link 1214 typically provides data communication through one or more networks to other data devices. For example, the network link 1214 may provide a connection to another computer through a local network 1215 (e.g., a LAN) or through equipment operated by a service provider, which provides communication services through a communications network 1216. The local network 1214 and the communications network 1216 use, for example, electrical, electromagnetic, or optical signals that carry digital data streams, and the associated physical layer (e.g., CAT 5 cable, coaxial cable, optical fiber, etc). The signals through the various networks and the signals on the network link 1214 and through the communication interface 1213, which carry the digital data to and from the computer system 1201 maybe implemented in baseband signals, or carrier wave based signals. The baseband signals convey the digital data as unmodulated electrical pulses that are descriptive of a stream of digital data bits, where the term "bits" is to be construed broadly to mean symbol, where each symbol conveys at least one or more information bits. The digital data may also be used to modulate a carrier wave, such as with amplitude, phase and/or frequency shift keyed signals that are propagated over a conductive media, or transmitted as electromagnetic waves through a propagation medium. Thus, the digital data may be sent as unmodulated baseband data through a "wired" communication channel and/or sent within a predetermined frequency band, different than baseband, by modulating a carrier wave. The computer system 1201 can transmit and receive data, including program code, through the network(s) 1215 and 1216, the network link 1214 and the communication interface 1213. Moreover, the network link 1214 may provide a connection through a LAN 1215 to a mobile device 1217 such as a personal digital assistant (PDA) laptop computer, or cellular telephone. See also, Figure 4.

### An Example of How ELP Works

The synthesis of two alternative sequences is enough to explore all the sequences having the same evolutionary power. The first output result is random but, in selecting a second sequence, one takes in account the first generated sequence. For each amino acid, it exists at the maximum three codon having different evolutionary landscapes. If two alternative sequences are constructed with ELP there are three alternative sequences:
- the original sequence,
- the first alternative sequence, and
- the second alternative sequence.

An amino acid can be imagined in a position n for which it can be found three codons with different evolutionary powers: c1, c2 and c3. Now, if the original sequence bears a codon c1, then ELP will be choose c2 or c3 randomly for the first alternative sequence and, during the determination of the second alternative sequence, ELP will take into account both, the first original sequence (bearing c1), but also the first alternative one (bearing c2. It will not have another choice than that of selecting the third alternative codon c3. This is the reason why the synthesis of two alternative sequences is enough to explore the whole possibilities.

On the contrary, one can not to take in account the combinatory related to the incorporation of codons:
if the first original sequence bears in a position "n" an alternative codon cn1 and in position "m" an alternative codon cm1 and on the second sequence cn2 and cm2, one could imagine other alternative sequences with combinations (cn1,cm2) or (cn2, cm1) only if the amino acids placed at those position would have different evolutionary powers. It's impossible to extrapolate this to the all codons at the whole positions. The huge number of combinations would require millions of synthetic sequences.

An example of the ELP program and its program output is provided in the part "ANNEX" of the present description ("ANNEX", pages 1 to 105, after the figure sheets).

The content of this Annex forms part of this disclosure.

### Modifications and other embodiments

Various modifications and variations of the described methods as the concept of the invention will be apparent to those skilled in the art without departing from the scope and spirit of the invention. Although the invention has been described in connection with specific preferred embodiments, it should be understood that the invention as claimed is not intended to be limited to such specific embodiments. Various modifications of the described modes for carrying out the invention which are obvious to those skilled in the computer and programming arts, informatics, molecular biological, biological, chemical, medical, pharmaceutical or related fields are intended to be within the scope of the following claims.

### ANNEX

### Example of the ELP program and its program output

#!/local/bin/perl5.6.0

use FileHandle;
use Getopt::Long;
autoflush STDOUT;

die "wrong number of arguments\n" unless (@ARGV==1);
my ($file_name1) = @ARGV;


my %cg = ();
my @seq;
my @ln;
open IN,"<$file_name1";
warn "Reading $file_name1\n";

while(<IN>){

   $line = $_;
   if ($line =~ /^\>/) {
      $name = $line ;
   }
   else {
       @seq = split (//, $line);
   }
}
print "The initial sequence is $name \n";
print join ("" , @seq), "\n\n";
close IN;


######################### GENETIC CODE ########################################

my(%gc) = (

   'TCA' => 'S',    # Serine
   'TCC' => 'S',    # Serine
   'TCG' => 'S',    # Serine
   'TCT' => 'S',    # Serine
   'TTC' => 'F',    # Phenylalanine
   'TTT' => 'F',    # Phenylalanine
   'TTA' => 'L',    # Leucine
   'TTG' => 'L',    # Leucine
   'TAC' => 'Y',    # Tyrosine
   'TAT' => 'Y',    # Tyrosine
   'TAA' => 'STOP',    # Stop
   'TAG' => 'STOP',    # Stop
   'TGC' => 'C',    # Cysteine
   'TGT' => 'C',    # Cysteine
   'TGA' => 'STOP',    # Stop
   'TGG' => 'W',    # Tryptophan
   'CTA' => 'L',    # Leucine

'CTC' => 'L',   # Leucine
'CTG' => 'L',   # Leucine
'CTT' => 'L',   # Leucine
'CCA' => 'P',   # Proline
'CCC' => 'P',   # Proline
'CCG' => 'P',   # Proline
'CCT' => 'P',   # Proline
'CAC' => 'H',   # Histidine
'CAT' => 'H',   # Histidine
'CAA' => 'Q',   # Glutamine
'CAG' => 'Q',   # Glutamine
'CGA' => 'R',   # Arginine
'CGC' => 'R',   # Arginine
'CGG' => 'R',   # Arginine
'CGT' => 'R',   # Arginine
'ATA' => 'I',   # Isoleucine
'ATC' => 'I',   # Isoleucine
'ATT' => 'I',   # Isoleucine
'ATG' => 'M',   # Methionine
'ACA' => 'T',   # Threonine
'ACC' => 'T',   # Threonine
'ACG' => 'T',   # Threonine
'ACT' => 'T',   # Threonine
'AAC' => 'N',   # Asparagine
'AAT' => 'N',   # Asparagine
'AAA' => 'K',   # Lysine
'AAG' => 'K',   # Lysine
'AGC' => 'S',   # Serine
'AGT' => 'S',   # Serine
'AGA' => 'R',   # Arginine
'AGG' => 'R',   # Arginine
'GTA' => 'V',   # Valine
'GTC' => 'V',   # Valine
'GTG' => 'V',   # Valine
'GTT' => 'V',   # Valine
'GCA' => 'A',   # Alanine
'GCC' => 'A',   # Alanine
'GCG' => 'A',   # Alanine
'GCT' => 'A',   # Alanine
'GAC' => 'D',   # Aspartic Acid
'GAT' => 'D',   # Aspartic Acid
'GAA' => 'E',   # Glutamic Acid
'GAG' => 'E',   # Glutamic Acid
'GGA' => 'G',   # Glycine
'GGC' => 'G',   # Glycine
'GGG' => 'G',   # Glycine
'GGT' => 'G',   # Glycine
);

#################### USE OF E.Coli CODONS ##############################

my %uc = (

'TCA' => '13.10',   # Serine
'TCC' => '14.78',   # Serine
'TCG' => '15.21',   # Serine

'TCT' => '14.47', # Serine
'TTC' => '42.35', # Phenylalanine
'TTT' => '57.65', # Phenylalanine
'TTA' => '13.51', # Leucine
'TTG' => '13.70', # Leucine
'TAC' => '43.09', # Tyrosine
'TAT' => '56.91', # Tyrosine
'TAA' => '60.46', # Stop
'TAG' => '8.90', # Stop
'TGC' => '56.60', # Cysteine
'TGT' => '43.40', # Cysteine
'TGA' => '30.64', # Stop
'TGG' => '100', # Tryptophan
'CTA' => '3.86'", # Leucine
'CTC' => '10.48', # Leucine
'CTG' => '47.78', # Leucine
'CTT' => '10.67', # Leucine
'CCA' => '19.54', # Proline
'CCC' => '12.93', # Proline
'CCG' => '51.06', # Proline
'CCT' => '16.48', # Proline
'CAC' => '42.74', # Histidine
'CAT' => '57.26', # Histidine
'CAA' => '34.85', # Glutamine
'CAG' => '65.15', # Glutamine
'CGA' => '6.88', # Arginine
'CGC' => '38.44', # Arginine
'CGG' => '10.78', # Arginine
'CGT' => '35.64', # Arginine
'ATA' => '8.77', # Isoleucine
'ATC' => '41.40', # Isoleucine
'ATT' => '49.83', # Isoleucine
'ATG' => '100', # Methionine
'ACA' => '13.95', # Threonine
'ACC' => '42.37', # Threonine
'ACG' => '26.95', # Threonine
'ACT' => '16.74', # Threonine
'AAC' => '54.22', # Asparagine
'AAT' => '45.78', # Asparagine
'AAA' => '75.97', # Lysine
'AAG' => '24.03', # Lysine
'AGC' => '27.27', # Serine
'AGT' => '15.17', # Serine
'AGA' => '4.90', # Arginine
'AGG' => '3.36', # Arginine
'GTA' => '15.66', # Valine
'GTC' => '21.55', # Valine
'GTG' => '36.65', # Valine
'GTT' => '26.14', # Valine
'GCA' => '21.45', # Alanine
'GCC' => '26.96', # Alanine
'GCG' => '35.26', # Alanine
'GCT' => '16.33', # Alanine
'GAC' => '37.01', # Aspartic Acid
'GAT' => '62.99', # Aspartic Acid
'GAA' => '68.75', # Glutamic Acid
'GAG' => '31.25', # Glutamic Acid

'GGA' => '11.42',   # Glycine
'GGC' => '39.97',   # Glycine
'GGG' => '15.13',   # Glycine
'GGT' => '33.47',   # Glycine

);

##########################################################################################
####

my $l;
my $nbrnt;
my $nbrcodons;
my $pourcentage;
my $nbrGC = 0;

$nbrnt = scalar (@seq);
$nbrcodons = $nbrnt / 3;
if ( int($nbrcodons) != $nbrcodons){
   die "\nThe sequence contains extra bases which are not part of a codon...\nPlease check it
and try again !\n";
}
print "This sequence is $nbrnt bp long which corresponds to $nbrcodons codons \n\n";

foreach $seq (@seq){
      if ($seq eq G or $seq eq C){
         $nbrGC += 1;
      }
}
$pourcentage = ($nbrGC / $nbrnt)*100;
print "%G+C = $pourcentage \n\n";

##########################################################################################
##########

my $i = 0;
my $j = 2;
my $k = 0;

my $cod;
my $aa;
my $codcount;

my @scod;
my @allcod;
my @base;      #table of nitrogenated bases
my @newseq;
my @pe;
my @hypermatrix;
my @bestpot =();
my @taillecolcod = ();
my @codcountinf = ();
my @forbcoduc =();
my @sauvpotinf = ();
my @sauvpotinf2 = ();

my @anciencod = ();
my @choix =();

@allcod = keys %gc;
@base = ('A','T','G','C');
my @nbrcodalt =();

MAIN:
while ($i < scalar (@seq)){

###################### DEFINITION OF ALTERNATIVE CODONS
######################################

my $l;
my $m;
my $nt;          # aa encoded by codon
my $sauv;          # safeguard of nt
my @ancienaa = ();
my @nouveauaa = ();
my @potevo = ();
my $temoin4;
my $temoin3;
my $temoin2;
my $temoin;
my $pot;
my $countucmax = 0;
my $countucmin = 100;
my $forbcod;

    $codcount = 0;
    $taillecolcod[$k] = 0;

    @scod = @seq[$i..$j];
    $cod = join ('', @scod);
    $aa = $gc{$cod};

    push (@{$anciencod[0]}, $cod);

        foreach $allcod(@allcod){

            if ($gc{$allcod} eq "$aa"){
                if ($uc{$allcod} > $countucmax) {
                    $countucmax = $uc{$allcod};
                }
                if ($uc{$allcod} < $countucmin) {
                    $countucmin = $uc{$allcod};
                    $forbcod = $allcod;
                }
                if ($allcod ne $cod) {
                    push (@{$hypermatrix[0][$k]}, $allcod);
                    $codcount += 1;
                }
            }
        }

push (@nbrcodalt,$codcount);

if ($codcount == 0) {
   $hypermatrix[1][$k][$h] = "-";
   $hypermatrix[2][$k][$h] = "-";
   $hypermatrix[3][$k][$h] = "-";
}

else {
$l = 0;
if ($countucmax / $countucmin > 6) {
   foreach $forbcoduc (@forbcoduc){
      if ($forbcod eq $forbcoduc){
         $l +=1;
      }
   }
   if ($l == 0){
      push (@forbcoduc , $forbcod);
   }
}

################################## DEFINITION OF OLD AA
########################################

$anciencod[1][$k] = 0;

for ($l = 0 ; $l <= 2 ; $l +=1) {
   $nt = substr ($cod , $l , 1);

   foreach $base(@base) {
         $temoin = 0;

         if ($nt ne $base) {
            substr ($cod , $l , 1) = $base;

           if ($gc{$cod} ne 'STOP'){
            foreach $ancienaa(@ancienaa){
                  if ($gc{$cod} eq $ancienaa){
                     $temoin += 1;
                  }
            }

            if ($temoin == 0) {
               push (@ancienaa , $gc{$cod});
               if ($gc{$cod} ne '$aa'){
                  $anciencod[1][$k] += 1;
               }
            }
           }
           substr ($cod , $l , 1)= $nt;
         }
   }
}

############################## DEFINITION OF EVOLUTIVE POTENTIALS

##########################################

for $h (0 .. $codcount-1) {

$hypermatrix[1][$k][$h] = 0; # $hypermatrix[1] : nber of events reaching to an aa different
from those reachable through the initial aa
$hypermatrix[2][$k][$h] = 0; # $hypermatrix[2] : REP
$hypermatrix[3][$k][$h] = 0; # $hypermatrix[3] : nber of mutational events reaching to an
aa different from STOP and from initial aa
$hypermatrix[4][$k][$h] = 0; # $hypermatrix[4] :AEP(does not take in account the initial
aa)

for $l (0 .. 2) {
$nt = substr ($hypermatrix[0][$k][$h], $l , 1);

foreach $base (@base) {
$temoin2 = 0;
$temoin3 = 0;

if ($nt ne $base) {
substr ($hypermatrix[0][$k][$h] , $l , 1) = $base;

if ( ($gc{$hypermatrix[0][$k][$h]} ne 'STOP') and
($gc{$hypermatrix[0][$k][$h]} ne '$aa') ) {
$hypermatrix[3][$k][$h]+=1;
$temoin4 = 0;
foreach $potevo(@potevo){
if ($gc{$hypermatrix[0][$k][$h]} eq $potevo){
$temoin4 += 1;
}
}
foreach $ancienaa(@ancienaa) {
if ($gc{$hypermatrix[0][$k][$h]} eq $ancienaa) {
$temoin2 +=1;
}
}
if ($temoin4 == 0){
push (@potevo, $gc{$hypermatrix[0][$k][$h]});
$hypermatrix[4][$k][$h] += 1;
}
if ($temoin2 == 0) {
$hypermatrix[1][$k][$h] += 1;
foreach $nouveauaa(@nouveauaa) {
if ($gc{$hypermatrix[0][$k][$h]} eq $nouveauaa) {
$temoin3 +=1;
}
}
if ($temoin3 == 0) {
push (@nouveauaa, $gc{$hypermatrix[0][$k][$h]});
$hypermatrix[2][$k][$h] += 1;
}
}
}
}
}
substr ($hypermatrix[0][$k][$h], $l , 1) = $nt;
}

}
@nouveauaa = ();
@potevo = ();
}

########################## RESIZING THE MATRIX ##################################

$codcount1 = $codcount - 1 ;
$codcount2 = $codcount - 2 ;
my $potinf;

$pot = 0;
for $h (0 .. $codcount1) {
if ($hypermatrix[2][$k][$h] > $pot){
$pot = $hypermatrix[2][$k][$h];
}
}

$potinf = 0;
for $h (0 .. $codcount1) {
if ($hypermatrix[2][$k][$h] > $potinf){
if ($hypermatrix[2][$k][$h] < $pot){
$potinf = $hypermatrix[2][$k][$h];
}
}
}

$l = 0;
$m = 0;
for $h (0 .. $codcount1) {

if ($hypermatrix[2][$k][$h] == $pot){
$l += 1;
$bestpot[0][$k][$l-1] = $hypermatrix[0][$k][$h]; # codons
$bestpot[1][$k][$l-1] = $hypermatrix[2][$k][$h]; # rep
$bestpot[2][$k][$l-1] = $hypermatrix[1][$k][$h]; # nber events
$bestpot[3][$k][$l-1] = $hypermatrix[4][$k][$h]; # aep
}
if ($hypermatrix[2][$k][$h] == $potinf){
$m += 1;
$sauvpotinf[0][$k][$m-1] = $hypermatrix[0][$k][$h]; # codons
$sauvpotinf[1][$k][$m-1] = $hypermatrix[2][$k][$h]; # aep
$sauvpotinf[2][$k][$m-1] = $hypermatrix[1][$k][$h]; # nber events
$sauvpotinf[3][$k][$m-1] = $hypermatrix[4][$k][$h]; # aep
}

}

$taillecolcod[$k] = $l;
$codcountinf[$k] = $m;

if ($taillecolcod[$k] > 0){
push (@choix, $k);
}

}
  $i +=3;
  $j +=3;
  $k +=1;
}

my $nbrseq = 1;
foreach $choix (@choix){
      $nbrseq = $nbrseq * $taillecolcod[$choix];
}

############################## RESULTS DISPLAY
##################################################

my $d = -1;
my $t;
my $botab;

print "-------------------------------------------------------- \n";
print "*                    GENERAL REP TABLE                    * \n";
print "-------------------------------------------------------- \n";
print "\ni.cod = initial codon  ; alt.cod  = alternative codons  ; REP = Relative Evolutionary
Power ; #.event = number of simple mutational events leading to the codon\n";
print "\n     i.cod        alt.cod        REP        #.event  \n";

for $k (0 .. $nbrcodons - 1){
   print "________________________________________________________________\n\n";
   $d += 1;
   $e = 0;
   print "$d \t $anciencod[0][$k] \t\t";
   $botab = scalar (@{$hypermatrix[0][$k]})+1;
   if ($botab eq 1){
         print "  - \t\t- \t\t-";
      }
   for $t (0 .. $botab - 2){
      print "$hypermatrix[0][$k][$t]", "\t\t", "$hypermatrix[2][$k][$t]" , "\t\t",
"$hypermatrix[1][$k][$t]";
      if ($t ne ($botab - 2)){
         print "\n\t\t\t";
      }
   }
   print "\n";
}

$d = -1;
print"\n \n";
print "-------------------------------------------------------- \n";
print "*                    BEST REP TABLE                    * \n";
print "-------------------------------------------------------- \n";
print "\ni.cod = initial codon  ; alt.cod  = alternative codons  ; REP = Relative Evolutionary
Power ; #.event = number of simple mutational events leading to the codon\n";

print "\n      i.cod      alt.cod      REP      #.event \n";

for $k (0 .. $nbrcodons - 1){
   print "________________________________________________________________\n\n";
   $d += 1;
   $e = 0;
   print "$d \t $anciencod[0][$k] \t\t";
   $botab = scalar (@{$bestpot[0][$k]})+1;
   if ($botab eq 1){
         print "  - \t\t- \t\t-";
      }
   for $t (0 .. $botab - 2){
      $e += 1;
      print "$bestpot[0][$k][$t]", "\t\t", "$bestpot[1][$k][$t]" , "\t\t",  "$bestpot[2][$k][$t]";
      if ($t ne ($botab - 2)){
         print "\n\t\t\t";
      }
   }
   print "\n";
}

$d = -1;
print"\n \n";
print "-------------------------------------------------------- \n";
print "*                    'SUB-BEST' REP TABLE                    * \n";
print "-------------------------------------------------------- \n";
print "\ni.cod = initial codon  ; alt.cod  = alternative codons  ; REP = Relative Evolutionary
Power ; #.event = number of simple mutational events leading to the codon\n";
print "\n      i.cod      alt.cod      REP      #.event \n";

for $k (0 .. $nbrcodons - 1){
   print "________________________________________________________________\n\n";
   $d += 1;
   $e = 0;
   print "$d \t $anciencod[0][$k] \t\t";
   $botab = scalar (@{$sauvpotinf[0][$k]})+1;
   if ($botab eq 1){
         print "  - \t\t- \t\t-";
      }
   for $t (0 .. $botab - 2 ){
      print "$sauvpotinf[0][$k][$t]", "\t\t", "$sauvpotinf[1][$k][$t]" , "\t\t",  "$sauvpotinf[2][$k][$t]";
      if ($t ne ($botab - 2)){
         print "\n\t\t\t";
      }
   }
   print "\n";
}



##################### RANDOM CONSTRUCTION OF A SEQUENCE WITH BETTER REP
##############################################


my $countdiffper = 0;

for $l (0 .. $nbrcodons - 1){

if ($taillecolcod[$l] > 0){
   $k = int rand ($taillecolcod[$l]);
   push (@newseq,   $bestpot[0][$l][$k]);
   push (@{$pe[1]}, $bestpot[1][$l][$k]);
   push (@{$pe[2]}, $bestpot[3][$l][$k]);
   if ($bestpot[1][$l][$k] > 0){
      $countdiffper += 1;
   }
}
else {
   push (@newseq, $anciencod[0][$l]);
   push (@{$pe[1]}, 0);
   push (@{$pe[2]}, $anciencod[1][$l]);
}
}

$nbrGC = 0;

foreach $newseq (@newseq){
   for $l (0 .. 2){
      if (substr ($newseq, $l , 1) eq G or substr ($newseq, $l , 1) eq C){
      $nbrGC += 1;
      }
   }
}

my $baduc;
my $testcod;
my $new100;
my $tesp100;
my $plouf = 0;
my $ploufplouf = 0;
my $ploufploufplouf = 0;
my $diff3;
my $badcodsauv;

$new100 = ($nbrGC / $nbrnt) * 100;

print "\n\n--------------------------------------------------------\n\n\n";
print "\nAlternative sequence randomly generated :  %G+C = $new100\n\n";
warn  "\nAlternative sequence randomly generated...\n";

@save = @newseq;

##################### PRELIMINARY ELIMINATION OF FORBIDDEN CODONS
#################

my @badcod = ();
$baduc = 0;
$l = -1;

foreach $newseq (@newseq){
   $l += 1;
   foreach $forbcoduc (@forbcoduc) {
      if ($newseq eq $forbcoduc){

$baduc += 1;
if ($taillecolcod[$I] > 0){
push (@badcod, $I);
}
else {
print "Impossible to replace the forbidden codon $newseq in position $I \n";
}
}
}
}

print "\nForbidden codons : ", join( " ; ", @forbcoduc), "\n\n";
print "The alternative sequence already contains $baduc forbidden codon(s) before optimisation
for %G+C content\n";
print "Incorporating too much weakly used codons in a synthetic sequence would lead to impair
expression of the corresponding protein\n\n";
print "Maximum number of forbidden codons tolerated in the final sequence ? \n";
warn "Maximum number of forbidden codons tolerated in the final sequence ? \n";

my $maxbadcod;
$maxbadcod = <STDIN>;

print "\t$maxbadcod\n";
warn "\n\n";

if ( $baduc > $maxbadcod ) {

my @badcodalea = ();
my @badcodaleachiffre = ();
my $decid1;
my $decid2;
my $decid3;
my $sommedecid = $ba[?]uc;

$decid3 = int rand ($maxbadcod + 1);

print "\n\n$decid3 forbidden codon(s) have been randomly removed\n";

while ($sommedecid > $decid3) {
$decid1 = int rand ($baduc);
$decid2 = int rand (2);
$I = 0;
if ($decid2 eq 1) {
foreach $badcodaleachiffre(@badcodaleachiffre) {
if ($badcodaleachiffre eq $decid1){
$I += 1;
}
}
if ($I eq 0){
$sommedecid -=1;
push (@badcodalea, $badcod[$decid1]);
push (@badcodaleachiffre, $decid1);
}
}
}

foreach $badcodalea (@badcodalea){

$badcodsauv = $newseq[$badcodalea];
$h = 0;
if ($taillecolcod[$badcodalea] > 1) {
while ($newseq[$badcodalea] eq $badcodsauv){
$h+=1;
$l = int rand ($taillecolcod[$badcodalea]);
$newseq[$badcodalea] = $bestpot[0][$badcodalea][$l];
$pe[1][$badcodalea]  = $bestpot[1][$badcodalea][$l];
$pe[2][$badcodalea]  = $bestpot[3][$badcodalea][$l];

if ($h > 30){
print "WARNING : there is a problem the program cannot solve - please press
Ctrl + C to end \n";
die  "\n\nWARNING : there is a problem the program cannot solve - please
press Ctrl + C to end \n\n";
}
}
print "At position $badcodalea, $badcodsauv is replaced by $newseq[$badcodalea] of
equivalent REP\n";
}

else {
while ($newseq[$badcodalea] eq $badcodsauv){
$l = int rand ( $codcountinf[$badcodalea]);
$newseq[$badcodalea] = $sauvpotinf[0][$badcodalea][$l];
$pe[1][$badcodalea]  = $sauvpotinf[1][$badcodalea][$l];
$pe[2][$badcodalea]  = $sauvpotinf[3][$badcodalea][$l];
}
print "At position $badcodalea, $badcodsauv is replaced by $newseq[$badcodalea] of
REP imediately inferior to maximum REP \n";
}
}
}


$nbrGC = 0;
foreach $newseq (@newseq){
for $l (0 .. 2){
if (substr ($newseq, $l , 1) eq G or substr ($newseq, $l , 1) eq C){
$nbrGC += 1;
}
};
}

$new100 = ($nbrGC / $nbrnt) * 100;
print "\n%G+C is now : $new100 \n\n";
warn  "%G+C = $new100 \n";


################################ CALCULATION OF THE %GC REACHABLE
DOMAIN################################



@save = @newseq;
my $save100 = $new100;

my @seqmin;
my @seqmax;
my $testcodmin;
my $testcodmax;
my $new100min;
my $new100max;
my $testp100min;
my $testp100max;
@seqmin = @newseq;
@seqmax = @newseq;
$new100min = $new100;
$new100max = $new100;


foreach $choix (@choix){
        for $l ( 0..($taillecolcod[$choix]-1) ) {
          $testcodmin =  $seqmin[$choix];
          $testcodmax =  $seqmax[$choix];
          $seqmin[$choix] = $bestpot[0][$choix][$l];
          $seqmax[$choix] = $bestpot[0][$choix][$l];

          $nbrGC = 0;
          foreach $seqmin (@seqmin){
              for $m (0 .. 2){
                if (substr ($seqmin, $m , 1) eq G or substr ($seqmin, $m , 1) eq C){
                $nbrGC += 1;
                }
              }
          }
          $testp100min = ($nbrGC / $nbrnt) * 100;

          $nbrGC = 0;
          foreach $seqmax (@[?]eqmax){
              for $m (0 .. 2){
                if (substr ($seqmax, $m , 1) eq G or substr ($seqmax, $m , 1) eq C){
                  $nbrGC += 1;
                }
              }
          }
          $testp100max = ($nbrGC / $nbrnt) * 100;

          if ( $testp100min < $new100min ) {
            $new100min = $testp100min;
          }
          else {
            $seqmin[$choix] = $testcodmin;
          }

          if ( $testp100max > $new100max ) {
            $new100max = $testp100max;
          }
          else {
            $seqmax[$choix] = $testcodmax;
          }
        }
}

print "Domain of reachable %G+C : [$new100min , $new100max]\n\n" ;
warn "Domain of reachable %G+C : [ $new100min , $new100max ]\n";

############################ TOWARDS THE DESIRED %GC
####################################

print "Final %G+C desired ?";
warn "Final %G+C desired ?\n";
my $GCf;
$GCf = <STDIN>;
print "\t$GCf\n";

print "Error allowed ?";
warn  "Error allowed ?\n";
my $seuil;
$seuil = <STDIN>;
print "\t$seuil\n";

print "\n\n";

my @subnewseq = ();
my $subnew100 = 0;
my $subtestp100 = 0;
my $subnbrGC = 0;
my $subnbrnt = 0;
my $abs = 100;
my $go_on;
my $xtrem;
my $localuc = $nbrecodons;

@newseq = @save;
$diff3 = abs($new100 - $GCf);
if ((100-$GCf) > ($GCf)){      # definition of local limits of %G+C
  $xtrem = $GCf/2;
}
else {
  $xtrem = (100-$GCf)/2;
}

while ( ($diff3 > $seuil) or ($localuc > 1) ) {

            $ploufploufplouf = int rand (scalar (@choix));
            $ploufplouf = $choix[$ploufploufplouf];

##########################################################################################
##########
            $i = $ploufplouf - 15;
            $j = $ploufplouf + 15;
            if ($i < 0){
               $i = 0;
            }

if ($j > $nbrcodons - 1){
  $j = $nbrcodons - 1;
}
$subnbrnt = $j - $i + 1;
$subnbrnt = $subnbrnt*3;
@subnewseq = @newseq[$i..$j];
$subnbrGC = 0;
foreach $subnewseq (@subnewseq){
    for $m (0..2){
        if (substr ($subnewseq, $m , 1) eq G or substr ($subnewseq, $m , 1) eq C){
            $subnbrGC += 1;
        }
    }
}
$subnew100 = ($subnbrGC / $subnbrnt) * 100;

##########################################################################################
############

$plouf   = int rand ($taillecolcod[$ploufplouf]);
$testcod =  $newseq[$ploufplouf];
$newseq[$ploufplouf] = $bestpot[0][$ploufplouf][$plouf];

if ($newseq[$ploufplouf] ne $testcod) {

  $baduc = 0;
  foreach $forbcoduc (@forbcoduc) {
    if ($newseq[$ploufplouf] eq $forbcoduc){
      $baduc += 1;
    }
    if ($baduc > 0){
      foreach $newseq (@newseq){
          foreach $forbcoduc (@forbcoduc) {
              if ($newseq eq $forbcoduc){
                $baduc += 1;
              }
          }
      }
      if ($baduc >= $maxbadcod) {
        $go_on = 0;
        print " At position $ploufplouf, $testcod is replaced by the weakly used
$newseq[$ploufplouf]\n";
        print " -> change is REJECTED : no more forbidden codons can be
incorporated\n\n";
        $newseq[$ploufplouf] = $testcod;
      }
      else {
        $go_on = 2;
      }
    }
    else {
      $go_on = 1;
    }
  }

  if ($go_on > 0){

$nbrGC = 0;
foreach $newseq (@newseq){
    for $l (0 .. 2){
        if (substr ($newseq, $l , 1) eq G or substr ($newseq, $l , 1) eq C){
        $nbrGC += 1;
        }
    }
}
$testp100 = ($nbrGC / $nbrnt) * 100;


$subnbrGC = 0;
@subnewseq = @newseq[$i..$j];
foreach $subnewseq (@subnewseq){
    for $m (0 .. 2){
        if (substr ($subnewseq, $m, 1) eq G or substr ($subnewseq, $m, 1) eq C){
        $subnbrGC += 1;
        }
    }
}
$subtestp100 = ($subnbrGC / $subnbrnt) * 100;


if ($go_on = 1){
    print "At position $ploufplouf, $testcod is replaced by $newseq[$ploufplouf]\n";
}
if ($go_on = 2){
    print " At position $ploufplouf, $testcod is replaced by the weakly used
$newseq[$ploufplouf]\n";
}
print " Globally the %G+C switch from $new100 to $testp100 \n ";
print " Locally the %G+C switch from $subnew100 to $subtestp100 \n ";

my $diff = ($testp100 - $GCf );
my $diff2 = ($new100 - $GCf);
my $abs = abs $diff;
my $abs2 = abs $diff2;

my $subdiff  = ($subtestp100 - $GCf);
my $subdiff2 = ($subnew100 - $GCf);
my $subabs   = abs $subdiff;
my $subabs2  = abs $subdiff2;

if (( $abs <= $abs2 ) and ($subabs <= $subabs2)) {
    $new100 = $testp100;
    $diff3 = abs($new100 - $GCf);
    $pe[1][$ploufplouf]  = $bestpot[1][$ploufplouf][$plouf];
    $pe[2][$ploufplouf]  = $bestpot[3][$ploufplouf][$plouf];
    print " -> Change is ACCEPTED\n\n";
}

else {
    $newseq[$ploufplouf] = $testcod;
    print " -> Change is REJECTED\n\n";
}
}
}

else {
print " At position $ploufplouf, $testcod is replaced by $newseq[$ploufplouf] : no
change\n\n";
}

$localuc = 0;
for $h (0..($nbrcodons-1)){
$i = $h - 15;
$j = $h + 15;
if ($i < 0){
$i = 0;
}
if ($j > $nbrcodons - 1){
$j = $nbrcodons - 1;
}
$subnbrnt = $j - $i + 1;
$subnbrnt = $subnbrnt*3;
@subnewseq = @newseq[$i..$j];
$subnbrGC = 0;
foreach $subnewseq (@subnewseq){
for $m (0..2){
if (substr ($subnewseq, $m , 1) eq G or substr ($subnewseq, $m , 1) eq C){
$subnbrGC += 1;
}
}
}
$subnew100 = ($subnbrGC / $subnbrnt) * 100;


if ( ($subnew100 < $xtrem) or ($subnew100 > (100 - $xtrem)) ){
$localuc += 1;
}
}
}



################################## ALTERNATIVE FINAL SEQUENCE
########################################


my @finalseq;
foreach $newseq (@newseq){
for ($l = 0 ; $l <= 2 ; $l +=1) {
$nt = substr ($newseq, $l , 1);
push (@finalseq, $nt);
}
}
my $homo = 0;
for $l (0..$nbrnt - 1){
if ($finalseq[$l] eq $seq[$l]){
$homo += 1;
}
}
$homo = $homo * 100 / $nbrnt;

################################# CALCULATION OF MEAN AND STOCHASTIC AEP
####################################

my $peaseq = 0;
my $perseq = 0;
my $peainit = 0;

for $i (1..($nbrcodons - 2)){
      $peainit += $anciencod[1][$i];
}

for $i (1 .. ($nbrcodons - 2)){
      $peaseq += $pe[2][$i];
      $perseq += $pe[1][$i];
}

$peainit = $peainit / ($nbrcodons - 2);
$peaseq = $peaseq / ($nbrcodons - 2);
$perseq = $perseq / ($nbrcodons - 2);

my $moy;
my $std;
my $o;
my $k;
my @aleape = ();
my @peaseqalea = ();
my @pealea =();
my @aleaseq=();
my $stat = 0 * ($nbrcodons - 1);
@pea = ();

print "\n\nYou can calculate statistics regarding AEP and REP by generating random
synonymous sequences ?\n";
print "How many interations do you wish for these calculations ? (no more than 10000 is
strongly recommended...)\n";
warn "\n\nThe program can calculate statistics regarding AEP and REP by generating random
synonymous sequences ?\n";
warn "How many interations do you wish for these calculations ? (no more than 10000 is
strongly recommended...)\n";
my $stat;
$stat = <STDIN>;
print "\t$stat\n";

for $o (1 .. $stat ){

   @aleaseq = ();

   for $d (1 .. ($nbrcodons - 2)){
      if (scalar (@{$hypermatrix[0][$d]}) > 0){
         $k = 0;
         $k = int rand ( scalar (@{$hypermatrix[0][$d]}));
         push (@aleaseq, $hypermatrix[0][$d][$k]);
         push (@{$aleape[1]}, $hypermatrix[2][$d][$k]);
         push (@{$aleape[2]}, $hypermatrix[4][$d][$k]);

}
      else {
         push (@aleaseq, $anciencod[0][$d]);
         push (@{$aleape[1]}, 0);
         push (@{$aleape[2]}, $anciencod[1][$l]);
      }
   }

   for $i (1 .. ($nbrcodons - 2)){
      $peseqalea[1][$o] += $aleape[1][$i];
      $peseqalea[2][$o] += $aleape[2][$i];
   }
   $peseqalea[1][$o] = $peseqalea[1][$o] / ($nbrcodons - 2);
   $peseqalea[2][$o] = $peseqalea[2][$o] / ($nbrcodons - 2);
}

$moypea = 0;
$stdpea = 0;
$moyper = 0;
$stdper = 0;

for $i (1 .. $stat){
      $moypea += $peseqalea[2][$i];
      $moyper += $peseqalea[1][$i];
}
$moypea = $moypea / $stat;
$moyper = $moyper / $stat;

for $i (1 .. $stat){
      $stdpea += ($peseqalea[2][$i] - $moypea)*($peseqalea[2][$i] - $moypea);
      $stdper += ($peseqalea[1][$i] - $moyper)*($peseqalea[1][$i] - $moyper);
}
   $stdpea = sqrt($stdpea / $stat);
   $stdper = sqrt($stdper / $stat);



################################## RESULTS
##############################################

print "--------------------------------------------------------\n";
print "*                    FINAL SEQUENCE                    *\n";
print "--------------------------------------------------------\n\n";

print "A synonymous sequence of maximum REP with respect to the initial sequence is :\n\n";

print join ("",@newseq),"\n\n\n";

print "--------------------------------------------------------\n";
print "*                       FEATURES                       *\n";
print "--------------------------------------------------------\n\n";

print "%GC = $new100\n";
print "Number of codon with different PER     : $countdiffper\n";
print "Number of different best REP sequences : $nbrseq\n";
print "Similarity with the initial sequence   : $homo\n\n";

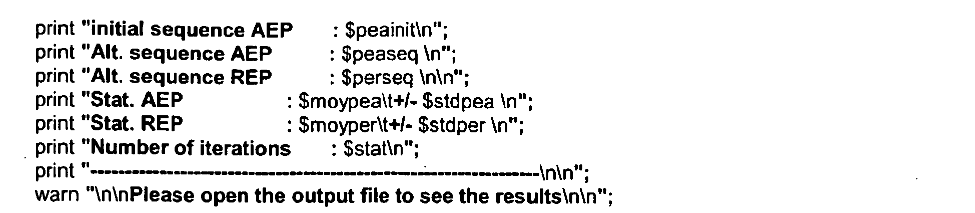

The initial sequence is >AWT 
ATGACCAACAGCAACGATTCCGTCACACTGCGCCTCATGACTGAGCATGACCTT
GCGATGCTCTATGAGTGGCTAAATCGATCTCATATCGTCGAGTGGTGGGCGGA
GAAGAAGCACGCCCGACACTTGCTGACGTACAGGAACAGTACTTGCCAAGCGTT
TTAGCGCAAGAGTCCGTCACTCCATACATTGCAATGCTGAATGGAGAGCCGATT
GGGTATGCCCAGTCGTACGTTGCTCTTGGAAGCGGGGACGGATGGTGGGAAGA
AGAAACCGATCCAGGAGTACGCGGAATAGACCAGTTACTGGCGAATGCATCACA
ACTGGGCAAAGGCTTGGGAACCAAGCTGGTTCGAGCTCTGGTTGAGTTGCTGTT
CAATGATCCCGAGGTCACCAAGATCCAAACGGACCCGTCGCCGAGCAACTTGC
GAGCGATCCGATGCTACGAGAAAGCGGGGTTTGAGAGGCAAGGTACCGTAACC
ACCCCAGATGGTCCAGCCGTGTACATGGTTCAAACACGCCAGGCATTCGAGCGA
ACACGCAGTGATGCCTAA
 This sequence is 555 bp long which corresponds to 185 codons
%G+C = 54.2342342342342

| * | GENERAL REP TABLE | | * | |
|---|---|---|---|---|
| i.cod = initial codon ; alt.cod = alternative codons ; REP = Relative Evolutionary Power ; #.event = number of simple mutational events leading to the codon | | | | |
| i.cod | alt.cod | REP | #.event | |
| 0 | ATG | - | - | - |
| 1 | ACC | ACA | 2 | 2 |
| | | ACG | 3 | 3 |
| | | ACT | 0 | 0 |
| 2 | AAC | AAT | 0 | 0 |
| 3 | AGC | AGT | 0 | 0 |
| | | TCG | 4 | 4 |
| | | TCA | 3 | 3 |
| | | TCC | 4 | 4 |
| | | TCT | 4 | 4 |
| 4 | AAC | AAT | 0 | 0 |
| 5 | GAT | GAC | 0 | 0 |
| 6 | TCC | AGT | 4 | 6 |
| | | AGC | 4 | 6 |
| | | TCG | 2 | 2 |
| | | TCA | 1 | 1 |
| | | TCT | 0 | 0 |
| 7 | GTC | GTG | 2 | 2 |
| | | GTT | 0 | 0 |
| | | GTA | 1 | 1 |
| 8 | ACA | ACC | 1 | 1 |
| | | ACG | 1 | 1 |
| | | ACT | 1 | 1 |
| 9 | CTG | CTC | 3 | 3 |
| | | TTG | 3 | 4 |
| | | CTT | 3 | 3 |
| | | TTA | 3 | 4 |
| | | CTA | 1 | 1 |
| 10 | CGC | CGA | 1 | 1 |
| | | AGA | 3 | 3 |
| | | AGG | 4 | 4 |
| | | CGT | 0 | 0 |
| | | CGG | 2 | 2 |
| 11 | CTC | CTG | 2 | 2 |
| | | TTG | 3 | 3 |
| | | CTT | 0 | 0 |
| | | TTA | 1 | 1 |
| | | CTA | 1 | 1 |
| 12 | ATG | - | - | - |
| 13 | ACT | ACA | 2 | 2 |
| | | ACC | 0 | 0 |
| | | ACG | 3 | 3 |
| 14 | GAG | GAA | 0 | 0 |
| 15 | CAT | CAC | 0 | 0 |
| 16 | GAC | GAT | 0 | 0 |
| 17 | CTT | CTG | 2 | 2 |
| | | CTC | 0 | 0 |
| | | TTG | 3 | 3 |
| | | TTA | 1 | 1 |
| | | CTA | 1 | 1 |
| 18 | GCG | GCC | 1 | 1 |
| | | GCA | 0 | 0 |
| | | GCT | 1 | 1 |
| 19 | ATG | - | - | - |
| 20 | CTC | CTG | 2 | 2 |
| | | TTG | 3 | 3 |
| | | CTT | 0 | 0 |
| | | TTA | 1 | 1 |
| | | CTA | 1 | 1 |
| 21 | TAT | TAC | 0 | 0 |
| 22 | GAG | GAA | 0 | 0 |
| 23 | TGG | - | - | - |
| 24 | CTA | CTG | 1 | 1 |
| | | CTC | 2 | 2 |
| | | TTG | 4 | 5 |
| | | CTT | 2 | 2 |
| | | TTA | 2 | 3 |
| 25 | AAT | AAC | 0 | 0 |
| 26 | CGA | AGA | 4 | 5 |
| | | CGC | 3 | 3 |
| | | AGG | 5 | 6 |
| | | CGT | 3 | 3 |
| | | CGG | 1 | 1 |
| 27 | TCT | AGT | 4 | 6 |
| | | AGC | 4 | 6 |
| | | TCG | 2 | 2 |
| | | TCA | 1 | 1 |
| | | TCC | 0 | 0 |
| 28 | CAT | CAC | 0 | 0 |
| 29 | ATC | ATT | 0 | 0 |
| | | ATA | 2 | 2 |
| 30 | GTC | GTG | 2 | 2 |
| | | GTT | 0 | 0 |
| | | GTA | 1 | 1 |
| 31 | GAG | GAA | 0 | 0 |
| 32 | TGG | - | - | - |
| 33 | TGG | - | - | - |
| 34 | GGC | GGA | 1 | 1 |
| | | GGT | 0 | 0 |
| | | GGG | 2 | 2 |
| 35 | GGA | GGT | 3 | 3 |
| | | GGG | 1 | 1 |
| | | GGC | 3 | 3 |
| 36 | GAA | GAG | 0 | 0 |
| 37 | GAA | GAG | 0 | 0 |
| 38 | GCA | GCC | 1 | 1 |
| | | GCG | 0 | 0 |
| | | GCT | 1 | 1 |
| 39 | CGC | CGA | 1 | 1 |
| | | AGA | 3 | 3 |
| | | AGG | 4 | 4 |
| | | CGT | 0 | 0 |
| | | CGG | 2 | 2 |
| 40 | CCG | CCA | 0 | 0 |
| | | CCC | 1 | 1 |
| | | CCT | 1 | 1 |
| 41 | ACA | ACC | 1 | 1 |
| | | ACG | 1 | 1 |
| | | ACT | 1 | 1 |
| 42 | CTT | CTG | 2 | 2 |
| | | CTC | 0 | 0 |
| | | TTG | 3 | 3 |
| | | TTA | 1 | 1 |
| | | CTA | 1 | 1 |
| 43 | GCT | GCC | 0 | 0 |
| | | GCA | 1 | 1 |
| | | GCG | 1 | 1 |
| 44 | GAC | GAT | 0 | 0 |
| 45 | GTA | GTG | 1 | 1 |
| | | GTT | 2 | 2 |
| | | GTC | 2 | 2 |
| 46 | CAG | CAA | 0 | 0 |
| 47 | GAA | GAG | 0 | 0 |
| 48 | CAG | CAA | 0 | 0 |
| 49 | TAC | TAT | 0 | 0 |
| 50 | TTG | CTG | 3 | 3 |
| | | CTC | 4 | 4 |
| | | CTT | 4 | 4 |
| | | TTA | 1 | 1 |
| | | CTA | 4 | 4 |
| 51 | CCA | CCG | 0 | 0 |
| | | CCC | 1 | 1 |
| | | CCT | 1 | 1 |
| 52 | AGC | AGT | 0 | 0 |
| | | TCG | 4 | 4 |
| | | TCA | 3 | 3 |
| | | TCC | 4 | 4 |
| | | TCT | 4 | 4 |
| 53 | GTT | GTG | 2 | 2 |
| | | GTA | 1 | 1 |
| | | GTC | 0 | 0 |
| 54 | TTA | CTG | 4 | 4 |
| | | CTC | 3 | 3 |
| | | TTG | 2 | 2 |
| | | CTT | 3 | 3 |
| | | CTA | 3 | 3 |
| 55 | GCG | GCC | 1 | 1 |
| | | GCA | 0 | 0 |
| | | GCT | 1 | 1 |
| 56 | CAA | CAG | 0 | 0 |
| 57 | GAG | GAA | 0 | 0 |
| 58 | TCC | AGT | 4 | 6 |
| | | AGC | 4 | 6 |
| | | TCG | 2 | 2 |
| | | TCA | 1 | 1 |
| | | TCT | 0 | 0 |
| 59 | GTC | GTG | 2 | 2 |
| | | GTT | 0 | 0 |
| | | GTA | 1 | 1 |
| 60 | ACT | ACA | 2 | 2 |
| | | ACC | 0 | 0 |
| | | ACG | 3 | 3 |
| 61 | CCA | CCG | 0 | 0 |
| | | CCC | 1 | 1 |
| | | CCT | 1 | 1 |
| 62 | TAC | TAT | 0 | 0 |
| 63 | ATT | ATC | 0 | 0 |
| | | ATA | 2 | 2 |
| 64 | GCA | GCC | 1 | 1 |
| | | GCG | 0 | 0 |
| | | GCT | 1 | 1 |
| 65 | ATG | - | - | - |
| 66 | CTG | CTC | 3 | 3 |
| | | TTG | 3 | 4 |
| | | CTT | 3 | 3 |
| | | TTA | 3 | 4 |
| | | CTA | 1 | 1 |
| 67 | AAT | AAC | 0 | 0 |
| 68 | GGA | GGT | 3 | 3 |
| | | GGG | 1 | 1 |
| | | GGC | 3 | 3 |
| 69 | GAG | GAA | 0 | 0 |
| 70 | CCG | CCA | 0 | 0 |
| | | CCC | 1 | 1 |
| | | CCT | 1 | 1 |
| 71 | ATT | ATC | 0 | 0 |
| | | ATA | 2 | 2 |
| 72 | GGG | GGA | 0 | 0 |
| | | GGT | 3 | 3 |
| | | GGC | 3 | 3 |
| 73 | TAT | TAC | 0 | 0 |
| 74 | GCC | GCA | 1 | 1 |
| | | GCG | 1 | 1 |
| | | GCT | 0 | 0 |
| 75 | CAG | CAA | 0 | 0 |
| 76 | TCG | AGT | 5 | 7 |
| | | AGC | 5 | 7 |
| | | TCA | 0 | 0 |
| | | TCC | 3 | 3 |
| | | TCT | 3 | 3 |
| 77 | TAC | TAT | 0 | 0 |
| 78 | GTT | GTG | 2 | 2 |
| | | GTA | 1 | 1 |
| | | GTC | 0 | 0 |
| 79 | GCT | GCC | 0 | 0 |
| | | GCA | 1 | 1 |
| | | GCG | 1 | 1 |
| 80 | CTT | CTG | 2 | 2 |
| | | CTC | 0 | 0 |
| | | TTG | 3 | 3 |
| | | TTA | 1 | 1 |
| | | CTA | 1 | 1 |
| 81 | GGA | GGT | 3 | 3 |
| | | GGG | 1 | 1 |
| | | GGC | 3 | 3 |
| 82 | AGC | AGT | 0 | 0 |
| | | TCG | 4 | 4 |
| | | TCA | 3 | 3 |
| | | TCC | 4 | 4 |
| | | TCT | 4 | 4 |
| 83 | GGG | GGA | 0 | 0 |
| | | GGT | 3 | 3 |
| | | GGC | 3 | 3 |
| 84 | GAC | GAT | 0 | 0 |
| 85 | GGA | GGT | 3 | 3 |
| | | GGG | 1 | 1 |
| | | GGC | 3 | 3 |
| 86 | TGG | - | - | - |
| 87 | TGG | - | - | - |
| 88 | GAA | GAG | 0 | 0 |
| 89 | GAA | GAG | 0 | 0 |
| 90 | GAA | GAG | 0 | 0 |
| 91 | ACC | ACA | 2 | 2 |
| | | ACG | 3 | 3 |
| | | ACT | 0 | 0 |
| 92 | GAT | GAC | 0 | 0 |
| 93 | CCA | CCG | 0 | 0 |
| | | CCC | 1 | 1 |
| | | CCT | 1 | 1 |
| 94 | GGA | GGT | 3 | 3 |
| | | GGG | 1 | 1 |
| | | GGC | 3 | 3 |
| 95 | GTA | GTG | 1 | 1 |
| | | GTT | 2 | 2 |
| | | GTC | 2 | 2 |
| 96 | CGC | CGA | 1 | 1 |
| | | AGA | 3 | 3 |
| | | AGG | 4 | 4 |
| | | CGT | 0 | 0 |
| | | CGG | 2 | 2 |
| 97 | GGA | GGT | 3 | 3 |
| | | GGG | 1 | 1 |
| | | GGC | 3 | 3 |
| 98 | ATA | ATC | 3 | 3 |
| | | ATT | 3 | 3 |
| 99 | GAC | GAT | 0 | 0 |
| 100 | CAG | CAA | 0 | 0 |
| 101 | TTA | CTG | 4 | 4 |
| | | CTC | 3 | 3 |
| | | TTG | 2 | 2 |
| | | CTT | 3 | 3 |
| | | CTA | 3 | 3 |
| 102 | CTG | CTC | 3 | 3 |
| | | TTG | 3 | 4 |
| | | CTT | 3 | 3 |
| | | TTA | 3 | 4 |
| | | CTA | 1 | 1 |
| 103 | GCG | GCC | 1 | 1 |
| | | GCA | 0 | 0 |
| | | GCT | 1 | 1 |
| 104 | AAT | AAC | 0 | 0 |
| 105 | GCA | GCC | 1 | 1 |
| | | GCG | 0 | 0 |
| | | GCT | 1 | 1 |
| 106 | TCA | AGT | 5 | 7 |
| | | AGC | 5 | 7 |
| | | TCG | 1 | 1 |
| | | TCC | 3 | 3 |
| | | TCT | 3 | 3 |
| 107 | CAA | CAG | 0 | 0 |
| 108 | CTG | CTC | 3 | 3 |
| | | TTG | 3 | 4 |
| | | CTT | 3 | 3 |
| | | TTA | 3 | 4 |
| | | CTA | 1 | 1 |
| 109 | GGC | GGA | 1 | 1 |
| | | GGT | 0 | 0 |
| | | GGG | 2 | 2 |
| 110 | AAA | AAG | 1 | 1 |
| 111 | GGC | GGA | 1 | 1 |
| | | GGT | 0 | 0 |
| | | GGG | 2 | 2 |
| 112 | TTG | CTG | 3 | 3 |
| | | CTC | 4 | 4 |
| | | CTT | 4 | 4 |
| | | TTA | 1 | 1 |
| | | CTA | 4 | 4 |
| 113 | GGA | GGT | 3 | 3 |
| | | GGG | 1 | 1 |
| | | GGC | 3 | 3 |
| 114 | ACC | ACA | 2 | 2 |
| | | ACG | 3 | 3 |
| | | ACT | 0 | 0 |
| 115 | AAG | AAA | 1 | 1 |
| 116 | CTG | CTC | 3 | 3 |
| | | TTG | 3 | 4 |
| | | CTT | 3 | 3 |
| | | TTA | 3 | 4 |
| | | CTA | 1 | 1 |
| 117 | GTT | GTG | 2 | 2 |
| | | GTA | 1 | 1 |
| | | GTC | 0 | 0 |
| 118 | CGA | AGA | 4 | 5 |
| | | CGC | 3 | 3 |
| | | AGG | 5 | 6 |
| | | CGT | 3 | 3 |
| | | CGG | 1 | 1 |
| 119 | GCT | GCC | 0 | 0 |
| | | GCA | 1 | 1 |
| | | GCG | 1 | 1 |
| 120 | CTG | CTC | 3 | 3 |
| | | TTG | 3 | 4 |
| | | CTT | 3 | 3 |
| | | TTA | 3 | 4 |
| | | CTA | 1 | 1 |
| 121 | GTT | GTG | 2 | 2 |
| | | GTA | 1 | 1 |
| | | GTC | 0 | 0 |
| 122 | GAG | GAA | 0 | 0 |
| 123 | TTG | CTG | 3 | 3 |
| | | CTC | 4 | 4 |
| | | CTT | 4 | 4 |
| | | TTA | 1 | 1 |
| | | CTA | 4 | 4 |
| 124 | CTG | CTC | 3 | 3 |
| | | TTG | 3 | 4 |
| | | CTT | 3 | 3 |
| | | TTA | 3 | 4 |
| | | CTA | 1 | 1 |
| 125 | TTC | TTT | 0 | 0 |
| 126 | AAT | AAC | 0 | 0 |
| 127 | GAT | GAC | 0 | 0 |
| 128 | CCC | CCG | 1 | 1 |
| | | CCA | 1 | 1 |
| | | CCT | 0 | 0 |
| 129 | GAG | GAA | 0 | 0 |
| 130 | GTC | GTG | 2 | 2 |
| | | GTT | 0 | 0 |
| | | GTA | 1 | 1 |
| 131 | ACC | ACA | 2 | 2 |
| | | ACG | 3 | 3 |
| | | ACT | 0 | 0 |
| 132 | AAG | AAA | 1 | 1 |
| 133 | ATC | ATT | 0 | 0 |
| | | ATA | 2 | 2 |
| 134 | CAA | CAG | 0 | 0 |
| 135 | ACG | ACA | 1 | 1 |
| | | ACC | 2 | 2 |
| | | ACT | 2 | 2 |
| 136 | GAC | GAT | 0 | 0 |
| 137 | CCG | CCA | 0 | 0 |
| | | CCC | 1 | 1 |
| | | CCT | 1 | 1 |
| 138 | TCG | AGT | 5 | 7 |
| | | AGC | 5 | 7 |
| | | TCA | 0 | 0 |
| | | TCC | 3 | 3 |
| | | TCT | 3 | 3 |
| 139 | CCG | CCA | 0 | 0 |
| | | CCC | 1 | 1 |
| | | CCT | 1 | 1 |
| 140 | AGC | AGT | 0 | 0 |
| | | TCG | 4 | 4 |
| | | TCA | 3 | 3 |
| | | TCC | 4 | 4 |
| | | TCT | 4 | 4 |
| 141 | AAC | AAT | 0 | 0 |
| 142 | TTG | CTG | 3 | 3 |
| | | CTC | 4 | 4 |
| | | CTT | 4 | 4 |
| | | TTA | 1 | 1 |
| | | CTA | 4 | 4 |
| 143 | CGA | AGA | 4 | 5 |
| | | CGC | 3 | 3 |
| | | AGG | 5 | 6 |
| | | CGT | 3 | 3 |
| | | CGG | 1 | 1 |
| 144 | GCG | GCC | 1 | 1 |
| | | GCA | 0 | 0 |
| | | GCT | 1 | 1 |
| 145 | ATC | ATT | 0 | 0 |
| | | ATA | 2 | 2 |
| 146 | CGA | AGA | 4 | 5 |
| | | CGC | 3 | 3 |
| | | AGG | 5 | 6 |
| | | CGT | 3 | 3 |
| | | CGG | 1 | 1 |
| 147 | TGC | TGT | 0 | 0 |
| 148 | TAC | TAT | 0 | 0 |
| 149 | GAG | GAA | 0 | 0 |
| 150 | AAA | AAG | 1 | 1 |
| 151 | GCG | GCC | 1 | 1 |
| | | GCA | 0 | 0 |
| | | GCT | 1 | 1 |
| 152 | GGG | GGA | 0 | 0 |
| | | GGT | 3 | 3 |
| | | GGC | 3 | 3 |
| 153 | TTT | TTC | 0 | 0 |
| 154 | GAG | GAA | 0 | 0 |
| 155 | AGG | CGA | 3 | 3 |
| | | AGA | 1 | 1 |
| | | CGC | 4 | 4 |
| | | CGT | 4 | 4 |
| | | CGG | 3 | 3 |
| 156 | CAA | CAG | 0 | 0 |
| 157 | GGT | GGA | 1 | 1 |
| | | GGG | 2 | 2 |
| | | GGC | 0 | 0 |
| 158 | ACC | ACA | 2 | 2 |
| | | ACG | 3 | 3 |
| | | ACT | 0 | 0 |
| 159 | GTA | GTG | 1 | 1 |
| | | GTT | 2 | 2 |
| | | GTC | 2 | 2 |
| 160 | ACC | ACA | 2 | 2 |
| | | ACG | 3 | 3 |
| | | ACT | 0 | 0 |
| 161 | ACC | ACA | 2 | 2 |
| | | ACG | 3 | 3 |
| | | ACT | 0 | 0 |
| 162 | CCA | CCG | 0 | 0 |
| | | CCC | 1 | 1 |
| | | CCT | 1 | 1 |
| 163 | GAT | GAC | 0 | 0 |
| 164 | GGT | GGA | 1 | 1 |
| | | GGG | 2 | 2 |
| | | GGC | 0 | 0 |
| 165 | CCA | CCG | 0 | 0 |
| | | CCC | 1 | 1 |
| | | CCT | 1 | 1 |
| 166 | GCC | GCA | 1 | 1 |
| | | GCG | 1 | 1 |
| | | GCT | 0 | 0 |
| 167 | GTG | GTT | 3 | 3 |
| | | GTA | 1 | 1 |
| | | GTC | 3 | 3 |
| 168 | TAC | TAT | 0 | 0 |
| 169 | ATG | - | - | - |
| 170 | GTT | GTG | 2 | 2 |
| | | GTA | 1 | 1 |
| | | GTC | 0 | 0 |
| 171 | CAA | CAG | 0 | 0 |
| 172 | ACA | ACC | 1 | 1 |
| | | ACG | 1 | 1 |
| | | ACT | 1 | 1 |
| 173 | CGC | CGA | 1 | 1 |
| | | AGA | 3 | 3 |
| | | AGG | 4 | 4 |
| | | CGT | 0 | 0 |
| | | CGG | 2 | 2 |
| 174 | CAG | CAA | 0 | 0 |
| 175 | GCA | GCC | 1 | 1 |
| | | GCG | 0 | 0 |
| | | GCT | 1 | 1 |
| 176 | TTC | TTT | 0 | 0 |
| 177 | GAG | GAA | 0 | 0 |
| 178 | CGA | AGA | 4 | 5 |
| | | CGC | 3 | 3 |
| | | AGG | 5 | 6 |
| | | CGT | 3 | 3 |
| | | CGG | 1 | 1 |
| 179 | ACA | ACC | 1 | 1 |
| | | ACG | 1 | 1 |
| | | ACT | 1 | 1 |
| 180 | CGC | CGA | 1 | 1 |
| | | AGA | 3 | 3 |
| | | AGG | 4 | 4 |
| | | CGT | 0 | 0 |
| | | CGG | 2 | 2 |
| 181 | AGT | AGC | 0 | 0 |
| | | TCG | 4 | 4 |
| | | TCA | 3 | 3 |
| | | TCC | 4 | 4 |
| | | TCT | 4 | 4 |
| 182 | GAT | GAC | 0 | 0 |
| 183 | GCC | GCA | 1 | 1 |
| | | GCG | 1 | 1 |
| | | GCT | 0 | 0 |
| 184 | TAA | TGA | 4 | 6 |
| | | TAG | 1 | 1 |

| * | BEST REP TABLE | | * | |
|---|---|---|---|---|
| i.cod = initial codon ; alt.cod = alternative codons ; REP = Relative Evolutionary Power; #.event = number of simple mutational events leading to the codon | | | | |
| i.cod | alt.cod | REP | #.event | |
| 0 | ATG | - | - | - |
| 1 | ACC | ACG | 3 | 3 |
| 2 | AAC | AAT | 0 | 0 |
| 3 | AGC | TCG | 4 | 4 |
| | | TCC | 4 | 4 |
| | | TCT | 4 | 4 |
| 4 | AAC | AAT | 0 | 0 |
| 5 | GAT | GAC | 0 | 0 |
| 6 | TCC | AGT | 4 | 6 |
| | | AGC | 4 | 6 |
| 7 | GTC | GTG | 2 | 2 |
| 8 | ACA | ACC | 1 | 1 |
| | | ACG | 1 | 1 |
| | | ACT | 1 | 1 |
| 9 | CTG | CTC | 3 | 3 |
| | | TTG | 3 | 4 |
| | | CTT | 3 | 3 |
| | | TTA | 3 | 4 |
| 10 | CGC | AGG | 4 | 4 |
| 11 | CTC | TTG | 3 | 3 |
| 12 | ATG | - | - | - |
| 13 | ACT | ACG | 3 | 3 |
| 14 | GAG | GAA | 0 | 0 |
| 15 | CAT | CAC | 0 | 0 |
| 16 | GAC | GAT | 0 | 0 |
| 17 | CTT | TTG | 3 | 3 |
| 18 | GCG | GCC | 1 | 1 |
| | | GCT | 1 | 1 |
| 19 | ATG | - | - | - |
| 20 | CTC | TTG | 3 | 3 |
| 21 | TAT | TAC | 0 | 0 |
| 22 | GAG | GAA | 0 | 0 |
| 23 | TGG | - | - | - |
| 24 | CTA | TTG | 4 | 5 |
| 25 | AAT | AAC | 0 | 0 |
| 26 | CGA | AGG | 5 | 6 |
| 27 | TCT | AGT | 4 | 6 |
| | | AGC | 4 | 6 |
| 28 | CAT | CAC | 0 | 0 |
| 29 | ATC | ATA | 2 | 2 |
| 30 | GTC | GTG | 2 | 2 |
| 31 | GAG | GAA | 0 | 0 |
| 32 | TGG | - | - | - |
| 33 | TGG | - | - | - |
| 34 | GGC | GGG | 2 | 2 |
| 35 | GGA | GGT | 3 | 3 |
| | | GGC | 3 | 3 |
| 36 | GAA | GAG | 0 | 0 |
| 37 | GAA | GAG | 0 | 0 |
| 38 | GCA | GCC | 1 | 1 |
| | | GCT | 1 | 1 |
| 39 | CGC | AGG | 4 | 4 |
| 40 | CCG | CCC | 1 | 1 |
| | | CCT | 1 | 1 |
| 41 | ACA | ACC | 1 | 1 |
| | | ACG | 1 | 1 |
| | | ACT | 1 | 1 |
| 42 | CTT | TTG | 3 | 3 |
| 43 | GCT | GCA | 1 | 1 |
| | | GCG | 1 | 1 |
| 44 | GAC | GAT | 0 | 0 |
| 45 | GTA | GTT | 2 | 2 |
| | | GTC | 2 | 2 |
| 46 | CAG | CAA | 0 | 0 |
| 47 | GAA | GAG | 0 | 0 |
| 48 | CAG | CAA | 0 | 0 |
| 49 | TAC | TAT | 0 | 0 |
| 50 | TTG | CTC | 4 | 4 |
| | | CTT | 4 | 4 |
| | | CTA | 4 | 4 |
| 51 | CCA | CCC | 1 | 1 |
| | | CCT | 1 | 1 |
| 52 | AGC | TCG | 4 | 4 |
| | | TCC | 4 | 4 |
| | | TCT | 4 | 4 |
| 53 | GTT | GTG | 2 | 2 |
| 54 | TTA | CTG | 4 | 4 |
| 55 | GCG | GCC | 1 | 1 |
| | | GCT | 1 | 1 |
| 56 | CAA | CAG | 0 | 0 |
| 57 | GAG | GAA | 0 | 0 |
| 58 | TCC | AGT | 4 | 6 |
| | | AGC | 4 | 6 |
| 59 | GTC | GTG | 2 | 2 |
| 60 | ACT | ACG | 3 | 3 |
| 61 | CCA | CCC | 1 | 1 |
| | | CCT | 1 | 1 |
| 62 | TAC | TAT | 0 | 0 |
| 63 | ATT | ATA | 2 | 2 |
| 64 | GCA | GCC | 1 | 1 |
| | | GCT | 1 | 1 |
| 65 | ATG | - | - | - |
| 66 | CTG | CTC | 3 | 3 |
| | | TTG | 3 | 4 |
| | | CTT | 3 | 3 |
| | | TTA | 3 | 4 |
| 67 | AAT | AAC | 0 | 0 |
| 68 | GGA | GGT | 3 | 3 |
| | | GGC | 3 | 3 |
| 69 | GAG | GAA | 0 | 0 |
| 70 | CCG | CCC | 1 | 1 |
| | | CCT | 1 | 1 |
| 71 | ATT | ATA | 2 | 2 |
| 72 | GGG | GGT | 3 | 3 |
| | | GGC | 3 | 3 |
| 73 | TAT | TAC | 0 | 0 |
| 74 | GCC | GCA | 1 | 1 |
| | | GCG | 1 | 1 |
| 75 | CAG | CAA | 0 | 0 |
| 76 | TCG | AGT | 5 | 7 |
| | | AGC | 5 | 7 |
| 77 | TAC | TAT | 0 | 0 |
| 78 | GTT | GTG | 2 | 2 |
| 79 | GCT | GCA | 1 | 1 |
| | | GCG | 1 | 1 |
| 80 | CTT | TTG | 3 | 3 |
| 81 | GGA | GGT | 3 | 3 |
| | | GGC | 3 | 3 |
| 82 | AGC | TCG | 4 | 4 |
| | | TCC | 4 | 4 |
| | | TCT | 4 | 4 |
| 83 | GGG | GGT | 3 | 3 |
| | | GGC | 3 | 3 |
| 84 | GAC | GAT | 0 | 0 |
| 85 | GGA | GGT | 3 | 3 |
| | | GGC | 3 | 3 |
| 86 | TGG | - | - | - |
| 87 | TGG | - | - | |
| 88 | GAA | GAG | 0 | 0 |
| 89 | GAA | GAG | 0 | 0 |
| 90 | GAA | GAG | 0 | 0 |
| 91 | ACC | ACG | 3 | 3 |
| 92 | GAT | GAC | 0 | 0 |
| 93 | CCA | CCC | 1 | 1 |
| | | CCT | 1 | 1 |
| 94 | GGA | GGT | 3 | 3 |
| | | GGC | 3 | 3 |
| 95 | GTA | GTT | 2 | 2 |
| | | GTC | 2 | 2 |
| 96 | CGC | AGG | 4 | 4 |
| 97 | GGA | GGT | 3 | 3 |
| | | GGC | 3 | 3 |
| 98 | ATA | ATC | 3 | 3 |
| | | ATT | 3 | 3 |
| 99 | GAC | GAT | 0 | 0 |
| 100 | CAG | CAA | 0 | 0 |
| 101 | TTA | CTG | 4 | 4 |
| 102 | CTG | CTC | 3 | 3 |
| | | TTG | 3 | 4 |
| | | CTT | 3 | 3 |
| | | TTA | 3 | 4 |
| 103 | GCG | GCC | 1 | 1 |
| | | GCT | 1 | 1 |
| 104 | AAT | AAC | 0 | 0 |
| 105 | GCA | GCC | 1 | 1 |
| | | GCT | 1 | 1 |
| 106 | TCA | AGT | 5 | 7 |
| | | AGC | 5 | 7 |
| 107 | CAA | CAG | 0 | 0 |
| 108 | CTG | CTC | 3 | 3 |
| | | TTG | 3 | 4 |
| | | CTT | 3 | 3 |
| | | TTA | 3 | 4 |
| 109 | GGC | GGG | 2 | 2 |
| 110 | AAA | AAG | 1 | 1 |
| 111 | GGC | GGG | 2 | 2 |
| 112 | TTG | CTC | 4 | 4 |
| | | CTT | 4 | 4 |
| | | CTA | 4 | 4 |
| 113 | GGA | GGT | 3 | 3 |
| | | GGC | 3 | 3 |
| 114 | ACC | ACG | 3 | 3 |
| 115 | AAG | AAA | 1 | 1 |
| 116 | CTG | CTC | 3 | 3 |
| | | TTG | 3 | 4 |
| | | CTT | 3 | 3 |
| | | TTA | 3 | 4 |
| 117 | GTT | GTG | 2 | 2 |
| 118 | CGA | AGG | 5 | 6 |
| 119 | GCT | GCA | 1 | 1 |
| | | GCG | 1 | 1 |
| 120 | CTG | CTC | 3 | 3 |
| | | TTG | 3 | 4 |
| | | CTT | 3 | 3 |
| | | TTA | 3 | 4 |
| 121 | GTT | GTG | 2 | 2 |
| 122 | GAG | GAA | 0 | 0 |
| 123 | TTG | CTC | 4 | 4 |
| | | CTT | 4 | 4 |
| | | CTA | 4 | 4 |
| 124 | CTG | CTC | 3 | 3 |
| | | TTG | 3 | 4 |
| | | CTT | 3 | 3 |
| | | TTA | 3 | 4 |
| 125 | TTC | TTT | 0 | 0 |
| 126 | AAT | AAC | 0 | 0 |
| 127 | GAT | GAC | 0 | 0 |
| 128 | CCC | CCG | 1 | 1 |
| | | CCA | 1 | 1 |
| 129 | GAG | GAA | 0 | 0 |
| 130 | GTC | GTG | 2 | 2 |
| 131 | ACC | ACG | 3 | 3 |
| 132 | AAG | AAA | 1 | 1 |
| 133 | ATC | ATA | 2 | 2 |
| 134 | CAA | CAG | 0 | 0 |
| 135 | ACG | ACC | 2 | 2 |
| | | ACT | 2 | 2 |
| 136 | GAC | GAT | 0 | 0 |
| 137 | CCG | CCC | 1 | 1 |
| | | CCT | 1 | 1 |
| 138 | TCG | AGT | 5 | 7 |
| | | AGC | 5 | 7 |
| 139 | CCG | CCC | 1 | 1 |
| | | CCT | 1 | 1 |
| 140 | AGC | TCG | 4 | 4 |
| | | TCC | 4 | 4 |
| | | TCT | 4 | 4 |
| 141 | AAC | AAT | 0 | 0 |
| 142 | TTG | CTC | 4 | 4 |
| | | CTT | 4 | 4 |
| | | CTA | 4 | 4 |
| 143 | CGA | AGG | 5 | 6 |
| 144 | GCG | GCC | 1 | 1 |
| | | GCT | 1 | 1 |
| 145 | ATC | ATA | 2 | 2 |
| 146 | CGA | AGG | 5 | 6 |
| 147 | TGC | TGT | 0 | 0 |
| 148 | TAC | TAT | 0 | 0 |
| 149 | GAG | GAA | 0 | 0 |
| 150 | AAA | AAG | 1 | 1 |
| 151 | GCG | GCC | 1 | 1 |
| | | GCT | 1 | 1 |
| 152 | GGG | GGT | 3 | 3 |
| | | GGC | 3 | 3 |
| 153 | TTT | TTC | 0 | 0 |
| 154 | GAG | GAA | 0 | 0 |
| 155 | AGG | CGC | 4 | 4 |
| | | CGT | 4 | 4 |
| 156 | CAA | CAG | 0 | 0 |
| 157 | GGT | GGG | 2 | 2 |
| 158 | ACC | ACG | 3 | 3 |
| 159 | GTA | GTT | 2 | 2 |
| | | GTC | 2 | 2 |
| 160 | ACC | ACG | 3 | 3 |
| 161 | ACC | ACG | 3 | 3 |
| 162 | CCA | CCC | 1 | 1 |
| | | CCT | 1 | 1 |
| 163 | GAT | GAC | 0 | 0 |
| 164 | GGT | GGG | 2 | 2 |
| 165 | CCA | CCC | 1 | 1 |
| | | CCT | 1 | 1 |
| 166 | GCC | GCA | 1 | 1 |
| | | GCG | 1 | 1 |
| 167 | GTG | GTT | 3 | 3 |
| | | GTC | 3 | 3 |
| 168 | TAC | TAT | 0 | 0 |
| 169 | ATG | - | - | - |
| 170 | GTT | GTG | 2 | 2 |
| 171 | CAA | CAG | 0 | 0 |
| 172 | ACA | ACC | 1 | 1 |
| | | ACG | 1 | 1 |
| | | ACT | 1 | 1 |
| 173 | CGC | AGG | 4 | 4 |
| 174 | CAG | CAA | 0 | 0 |
| 175 | GCA | GCC | 1 | 1 |
| | | GCT | 1 | 1 |
| 176 | TTC | TTT | 0 | 0 |
| 177 | GAG | GAA | 0 | 0 |
| 178 | CGA | AGG | 5 | 6 |
| 179 | ACA | ACC | 1 | 1 |
| | | ACG | 1 | 1 |
| | | ACT | 1 | 1 |
| 180 | CGC | AGG | 4 | 4 |
| 181 | AGT | TCG | 4 | 4 |
| | | TCC | 4 | 4 |
| | | TCT | 4 | 4 |
| 182 | GAT | GAC | 0 | 0 |
| 183 | GCC | GCA | 1 | 1 |
| | | GCG | 1 | 1 |
| 184 | TAA | TGA | 4 | 6 |

| * | 'SUB-BEST' REP TABLE | | * | |
|---|---|---|---|---|
| i.cod = initial codon ; alt.cod = alternative codons ; REP = Relative Evolutionary Power; #.event = number of simple mutational events leading to the codon | | | | |
| i.cod | alt.cod | REP | #.event | |
| 0 | ATG | - | - | - |
| 1 | ACC | ACA | 2 | 2 |
| 2 | AAC | AAT | 0 | 0 |
| 3 | AGC | TCA | 3 | 3 |
| 4 | AAC | AAT | 0 | 0 |
| 5 | GAT | GAC | 0 | 0 |
| 6 | TCC | TCG | 2 | 2 |
| 7 | GTC | GTA | 1 | 1 |
| 8 | ACA | - | - | - |
| 9 | CTG | CTA | 1 | 1 |
| 10 | CGC | AGA | 3 | 3 |
| 11 | CTC | CTG | 2 | 2 |
| 12 | ATG | - | - | - |
| 13 | ACT | ACA | 2 | 2 |
| 14 | GAG | GAA | 0 | 0 |
| 15 | CAT | CAC | 0 | 0 |
| 16 | GAC | GAT | 0 | 0 |
| 17 | CTT | CTG | 2 | 2 |
| 18 | GCG | GCA | 0 | 0 |
| 19 | ATG | - | - | - |
| 20 | CTC | CTG | 2 | 2 |
| 21 | TAT | TAC | 0 | 0 |
| 22 | GAG | GAA | 0 | 0 |
| 23 | TGG | - | - | - |
| 24 | CTA | CTC | 2 | 2 |
| | | CTT | 2 | 2 |
| | | TTA | 2 | 3 |
| 25 | AAT | AAC | 0 | 0 |
| 26 | CGA | AGA | 4 | 5 |
| 27 | TCT | TCG | 2 | 2 |
| 28 | CAT | CAC | 0 | 0 |
| 29 | ATC | ATT | 0 | 0 |
| 30 | GTC | GTA | 1 | 1 |
| 31 | GAG | GAA | 0 | 0 |
| 32 | TGG | - | - | - |
| 33 | TGG | - | - | - |
| 34 | GGC | GGA | 1 | 1 |
| 35 | GGA | GGG | 1 | 1 |
| 36 | GAA | GAG | 0 | 0 |
| 37 | GAA | GAG | 0 | 0 |
| 38 | GCA | GCG | 0 | 0 |
| 39 | CGC | AGA | 3 | 3 |
| 40 | CCG | CCA | 0 | 0 |
| 41 | ACA | - | - | - |
| 42 | CTT | CTG | 2 | 2 |
| 43 | GCT | GCC | 0 | 0 |
| 44 | GAC | GAT | 0 | 0 |
| 45 | GTA | GTG | 1 | 1 |
| 46 | CAG | CAA | 0 | 0 |
| 47 | GAA | GAG | 0 | 0 |
| 48 | CAG | CAA | 0 | 0 |
| 49 | TAC | TAT | 0 | 0 |
| 50 | TTG | CTG | 3 | 3 |
| 51 | CCA | CCG | 0 | 0 |
| 52 | AGC | TCA | 3 | 3 |
| 53 | GTT | GTA | 1 | 1 |
| 54 | TTA | CTC | 3 | 3 |
| | | CTT | 3 | 3 |
| | | CTA | 3 | 3 |
| 55 | GCG | GCA | 0 | 0 |
| 56 | CAA | CAG | 0 | 0 |
| 57 | GAG | GAA | 0 | 0 |
| 58 | TCC | TCG | 2 | 2 |
| 59 | GTC | GTA | 1 | 1 |
| 60 | ACT | ACA | 2 | 2 |
| 61 | CCA | CCG | 0 | 0 |
| 62 | TAC | TAT | 0 | 0 |
| 63 | ATT | ATC | 0 | 0 |
| 64 | GCA | GCG | 0 | 0 |
| 65 | ATG | - | - | - |
| 66 | CTG | CTA | 1 | 1 |
| 67 | AAT | AAC | 0 | 0 |
| 68 | GGA | GGG | 1 | 1 |
| 69 | GAG | GAA | 0 | 0 |
| 70 | CCG | CCA | 0 | 0 |
| 71 | ATT | ATC | 0 | 0 |
| 72 | GGG | GGA | 0 | 0 |
| 73 | TAT | TAC | 0 | 0 |
| 74 | GCC | GCT | 0 | 0 |
| 75 | CAG | CAA | 0 | 0 |
| 76 | TCG | TCC | 3 | 3 |
| | | TCT | 3 | 3 |
| 77 | TAC | TAT | 0 | 0 |
| 78 | GTT | GTA | 1 | 1 |
| 79 | GCT | GCC | 0 | 0 |
| 80 | CTT | CTG | 2 | 2 |
| 81 | GGA | GGG | 1 | 1 |
| 82 | AGC | TCA | 3 | 3 |
| 83 | GGG | GGA | 0 | 0 |
| 84 | GAC | GAT | 0 | 0 |
| 85 | GGA | GGG | 1 | 1 |
| 86 | TGG | - | - | - |
| 87 | TGG | - | - | - |
| 88 | GAA | GAG | 0 | 0 |
| 89 | GAA | GAG | 0 | 0 |
| 90 | GAA | GAG | 0 | 0 |
| 91 | ACC | ACA | 2 | 2 |
| 92 | GAT | GAC | 0 | 0 |
| 93 | CCA | CCG | 0 | 0 |
| 94 | GGA | GGG | 1 | 1 |
| 95 | GTA | GTG | 1 | 1 |
| 96 | CGC | AGA | 3 | 3 |
| 97 | GGA | GGG | 1 | 1 |
| 98 | ATA | - | - | - |
| 99 | GAC | GAT | 0 | 0 |
| 100 | CAG | CAA | 0 | 0 |
| 101 | TTA | CTC | 3 | 3 |
| | | CTT | 3 | 3 |
| | | CTA | 3 | 3 |
| 102 | CTG | CTA | 1 | 1 |
| 103 | GCG | GCA | 0 | 0 |
| 104 | AAT | AAC | 0 | 0 |
| 105 | GCA | GCG | 0 | 0 |
| 106 | TCA | TCC | 3 | 3 |
| | | TCT | 3 | 3 |
| 107 | CAA | CAG | 0 | 0 |
| 108 | CTG | CTA | 1 | 1 |
| 109 | GGC | GGA | 1 | 1 |
| 110 | AAA | - | - | - |
| 111 | GGC | GGA | 1 | 1 |
| 112 | TTG | CTG | 3 | 3 |
| 113 | GGA | GGG | 1 | 1 |
| 114 | ACC | ACA | 2 | 2 |
| 115 | AAG | - | - | - |
| 116 | CTG | CTA | 1 | 1 |
| 117 | GTT | GTA | 1 | 1 |
| 118 | CGA | AGA | 4 | 5 |
| 119 | GCT | GCC | 0 | 0 |
| 120 | CTG | CTA | 1 | 1 |
| 121 | GTT | GTA | 1 | 1 |
| 122 | GAG | GAA | 0 | 0 |
| 123 | TTG | CTG | 3 | 3 |
| 124 | CTG | CTA | 1 | 1 |
| 125 | TTC | TTT | 0 | 0 |
| 126 | AAT | AAC | 0 | 0 |
| 127 | GAT | GAC | 0 | 0 |
| 128 | CCC | CCT | 0 | 0 |
| 129 | GAG | GAA | 0 | 0 |
| 130 | GTC | GTA | 1 | 1 |
| 131 | ACC | ACA | 2 | 2 |
| 132 | AAG | - | - | - |
| 133 | ATC | ATT | 0 | 0 |
| 134 | CAA | CAG | 0 | 0 |
| 135 | ACG | ACA | 1 | 1 |
| 136 | GAC | GAT | 0 | 0 |
| 137 | CCG | CCA | 0 | 0 |
| 138 | TCG | TCC | 3 | 3 |
| | | TCT | 3 | 3 |
| 139 | CCG | CCA | 0 | 0 |
| 140 | AGC | TCA | 3 | 3 |
| 141 | AAC | AAT | 0 | 0 |
| 142 | TTG | CTG | 3 | 3 |
| 143 | CGA | AGA | 4 | 5 |
| 144 | GCG | GCA | 0 | 0 |
| 145 | ATC | ATT | 0 | 0 |
| 146 | CGA | AGA | 4 | 5 |
| 147 | TGC | TGT | 0 | 0 |
| 148 | TAC | TAT | 0 | 0 |
| 149 | GAG | GAA | 0 | 0 |
| 150 | AAA | - | - | - |
| 151 | GCG | GCA | 0 | 0 |
| 152 | GGG | GGA | 0 | 0 |
| 153 | TTT | TTC | 0 | 0 |
| 154 | GAG | GAA | 0 | 0 |
| 155 | AGG | CGA | 3 | 3 |
| | | CGG | 3 | 3 |
| 156 | CAA | CAG | 0 | 0 |
| 157 | GGT | GGA | 1 | 1 |
| 158 | ACC | ACA | 2 | 2 |
| 159 | GTA | GTG | 1 | 1 |
| 160 | ACC | ACA | 2 | 2 |
| 161 | ACC | ACA | 2 | 2 |
| 162 | CCA | CCG | 0 | 0 |
| 163 | GAT | GAC | 0 | 0 |
| 164 | GGT | GGA | 1 | 1 |
| 165 | CCA | CCG | 0 | 0 |
| 166 | GCC | GCT | 0 | 0 |
| 167 | GTG | GTA | 1 | 1 |
| 168 | TAC | TAT | 0 | 0 |
| 169 | ATG | - | - | - |
| 170 | GTT | GTA | 1 | 1 |
| 171 | CAA | CAG | 0 | 0 |
| 172 | ACA | - | - | - |
| 173 | CGC | AGA | 3 | 3 |
| 174 | CAG | CAA | 0 | 0 |
| 175 | GCA | GCG | 0 | 0 |
| 176 | TTC | TTT | 0 | 0 |
| 177 | GAG | GAA | 0 | 0 |
| 178 | CGA | AGA | 4 | 5 |
| 179 | ACA | - | - | - |
| 180 | CGC | AGA | 3 | 3 |
| 181 | AGT | TCA | 3 | 3 |
| 182 | GAT | GAC | 0 | 0 |
| 183 | GCC | GCT | 0 | 0 |
| 184 | TAA | TAG | 1 | 1 |

Alternative sequence randomly generated : %G+C = 52.7927927927928
Forbidden codons : CTA ; AGG ; TAG
The alternative sequence already contains 11 forbidden codon(s) before optimisation for %G+C content
   Incorporating too much weakly used codons in a synthetic sequence would lead to impair expression of the corresponding protein
Maximum number of forbidden codons tolerated in the final sequence ? 4
3 forbidden codon(s) have been randomly removed
   At position 180, AGG is replaced by AGA of REP imediately inferior to maximum REP
   At position 146, AGG is replaced by AGA of REP imediately inferior to maximum REP
   At position 178, AGG is replaced by AGA of REP imediately inferior to maximum REP
   At position 173, AGG is replaced by AGA of REP imediately inferior to maximum REP
   At position 96, AGG is replaced by AGA of REP imediately inferior to maximum REP
   At position 143, AGG is replaced by AGA of REP imediately inferior to maximum REP
   At position 123, CTA is replaced by CTT of equivalent REP
   At position 118, AGG is replaced by AGA of REP imediately inferior to maximum REP
%G+C is now : 51.531531-5315315
Domain of reachable %G+C : [45.4054054054054 , 60.18018018018021
Final %G+C desired ? 48
Error allowed ? 0.5
At position 58, AGC is replaced by AGC : no change
At position 53, GTG is replaced by GTG : no change
At position 97, GGT is replaced by GGC
   At position 97, GGT is replaced by the weakly used GGC
   Globally the %G+C switch from 51.5315315315315 to 51.7117117117117
   Locally the %G+C switch from 55.9139784946236 to 56.989247311828
   -> Change is REJECTED
At position 103, GCT is replaced by GCT : no change
At position 136, GAT is replaced by GAT : no change
At position 20, TTG is replaced by TTG : no change
At position 26, AGG is replaced by AGG : no changeJ At position 130, GTG is replaced by GTG : no changeJ At position 140, TCC is replaced by TCG
   At position 140, TCC is replaced by the weakly used TCG
   Globally the %G+C switch from 51.5315315315315 to 51.5315315315315
   Locally the %G+C switch from 46.2365591397849 to 46.2365591397849
   -> Change is ACCEPTED
At position 114, ACG is replaced by ACG : no change
At position 28, CAC is replaced by CAC : no change
At position 8, ACG is replaced by ACG : no change
At position 157, GGG is replaced by GGG : no change
At position 58, AGC is replaced by AGT
   At position 58, AGC is replaced by the weakly used AGT
   Globally the %G+C switch from 51.5315315315315 to 51.3513513513513
   Locally the %G+C switch from 49.4623655913978 to 48.3870967741936
   -> Change is ACCEPTED
At position 50, CTT is replaced by the weakly used CTA
   -> change is REJECTED : no more forbidden codons can be incorporated
At position 50, CTT is replaced by the weakly used CTT
   -> change is REJECTED : no more forbidden codons can be incorporated
At position 50, CTT is replaced by the weakly used CTT
   -> change is REJECTED : no more forbidden codons can be incorporated
At position 54, CTG is replaced by CTG : no change
At position 81, GGT is replaced by GGT : no change
At position 68, GGT is replaced by GGT : no change
At position 51, CCT is replaced by CCC
   At position 51, CCT is replaced by the weakly used CCC
   Globally the %G+C switch from 51.3513513513513 to 51.5315315315315
   Locally the %G+C switch from 51.6129032258064 to 52.6881720430108
   -> Change is REJECTED
At position 34, GGG is replaced by GGG : no change
At position 148, TAT is replaced by TAT : no change
At position 63, ATA is replaced by ATA : no change
At position 155, CGC is replaced by CGC : no change
At position 115, AAA is replaced by AAA : no change
At position 147, TGT is replaced by TGT : no change
At position 111, GGG is replaced by GGG : no change
At position 174, CAA is replaced by CAA : no change
At position 69, GAA is replaced by GAA : no change
At position 175, GCT is replaced by GCC
   At position 175, GCT is replaced by the weakly used GCC
   Globally the %G+C switch from 51.3513513513513 to 51.5315315315315
   Locally the %G+C switch from 56 to 57.3333333333333
   -> Change is REJECTED
At position 52, TCT is replaced by TCG
   At position 52, TCT is replaced by the weakly used TCG
   Globally the %G+C switch from 51.3513513513513 to 51.5315315315315
   Locally the %G+C switch from 50.5376344086022 to 51.6129032258064
   -> Change is REJECTED
At position 157, GGG is replaced by GGG : no change
At position 54, CTG is replaced by CTG : no change
At position 73, TAC is replaced by TAC : no change
At position 168, TAT is replaced by TAT : no change
At position 154, GAA is replaced by GAA : no change
At position 69, GAA is replaced by GAA : no change
At position 52, TCT is replaced by TCT : no change
At position 140, TCG is replaced by TCC
   At position 140, TCG is replaced by the weakly used TCC
   Globally the %G+C switch from 51.3513513513513 to 51.3513513513513
   Locally the %G+C switch from 46.2365591397849 to 46.2365591397849
   -> Change is ACCEPTED
At position 79, GCA is replaced by GCA : no change
At position 43, GCA is replaced by GCG
   At position 43, GCA is replaced by the weakly used GCG
   Globally the %G+C switch from 51.3513513513513 to 51.5315315315315
   Locally the %G+C switch from 55.9139784946236 to 56.989247311828
   -> Change is REJECTED
At position 168, TAT is replaced by TAT : no change
At position 106, AGT is replaced by AGT : no change
At position 60, ACG is replaced by ACG : no change
At position 62, TAT is replaced by TAT : no change
At position 95, GTT is replaced by GTT : no change
At position 27, AGT is replaced by AGT : no change
At position 173, AGA is replaced by the weakly used AGG
-> change is REJECTED : no more forbidden codons can be incorporated
At position 173, AGA is replaced by the weakly used AGA
   -> change is REJECTED : no more forbidden codons can be incorporated
At position 153, TTC is replaced by TTC : no change
At position 77, TAT is replaced by TAT : no change
At position 96, AGA is replaced by the weakly used AGG
   -> change is REJECTED : no more forbidden codons can be incorporated
At position 96, AGA is replaced by the weakly used AGA
   -> change is REJECTED : no more forbidden codons can be incorporated
At position 111, GGG is replaced by GGG : no change
At position 177, GAA is replaced by GAA : no change
At position 44, GAT is replaced by GAT : no change
At position 110, AAG is replaced by AAG : no change
At position 42, TTG is replaced by TTG : no change
At position 27, AGT is replaced by AGT : no change
At position 59, GTG is replaced by GTG : no change
At position 118, AGA is replaced by the weakly used AGG
   -> change is REJECTED : no more forbidden codons can be incorporated
At position 118, AGA is replaced by the weakly used AGA
   -> change is REJECTED : no more forbidden codons can be incorporated
At position 153, TTC is replaced by TTC : no change
At position 118, AGA is replaced by the weakly used AGG
   -> change is REJECTED : no more forbidden codons can be incorporated
At position 118, AGA is replaced by the weakly used AGA
   -> change is REJECTED : no more forbidden codons can be incorporated
At position 6, AGC is replaced by AGC : no change
At position 40, CCC is replaced by CCT
   At position 40, CCC is replaced by the weakly used CCT
   Globally the %G+C switch from 51.3513513513513 to 51.1711711711712
   Locally the %G+C switch from 55.9139784946236 to 54.8387096774194
   -> Change is ACCEPTED
At position 107, CAG is replaced by CAG : no change
At position 162, CCC is replaced by CCT At position 162, CCC is replaced by the weakly used CCT
   Globally the %G+C switch from 51.1711711711712 to 50.990990990991
   Locally the %G+C switch from 58.0645161290323 to 56.989247311828
   -> Change is ACCEPTED
At position 118, AGA is replaced by the weakly used AGG
   -> change is REJECTED : no more forbidden codons can be incorporated
At position 118, AGA is replaced by the weakly used AGA
   -> change is REJECTED : no more forbidden codons can be incorporated
At position 150, AAG is replaced by AAG : no change
At position 58, AGT is replaced by AGC
   At position 58, AGT is replaced by the weakly used AGC
   Globally the %G+C switch from 50.990990990991 to 51.1711711711712
   Locally the %G+C switch from 48.3870967741936 to 49.4623655913978
   -> Change is REJECTED
At position 91, ACG is replaced by ACG : no change
At position 120, CTT is replaced by TTA
   At position 120, CTT is replaced by the weakly used TTA
   Globally the %G+C switch from 50.990990990991 to 50.8108108108108
   Locally the %G+C switch from 46.2365591397849 to 45.1612903225806
   -> Change is REJECTED
At position 35, GGC is replaced by GGT
   At position 35, GGC is replaced by the weakly used GGT
   Globally the %G+C switch from 50.990990990991 to 50.8108108108108
   Locally the %G+C switch from 50.5376344086022 to 49.4623655913978
   -> Change is ACCEPTED
At position 179, ACG is replaced by ACT
   At position 179, ACG is replaced by the weakly used ACT
   Globally the %G+C switch from 50.8108108108108 to 50.6306306306306
   Locally the %G+C switch from 52.3809523809524 to 50.7936507936508
   -> Change is ACCEPTED
At position 124, TTG is replaced by CTC
   At position 124, TTG is replaced by the weakly used CTC
   Globally the %G+C switch from 50.6306306306306 to 50.8108108108108
   Locally the %G+C switch from 48.3870967741936 to 49.4623655913978
   -> Change is REJECTED
At position 10, AGG is replaced by AGG : no change
At position 14, GAA is replaced by GAA : no change
At position 91, ACG is replaced by ACG : no change
At position 109, GGG is replaced by GGG : no change
At position 183, GCA is replaced by GCG
   At position 183, GCA is replaced by the weakly used GCG
   Globally the %G+C switch from 50.6306306306306 to 50.8108108108108
   Locally the %G+C switch from 43.1372549019608 to 45.0980392156863
   -> Change is REJECTED
At position 163, GAC is replaced by GAC : no change
At position 38, GCT is replaced by GCC
   At position 38, GCT is replaced by the weakly used GCC
   Globally the %G+C switch from 50.6306306306306 to 50.8108108108108
   Locally the %G+C switch from 51.6129032258064 to 52.6881720430108
   -> Change is REJECTED
At position 9, CTT is replaced by TTA
   At position 9, CTT is replaced by the weakly used TTA
   Globally the %G+C switch from 50.6306306306306 to 50.4504504504504
   Locally the %G+C switch from 45.3333333333333 to 44
   -> Change is REJECTED
At position 16, GAT is replaced by GAT : no change
At position 129, GAA is replaced by GAA : no change
At position 31, GAA is replaced by GAA : no change
At position 9, CTT is replaced by TTG
   At position 9, CTT is replaced by the weakly used TTG
   Globally the %G+C switch from 50.6306306306306 to 50.6306306306306
   Locally the %G+C switch from 45.3333333333333 to 45.3333333333333
   -> Change is ACCEPTED
At position 79, GCA is replaced by GCA : no change
At position 8, ACG is replaced by ACC
   At position 8, ACG is replaced by the weakly used ACC
   Globally the %G+C switch from 50.6306306306306 to 50.6306306306306
   Locally the %G+C switch from 45.8333333333333 to 45.8333333333333
   -> Change is ACCEPTED
At position 159, GTC is replaced by GTC : no change
At position 137, CCC is replaced by CCT
   At position 137, CCC is replaced by the weakly used CCT
   Globally the %G+C switch from 50.6306306306306 to 50.4504504504504
   Locally the %G+C switch from 44.0860215053763 to 43.010752688172
   -> Change is REJECTED
At position 29, ATA is replaced by ATA : no change
At position 63, ATA is replaced by ATA : no change
At position 119, GCA is replaced by GCG
   At position 119, GCA is replaced by the weakly used GCG
   Globally the %G+C switch from 50.6306306306306 to 50.8108108108108
   Locally the %G+C switch from 46.2365591397849 to 47.3118279569892
   -> Change is REJECTED
At position 35, GGT is replaced by GGC
   At position 35, GGT is replaced by the weakly used GGC
   Globally the %G+C switch from 50.6306306306306 to 50.8108108108108
   Locally the %G+C switch from 49.4623655913978 to 50.5376344086022
   -> Change is REJECTED
At position 135, ACT is replaced by ACT : no change
At position 147, TGT is replaced by TGT : no change
At position 127, GAC is replaced by GAC : no change
At position 116, CTT is replaced by TTA
   At position 116, CTT is replaced by the weakly used TTA
   Globally the %G+C switch from 50.6306306306306 to 50.4504504504504
   Locally the %G+C switch from 50.5376344086022 to 49.4623655913978
   -> Change is ACCEPTED
At position 135, ACT is replaced by ACT : no change
At position 31, GAA is replaced by GAA: no change
At position 140, TCC is replaced by TCT
   At position 140, TCC is replaced by the weakly used TCT
   Globally the %G+C switch from 50.4504504504504 to 50.2702702702703
   Locally the %G+C switch from 46.2365591397849 to 45.1612903225806
   -> Change is REJECTED
At position 96, AGA is replaced by the weakly used AGG
   -> change is REJECTED : no more forbidden codons can be incorporated
At position 96, AGA is replaced by the weakly used AGA
   -> change is REJECTED : no more forbidden codons can be incorporated
At position 67, AAC is replaced by AAC : no change
At position 160, ACG is replaced by ACG : no change
At position 88, GAG is replaced by GAG : no change
At position 177, GAA is replaced by GAA : no change
At position 93, CCT is replaced by CCT : no change
At position 182, GAC is replaced by GAC : no change
At position 20, TTG is replaced by TTG : no change
At position 6, AGC is replaced by AGC : no change
At position 90, GAG is replaced by GAG : no change
At position 128, CCA is replaced by CCG
   At position 128, CCA is replaced by the weakly used CCG
   Globally the %G+C switch from 50.4504504504504 to 50:6306306306306
   Locally the %G+C switch from 43.010752688172 to 44.0860215053763
   -> Change is REJECTED
At position 57, GAA is replaced by GAA : no change
At position 95, GTT is replaced by GTC
   At position 95, GTT is replaced by the weakly used GTC
   Globally the %G+C switch from 50.4504504504504 to 50.6306306306306
   Locally the %G+C switch from 54.8387096774194 to 55.9139784946236
   -> Change is REJECTED
At position 76, AGT is replaced by AGC
   At position 76, AGT is replaced by the weakly used AGC
   Globally the %G+C switch from 50.4504504504504 to 50.6306306306306
   Locally the %G+C switch from 50.5376344086022 to 51.6129032258064
   -> Change is REJECTED
At position 72, GGC is replaced by GGT
   At position 72, GGC is replaced by the weakly used GGT
   Globally the %G+C switch from 50.4504504504504 to 50.2702702702703
   Locally the %G+C switch from 48.3870967741936 to 47.3118279569892
   -> Change is REJECTED
At position 16, GAT is replaced by GAT : no change
At position 56, CAG is replaced by CAG : no change
At position 27, AGT is replaced by AGC
   At position 27, AGT is replaced by the weakly used AGC
   Globally the %G+C switch from 50.4504504504504 to 50.6306306306306
   Locally the %G+C switch from 51.6129032258064 to 52.6881720430108
   -> Change is REJECTED
At position 92, GAC is replaced by GAC : no change
At position 133, ATA is replaced by ATA : no change
At position 110, AAG is replaced by AAG : no change
At position 161, ACG is replaced by ACG : no change
At position 145, ATA is replaced by ATA : no change
At position 14, GAA is replaced by GAA : no change
At position 90, GAG is replaced by GAG : no change
At position 74, GCG is replaced by GCA
   At position 74, GCG is replaced by the weakly used GCA
   Globally the %G+C switch from 50.4504504504504 to 50.2702702702703
   Locally the %G+C switch from 50.5376344086022 to 49.4623655913978
   -> Change is ACCEPTED
At position 88, GAG is replaced by GAG : no change
At position 135, ACT is replaced by ACT : no change
At position 55, GCC is replaced by GCT
   At position 55, GCC is replaced by the weakly used GCT
   Globally the %G+C switch from 50.2702702702703 to 50.0900900900901
   Locally the %G+C switch from 48.3870967741936 to 47.3118279569892
   -> Change is REJECTED
At position 160, ACG is replaced by ACG : no change
At position 125, TTT is replaced by TTT : no change
At position 103, GCT is replaced by GCT : no change
At position 104, AAC is replaced by AAC : no change
At position 75, CAA is replaced by CAA : no change
At position 160, ACG is replaced by ACG : no change
At position 4, AAT is replaced by AAT : no change
At position 100, CAA is replaced by CAA : no change
At position 4, AAT is replaced by AAT : no change
At position 118, AGA is replaced by the weakly used AGG
   -> change is REJECTED : no more forbidden codons can be incorporated
At position 118, AGA is replaced by the weakly used AGA
   -> change is REJECTED : no more forbidden codons can be incorporated
At position 34, GGG is replaced by GGG : no change
At position 81, GGT is replaced by GGT : no change
At position 104, AAC is replaced by AAC : no change
At position 36, GAG is replaced by GAG : no change
At position 8, ACC is replaced by ACG
   At position 8, ACC is replaced by the weakly used ACG
   Globally the %G+C switch from 50.2702702702703 to 50.2702702702703
   Locally the %G+C switch from 45.8333333333333 to 45.8333333333333
   -> Change is ACCEPTED
At position 43, GCA is replaced by GCG
   At position 43, GCA is replaced by the weakly used GCG
   Globally the %G+C switch from 50.2702702702703 to 50.4504504504504
   Locally the %G+C switch from 53.763440860215 to 54.8387096774194
   -> Change is REJECTED
At position 147, TGT is replaced by TGT : no change
At position 98, ATC is replaced by ATT
   At position 98, ATC is replaced by the weakly used ATT
   Globally the %G+C switch from 50.2702702702703 to 50.0900900900901
   Locally the %G+C switch from 56.989247311828 to 55.9139784946236
   -> Change is ACCEPTED
At position 175, GCT is replaced by GCT : no change
At position 9, TTG is replaced by CTT
   At position 9, TTG is replaced by the weakly used CTT
   Globally the %G+C switch from 50.0900900900901 to 50.0900900900901
   Locally the %G+C switch from 45.3333333333333 to 45.3333333333333
   > Change is ACCEPTED
t position 118, AGA is replaced by the weakly used AGG
   · change is REJECTED : no more forbidden codons can be incorporated
t position 118, AGA is replaced by the weakly used AGA
   · change is REJECTED : no more forbidden codons can be incorporated
t position 34, GGG is replaced by GGG : no change
t position 31, GAA is replaced by GAA : no change
t position 68, GGT is replaced by GGT : no change
t position 105, GCT is replaced by GCT : no change
t position 108, TTG is replaced by CTC
   t position 108, TTG is replaced by the weakly used CTC
   lobally the %G+C switch from 50.0900900900901 to 50.2702702702703
   ocally the %G+C switch from 48.3870967741936 to 49.4623655913978
   > Change is REJECTED
t position 72, GGC is replaced by GGT
   t position 72, GGC is replaced by the weakly used GGT
   lobally the %G+C switch from 50.0900900900901 to 49.9099099099099
   ocally the %G+C switch from 47.3118279569892 to 46.2365591397849
   > Change is REJECTED
t position 99, GAT is replaced by GAT : no change
t position 167, GTC is replaced by GTT
   t position 167, GTC is replaced by the weakly used GTT
   lobally the %G+C switch from 50.0900900900901 to 49.9099099099099
   ocally the %G+C switch from 58.0645161290323 to 56.989247311828
   > Change is ACCEPTED
: position 13, ACG is replaced by ACG : no change
: position 139, CCC is replaced by CCT
   : position 139, CCC is replaced by the weakly used CCT
   lobally the %G+C switch from 49.9099099099099 to 49.7297297297297
   ocally the %G+C switch from 44.0860215053763 to 43.010752688172
   > Change is REJECTED
: position 38, GCT is replaced by GCC
   : position 38, GCT is replaced by the weakly used GCC
   lobally the %G+C switch from 49.9099099099099 to 50.0900900900901
   ocally the %G+C switch from 51.6129032258064 to 52.6881720430108
   > Change is REJECTED
: position 138, AGT is replaced by AGC
   At position 138, AGT is replaced by the weakly used AGC
   Globally the %G+C switch from 49.9099099099099 to 50.0900900900901
   Locally the %G+C switch from 44.0860215053763 to 45.1612903225806
   -> Change is REJECTED
At position 103, GCT is replaced by GCC
   At position 103, GCT is replaced by the weakly used GCC
   Globally the %G+C switch from 49.9099099099099 to 50.0900900900901
   Locally the %G+C switch from 51.6129032258064 to 52.6881720430108
   -> Change is REJECTED
At position 98, ATT is replaced by ATT : no change
At position 45, GTC is replaced by GTC : no change
At position 53, GTG is replaced by GTG : no change
At position 127, GAC is replaced by GAC : no change
At position 63, ATA is replaced by ATA : no change
At position 57, GAA is replaced by GAA : no change
At position 181, TCC is replaced by TCT
   At position 181, TCC is replaced by the weakly used TCT
   Globally the %G+C switch from 49.9099099099099 to 49.7297297297297
   Locally the %G+C switch from 45.6140350877193 to 43.859649122807
   -> Change is REJECTED
At position 152, GGC is replaced by GGC : no change
At position 27, AGT is replaced by AGC
   At position 27, AGT is replaced by the weakly used AGC
   Globally the %G+C switch from 49.9099099099099 to 50.0900900900901
   Locally the %G+C switch from 51.6129032258064 to 52.6881720430108
   -> Change is REJECTED
At position 184, TGA is replaced by TGA : no change
At position 68, GGT is replaced by GGT : no change
At position 96, AGA is replaced by the weakly used AGG
   -> change is REJECTED : no more forbidden codons can be incorporated
At position 96, AGA is replaced by the weakly used AGA
   -> change is REJECTED : no more forbidden codons can be incorporated
At position 134, CAG is replaced by CAG : no change
At position 140, TCC is replaced by TCC : no change
At position 40, CCT is replaced by CCT : no change
At position 105, GCT is replaced by GCC
   At position 105, GCT is replaced by the weakly used GCC
   Globally the %G+C switch from 49.9099099099099 to 50.0900900900901
   Locally the %G+C switch from 50.5376344086022 to 51.6129032258064
   -> Change is REJECTED
At position 158, ACG is replaced by ACG : no change
At position 37, GAG is replaced by GAG : no change
At position 122, GAA is replaced by GAA : no change
At position 159, GTC is replaced by GTT
   At position 159, GTC is replaced by the weakly used GTT
   Globally the %G+C switch from 49.9099099099099 to 49.7297297297297
   Locally the %G+C switch from 56.989247311828 to 55.9139784946236
   -> Change is ACCEPTED
At position 24, TTG is replaced by TTG : no change
At position 45, GTC is replaced by GTC : no change
At position 139, CCC is replaced by CCC : no change
At position 142, CTT is replaced by CTT : no change
At position 51, CCT is replaced by CCT : no change
At position 148, TAT is replaced by TAT : no change
At position 124, TTG is replaced by TTG : no change
At position 4, AAT is replaced by AAT : no change
At position 52, TCT is replaced by TCG
   At position 52, TCT is replaced by the weakly used TCG
   Globally the %G+C switch from 49.7297297297297 to 49.9099099099099
   Locally the %G+C switch from 49.4623655913978 to 50.5376344086022
   -> Change is REJECTED
At position 24, TTG is replaced by TTG : no change
At position 103, GCT is replaced by GCC
   At position 103, GCT is replaced by the weakly used GCC
   Globally the %G+C switch from 49.7297297297297 to 49.9099099099099
   Locally the %G+C switch from 51.6129032258064 to 52.6881720430108
   -> Change is REJECTED
At position 94, GGT is replaced by GGC
   At position 94, GGT is replaced by the weakly used GGC
   Globally the %G+C switch from 49.7297297297297 to 49.9099099099099
   Locally the %G+C switch from 54.8387096774194 to 55.9139784946236
   -> Change is REJECTED
At position 2, AAT is replaced by AAT : no change
At position 123, CTT is replaced by the weakly used CTA
   -> change is REJECTED : no more forbidden codons can be incorporated
At position 123, CTT is replaced by the weakly used CTT
   -> change is REJECTED : no more forbidden codons can be incorporated
At position 123, CTT is replaced by the weakly used CTT
   -> change is REJECTED : no more forbidden codons can be incorporated
At position 183, GCA is replaced by GCA : no change
At position 62, TAT is replaced by TAT : no change
At position 102, CTC is replaced by TTA
   At position 102, CTC is replaced by the weakly used TTA
   Globally the %G+C switch from 49.7297297297297 to 49.3693693693694
   Locally the %G+C switch from 52.6881720430108 to 50.5376344086022
   -> Change is ACCEPTED
At position 128, CCA is replaced by CCA : no change
At position 36, GAG is replaced by GAG : no change
At position 167, GTT is replaced by GTC
   At position 167, GTT is replaced by the weakly used GTC
   Globally the %G+C switch from 49.3693693693694 to 49.5495495495495
   Locally the %G+C switch from 55.9139784946236 to 56.989247311828
   -> Change is REJECTED
At position 181, TCC is replaced by TCT
   At position 181, TCC is replaced by the weakly used TCT
   Globally the %G+C switch from 49.3693693693694 to 49.1891891891892
   Locally the %G+C switch from 45.6140350877193 to 43.859649122807
   -> Change is REJECTED
At position 93, CCT is replaced by CCC
   At position 93, CCT is replaced by the weakly used CCC
   Globally the %G+C switch from 49.3693693693694 to 49.5495495495495
   Locally the %G+C switch from 51.6129032258064 to 52.6881720430108
   -> Change is REJECTED
At position 99, GAT is replaced by GAT : no change
At position 60, ACG is replaced by ACG : no change
At position 18, GCC is replaced by GCT
   At position 18, GCC is replaced by the weakly used GCT
   Globally the %G+C switch from 49.3693693693694 to 49.1891891891892
   Locally the %G+C switch from 47.3118279569892 to 46.2365591397849
   -> Change is REJECTED
At position 30, GTG is replaced by GTG : no change
At position 11, TTG is replaced by TTG : no change
At position 88, GAG is replaced by GAG : no change
At position 76, AGT is replaced by AGC
   At position 76, AGT is replaced by the weakly used AGC
   Globally the %G+C switch from 49.3693693693694 to 49.5495495495495
   Locally the %G+C switch from 49.4623655913978 to 50.5376344086022
   -> Change is REJECTED
At position 10, AGG is replaced by AGG : no change
At position 85, GGT is replaced by GGC
   At position 85, GGT is replaced by the weakly used GGC
   Globally the %G+C switch from 49.3693693693694 to 49.5495495495495
   Locally the %G+C switch from 50.5376344086022 to 51.6129032258064
   -> Change is REJECTED
At position 155, CGC is replaced by CGT
   At position 155, CGC is replaced by the weakly used CGT
   Globally the %G+C switch from 49.3693693693694 to 49.1891891891892
   Locally the %G+C switch from 53.763440860215 to 52.6881720430108
   -> Change is ACCEPTED
At position 183, GCA is replaced by GCG
   At position 183, GCA is replaced by the weakly used GCG
   Globally the %G+C switch from 49.1891891891892 to 49.3693693693694
   Locally the %G+C switch from 43.1372549019608 to 45.0980392156863
   -> Change is REJECTED
At position 160, ACG is replaced by ACG : no change
At position 99, GAT is replaced by GAT : no change
At position 175, GCT is replaced by GCT : no change
At position 174, CAA is replaced by CAA : no change
At position 57, GAA is replaced by GAA : no change
At position 99, GAT is replaced by GAT : no change
At position 44, GAT is replaced by GAT : no change
At position 61, CCT is replaced by CCT : no change
At position 73, TAC is replaced by TAC : no change
At position 176, TTT is replaced by TTT : no change
At position 133, ATA is replaced by ATA : no change
At position 161, ACG is replaced by ACG : no change
At position 83, GGT is replaced by GGT : no change
At position 11, TTG is replaced by TTG : no change
At position 62, TAT is replaced by TAT : no change
At position 59, GTG is replaced by GTG : no change
At position 117, GTG is replaced by GTG : no change
At position 171, CAG is replaced by CAG : no change
At position 133, ATA is replaced by ATA : no change
At position 37, GAG is replaced by GAG : no change
At position 24, TTG is replaced by TTG : no change
At position 34, GGG is replaced by GGG : no change
At position 138, AGT is replaced by AGC
   At position 138, AGT is replaced by the weakly used AGC
   Globally the %G+C switch from 49.1891891891892 to 49.3693693693694
   Locally the %G+C switch from 44.0860215053763 to 45.1612903225806
   -> Change is REJECTED
At position 89, GAG is replaced by GAG : no change
At position 180, AGA is replaced by the weakly used AGG
   -> change is REJECTED : no more forbidden codons can be incorporated
At position 180, AGA is replaced by the weakly used AGA
   -> change is REJECTED : no more forbidden codons can be incorporated
   At position 61, CCT is replaced by CCC
At position 61, CCT is replaced by the weakly used CCC
   Globally the %G+C switch from 49.1891891891892 to 49.3693693693694
   Locally the %G+C switch from 47.3118279569892 to 48.3870967741936
   -> Change is REJECTED
At position 126, AAC is replaced by AAC : no change
At position 126, AAC is replaced by AAC : no change
At position 100, CAA is replaced by CAA : no change
At position 145, ATA is replaced by ATA : no change
At position 160, ACG is replaced by ACG : no change
At position 130, GTG is replaced by GTG : no change
At position 85, GGT is replaced by GGT : no change
At position 83, GGT is replaced by GGT : no change
At position 40, CCT is replaced by CCC
   At position 40, CCT is replaced by the weakly used CCC
   Globally the %G+C switch from 49.1891891891892 to 49.3693693693694
   Locally the %G+C switch from 53.763440860215 to 54.8387096774194
   -> Change is REJECTED
At position 29, ATA is replaced by ATA : no change
At position 96, AGA is replaced by the weakly used AGG
   -> change is REJECTED : no more forbidden codons can be incorporated
At position 96, AGA is replaced by the weakly used AGA
   -> change is REJECTED : no more forbidden codons can be incorporated
At position 159, GTT is replaced by GTC
   At position 159, GTT is replaced by the weakly used GTC
   Globally the %G+C switch from 49.1891891891892 to 49.3693693693694
   Locally the %G+C switch from 54.8387096774194 to 55.9139784946236
   -> Change is REJECTED
At position 8, ACG is replaced by ACT
   At position 8, ACG is replaced by the weakly used ACT
   Globally the %G+C switch from 49.1891891891892 to 49.009009009009
   Locally the %G+C switch from 45.8333333333333 to 44.4444444444444
   -> Change is REJECTED
At position 84, GAT is replaced by GAT: no change
At position 144, GCC is replaced by GCC : no change
At position 34, GGG is replaced by GGG : no change
At position 168, TAT is replaced by TAT : no change
At position 88, GAG is replaced by GAG : no change
At position 128, CCA is replaced by CCA : no change
At position 91, ACG is replaced by ACG : no change
At position 130, GTG is replaced by GTG : no change
At position 147, TGT is replaced by TGT : no change
At position 100, CAA is replaced by CAA : no change
At position 6, AGC is replaced by AGT
   At position 6, AGC is replaced by the weakly used AGT
   Globally the %G+C switch from 49.1891891891892 to 49.009009009009
   Locally the %G+C switch from 45.4545454545455 to 43.9393939393939
   -> Change is REJECTED
At position 107, CAG is replaced by CAG : no change
At position 130, GTG is replaced by GTG : no change
At position 120, CTT is replaced by CTT : no change
At position 171, CAG is replaced by CAG : no change
At position 83, GGT is replaced by GGC
   At position 83, GGT is replaced by the weakly used GGC
   Globally the %G+C switch from 49.1891891891892 to 49.3693693693694
   Locally the %G+C switch from 51.6129032258064 to 52.6881720430108
   -> Change is REJECTED
At position 113, GGT is replaced by GGC
   At position 113, GGT is replaced by the weakly used GGC
   Globally the %G+C switch from 49.1891891891892 to 49.3693693693694
   Locally the %G+C switch from 44.0860215053763 to 45.1612903225806
   -> Change is REJECTED
At position 51, CCT is replaced by CCT : no change
At position 77, TAT is replaced by TAT : no change
At position 31, GAA is replaced by GAA : no change
At position 54, CTG is replaced by CTG : no change
At position 176, TTT is replaced by TTT : no change
At position 165, CCT is replaced by CCT : no change
At position 42, TTG is replaced by TTG : no change
At position 118, AGA is replaced by the weakly used AGG
   -> change is REJECTED : no more forbidden codons can be incorporated
At position 118, AGA is replaced by the weakly used AGA
   -> change is REJECTED : no more forbidden codons can be incorporated
At position 123, CTT is replaced by CTC
   At position 123, CTT is replaced by the weakly used CTC
   Globally the %G+C switch from 49.1891891891892 to 49.3693693693694
   Locally the %G+C switch from 45.1612903225806 to 46.2365591397849
   -> Change is REJECTED
At position 108, TTG is replaced by TTG : no change
At position 20, TTG is replaced by TTG : no change
At position 17, TTG is replaced by TTG : no change
At position 75, CAA is replaced by CAA : no change
At position 108, TTG is replaced by CTC
   At position 108, TTG is replaced by the weakly used CTC
   Globally the %G+C switch from 49.1891891891892 to 49.3693693693694
   Locally the %G+C switch from 46.2365591397849 to 47.3118279569892
   -> Change is REJECTED
At position 75, CAA is replaced by CAA : no change
At position 125, TTT is replaced by TTT : no change
At position 112, CTT is replaced by the weakly used CTA
   -> change is REJECTED : no more forbidden codons can be incorporated
At position 112, CTT is replaced by the weakly used CTT
   -> change is REJECTED : no more forbidden codons can be incorporated
At position 112, CTT is replaced by the weakly used CTT
   -> change is REJECTED : no more forbidden codons can be incorporated
At position 160, ACG is replaced by ACG : no change
At position 58, AGT is replaced by AGT : no change
At position 114, ACG is replaced by ACG : no change
At position 3, TCT is replaced by TCC
   At position 3, TCT is replaced by the weakly used TCC
   Globally the %G+C switch from 49.1891891891892 to 49.3693693693694
   Locally the %G+C switch from 47.3684210526316 to 49.1228070175439
   -> Change is REJECTED
At position 136, GAT is replaced by GAT : no change
At position 176, TTT is replaced by TTT : no change
At position 122, GAA is replaced by GAA : no change
At position 61, CCT is replaced by CCT: no change
At position 152, GGC is replaced by GGC : no change
At position 56, CAG is replaced by CAG : no change
At position 184, TGA is replaced by TGA : no change
At position 54, CTG is replaced by CTG : no change
At position 171, CAG is replaced by CAG : no change
At position 103, GCT is replaced by GCT : no change
At position 147, TGT is replaced by TGT : no change
At position 107, CAG is replaced by CAG : no change
At position 53, GTG is replaced by GTG : no change
At position 72, GGC is replaced by GGC : no change
At position 58, AGT is replaced by AGT : no change
At position 41, ACT is replaced by ACC
   At position 41, ACT is replaced by the weakly used ACC
   Globally the %G+C switch from 49.1891891891892 to 49.3693693693694
   Locally the %G+C switch from 54.8387096774194 to 55.9139784946236
   -> Change is REJECTED
At position 104, AAC is replaced by AAC : no change
At position 119, GCA is replaced by GCG
   At position 119, GCA is replaced by the weakly used GCG
   Globally the %G+C switch from 49.1891891891892 to 49.3693693693694
   Locally the %G+C switch from 45.1612903225806 to 46.2365591397849
   -> Change is REJECTED
At position 132, AAA is replaced by AAA : no change
At position 122, GAA is replaced by GAA : no change
At position 91, ACG is replaced by ACG : no change
At position 137, CCC is replaced by CCC : no change

At position 95, GTT is replaced by GTT : no change
At position 90, GAG is replaced by GAG : no change
At position 3, TCT is replaced by TCG
   At position 3, TCT is replaced by the weakly used TCG
   Globally the %G+C switch from 49.1891891891892 to 49.3693693693694
   Locally the %G+C switch from 47.3684210526316 to 49.1228070175439
   -> Change is REJECTED
At position 124, TTG is replaced by CTC
   At position 124, TTG is replaced by the weakly used CTC
   Globally the %G+C switch from 49.1891891891892 to 49.3693693693694
   Locally the %G+C switch from 47.3118279569892 to 48.3870967741936
   -> Change is REJECTED
At position 100, CAA is replaced by CAA : no change
At position 120, CTT is replaced by TTA
   At position 120, CTT is replaced by the weakly used TTA
   Globally the %G+C switch from 49.1891891891892 to 49.009009009009
   Locally the %G+C switch from 45.1612903225806 to 44.0860215053763
   -> Change is REJECTED
At position 49, TAT is replaced by TAT : no change
At position 110, AAG is replaced by AAG : no change
At position 123, CTT is replaced by CTT : no change
At position 101, CTG is replaced by CTG : no change
At position 155, CGT is replaced by CGT : no change
At position 22, GAA is replaced by GAA : no change
At position 8, ACG is replaced by ACT
   At position 8, ACG is replaced by the weakly used ACT Globally the %G+C switch from 49.1891891891892 to 49.009009009009
   Locally the %G+C switch from 45.8333333333333 to 44.4444444444444
   -> Change is REJECTED
At position 83, GGT is replaced by GGC
   At position 83, GGT is replaced by the weakly used GGC
   Globally the %G+C switch from 49.1891891891892 to 49.3693693693694
   Locally the %G+C switch from 51.6129032258064 to 52.6881720430108
   -> Change is REJECTED
At position 21, TAC is replaced by TAC : no change
At position 45, GTC is replaced by GTT
   At position 45, GTC is replaced by the weakly used GTT
   Globally the %G+C switch from 49.1891891891892 to 49.009009009009
   Locally the %G+C switch from 55.9139784946236 to 54.8387096774194
   -> Change is ACCEPTED
At position 11, TTG is replaced by TTG : no change
At position 145, ATA is replaced by ATA : no change
At position 145, ATA is replaced by ATA : no change
At position 130, GTG is replaced by GTG : no change
At position 153, TTC is replaced by TTC : no change
At position 172, ACG is replaced by ACT
   At position 172, ACG is replaced by the weakly used ACT
   Globally the %G+C switch from 49.009009009009 to 48.8288288288288
   Locally the %G+C switch from 53.5714285714286 to 52.3809523809524
   -> Change is ACCEPTED
At position 67, AAC is replaced by AAC : no change
At position 52, TCT is replaced by TCC
   At position 52, TCT is replaced by the weakly used TCC
   Globally the %G+C switch from 48.8288288288288 to 49.009009009009
   Locally the %G+C switch from 48.3870967741936 to 49.4623655913978
   -> Change is REJECTED
At position 67, AAC is replaced by AAC : no change
At position 120, CTT is replaced by CTT : no change
At position 182, GAC is replaced by GAC : no change
At position 78, GTG is replaced by GTG : no change
At position 63, ATA is replaced by ATA : no change
At position 22, GAA is replaced by GAA : no change
At position 43, GCA is replaced by GCA : no change
At position 128, CCA is replaced by CCA : no change
At position 111, GGG is replaced by GGG : no change
At position 131, ACG is replaced by ACG : no change
At position 14, GAA is replaced by GAA : no change
At position 137, CCC is replaced by CCC : no change
At position 9, CTT is replaced by TTG
   At position 9, CTT is replaced by the weakly used TTG
   Globally the %G+C switch from 48.8288288288288 to 48.8288288288288
   Locally the %G+C switch from 45.3333333333333 to 45.3333333333333
   -> Change is ACCEPTED
At position 42, TTG is replaced by TTG : no change
At position 1, ACG is replaced by ACG : no change
At position 176, TTT is replaced by TTT : no change
At position 89, GAG is replaced by GAG : no change
At position 157, GGG is replaced by GGG : no change
At position 57, GAA is replaced by GAA : no change
At position 131, ACG is replaced by ACG : no change
At position 119, GCA is replaced by GCG
   At position 119, GCA is replaced by the weakly used GCG
   Globally the %G+C switch from 48.8288288288288 to 49.009009009009
   Locally the %G+C switch from 45.1612903225806 to 46.2365591397849
   -> Change is REJECTED
At position 147, TGT is replaced by TGT : no change
At position 40, CCT is replaced by CCT : no change
At position 40, CCT is replaced by CCC
   At position 40, CCT is replaced by the weakly used CCC
   Globally the %G+C switch from 48.8288288288288 to 49.009009009009
   Locally the %G+C switch from 52.6881720430108 to 53.763440860215
   -> Change is REJECTED
At position 69, GAA is replaced by GAA : no change
At position 72, GGC is replaced by GGC : no change
At position 120, CTT is replaced by CTT : no change
At position 7, GTG is replaced by GTG : no change
At position 181, TCC is replaced by TCC : no change
At position 167, GTT is replaced by GTC
   At position 167, GTT is replaced by the weakly used GTC
   Globally the %G+C switch from 48.8288288288288 to 49.009009009009
   Locally the %G+C switch from 53.763440860215 to 54.8387096774194
   -> Change is REJECTED
At position 109, GGG is replaced by GGG : no change
At position 58, AGT is replaced by AGC
   At position 58, AGT is replaced by the weakly used AGC
   Globally the %G+C switch from 48.8288288288288 to 49.009009009009
   Locally the %G+C switch from 47.3118279569892 to 48.3870967741936
   -> Change is REJECTED
At position 96, AGA is replaced by the weakly used AGG
   -> change is REJECTED : no more forbidden codons can be incorporated
At position 96, AGA is replaced by the weakly used AGA
   -> change is REJECTED : no more forbidden codons can be incorporated
At position 24, TTG is replaced by TTG : no change
At position 178, AGA is replaced by the weakly used AGG
   -> change is REJECTED : no more forbidden codons can be incorporated
At position 178, AGA is replaced by the weakly used AGA
   -> change is REJECTED : no more forbidden codons can be incorporated
At position 59, GTG is replaced by GTG : no change
At position 79, GCA is replaced by GCG
   At position 79, GCA is replaced by the weakly used GCG
   Globally the %G+C switch from 48.8288288288288 to 49.009009009009
   Locally the %G+C switch from 53.763440860215 to 54.8387096774194
   -> Change is REJECTED
At position 44, GAT is replaced by GAT : no change
At position 69, GAA is replaced by GAA : no change
At position 3, TCT is replaced by TCC
   At position 3, TCT is replaced by the weakly used TCC
   Globally the %G+C switch from 48.8288288288288 to 49.009009009009
   Locally the %G+C switch from 47.3684210526316 to 49.1228070175439
   -> Change is REJECTED
At position 15, CAC is replaced by CAC : no change
At position 111, GGG is replaced by GGG : no change
At position 26, AGG is replaced by AGG : no change
At position 2, AAT is replaced by AAT : no change
At position 145, ATA is replaced by ATA : no change
At position 21, TAC is replaced by TAC : no change
At position 61, CCT is replaced by CCT : no change
At position 26, AGG is replaced by AGG : no change
At position 71, ATA is replaced by ATA : no change
At position 15, CAC is replaced by CAC : no change
At position 21, TAC is replaced by TAC : no change
At position 135, ACT is replaced by ACT : no change
At position 107, CAG is replaced by CAG : no change
At position 150, AAG is replaced by AAG : no change
At position 167, GTT is replaced by GTC
   At position 167, GTT is replaced by the weakly used GTC
   Globally the %G+C switch from 48.8288288288288 to 49.009009009009
   Locally the %G+C switch from 53.763440860215 to 54.8387096774194

   -> Change is REJECTED
At position 103, GCT is replaced by GCT : no change
At position 158, ACG is replaced by ACG : no change
At position 148, TAT is replaced by TAT : no change
At position 22, GAA is replaced by GAA : no change
At position 43, GCA is replaced by GCA : no change
At position 7, GTG is replaced by GTG : no change
At position 104, AAC is replaced by AAC : no change
At position 75, CAA is replaced by CAA : no change
At position 80, TTG is replaced by TTG : no change
At position 71, ATA is replaced by ATA : no change
At position 1, ACG is replaced by ACG : no change
At position 136, GAT is replaced by GAT : no change
At position 111, GGG is replaced by GGG : no change
At position 70, CCT is replaced by CCC
   At position 70, CCT is replaced by the weakly used CCC
   Globally the %G+C switch from 48.8288288288288 to 49.009009009009
   Locally the %G+C switch from 48.3870967741936 to 49.4623655913978
   -> Change is REJECTED
At position 120, CTT is replaced by CTT : no change
At position 179, ACT is replaced by ACC
   At position 179, ACT is replaced by the weakly used ACC
   Globally the %G+C switch from 48.8288288288288 to 49.009009009009
   Locally the %G+C switch from 47.6190476190476 to 49.2063492063492
   -> Change is REJECTED
At position 62, TAT is replaced by TAT : no change
At position 40, CCT is replaced by CCC
   At position 40, CCT is replaced by the weakly used CCC
   Globally the %G+C switch from 48.8288288288288 to 49.009009009009
   Locally the %G+C switch from 52.6881720430108 to 53.763440860215
   -> Change is REJECTED
At position 22, GAA is replaced by GAA : no change
At position 9, TTG is replaced by CTC
   At position 9, TTG is replaced by the weakly used CTC
   Globally the %G+C switch from 48.8288288288288 to 49.009009009009
   Locally the %G+C switch from 45.3333333333333 to 46.6666666666667
   -> Change is REJECTED
   At position 73, TAC is replaced by TAC : no change
At position 138, AGT is replaced by AGT : no change
At position 118, AGA is replaced by the weakly used AGG
   -> change is REJECTED no more forbidden codons can be incorporated
At position 118, AGA is replaced by the weakly used AGA
   -> change is REJECTED : no more forbidden codons can be incorporated
At position 162, CCT is replaced by CCC
   At position 162, CCT is replaced by the weakly used CCC
   Globally the %G+C switch from 48.8288288288288 to 49.009009009009
   Locally the %G+C switch from 52.6881720430108 to 53.763440860215
   -> Change is REJECTED
At position 60, ACG is replaced by ACG : no change
At position 44, GAT is replaced by GAT : no change
At position 154, GAA is replaced by GAA : no change
At position 143, AGA is replaced by the weakly used AGG
   -> change is REJECTED : no more forbidden codons can be incorporated
At position 143, AGA is replaced by the weakly used AGA
   -> change is REJECTED : no more forbidden codons can be incorporated
At position 132, AAA is replaced by AAA : no change
At position 97, GGT is replaced by GGT : no change
At position 80, TTG is replaced by TTG : no change
At position 106, AGT is replaced by AGC
   At position 106, AGT is replaced by the weakly used AGC
   Globally the %G+C switch from 48.8288288288288 to 49.009009009009
   Locally the %G+C switch from 48.3870967741936 to 49.4623655913978
   -> Change is REJECTED
At position 15, CAC is replaced by CAC : no change
At position 5, GAC is replaced by GAC : no change
At position 56, CAG is replaced by CAG : no change
At position 63, ATA is replaced by ATA : no change
At position 72, GGC is replaced by GGT
   At position 72, GGC is replaced by the weakly used GGT
   Globally the %G+C switch from 48.8288288288288 to 48.6486486486487
   Locally the %G+C switch from 47.3118279569892 to 46.2365591397849
   -> Change is REJECTED
   At position 55, GCC is replaced by GCT
At position 55, GCC is replaced by the weakly used GCT
   Globally the %G+C switch from 48.8288288288288 to 48.6486486486487
   Locally the %G+C switch from 47.3118279569892 to 46.2365591397849
   -> Change is REJECTED
At position 76, AGT is replaced by AGC
   At position 76, AGT is replaced by the weakly used AGC
   Globally the %G+C switch from 48.8288288288288 to 49.009009009009
   Locally the %G+C switch from 49.4623655913978 to 50.5376344086022
   -> Change is REJECTED
At position 139, CCC is replaced by CCC : no change
At position 17, TTG is replaced by TTG : no change
At position 8, ACG is replaced by ACG : no change
At position 31, GAA is replaced by GAA : no change
At position 8, ACG is replaced by ACT
   At position 8, ACG is replaced by the weakly used ACT
   Globally the %G+C switch from 48.8288288288288 to 48.6486486486487
   Locally the %G+C switch from 45.8333333333333 to 44.4444444444444
   -> Change is REJECTED
At position 5, GAC is replaced by GAC : no change
At position 17, TTG is replaced by TTG : no change
At position 103, GCT is replaced by GCC
   At position 103, GCT is replaced by the weakly used GCC
   Globally the %G+C switch from 48.8288288288288 to 49.009009009009
   Locally the %G+C switch from 49.4623655913978 to 50.5376344086022
   -> Change is REJECTED
At position 115, AAA is replaced by AAA : no change
At position 127, GAC is replaced by GAC : no change
At position 124, TTG is replaced by CTT
   At position 124, TTG is replaced by the weakly used CTT
   Globally the %G+C switch from 48.8288288288288 to 48.8288288288288
   Locally the %G+C switch from 47.3118279569892 to 47.3118279569892
   -> Change is ACCEPTED
At position 141, AAT is replaced by AAT : no change
At position 44, GAT is replaced by GAT : no change
At position 47, GAG is replaced by GAG : no change
At position 91, ACG is replaced by ACG : no change
At position 108, TTG is replaced by CTT
   At position 108, TTG is replaced by the weakly used CTT
   Globally the %G+C switch from 48.8288288288288 to 48.8288288288288
   Locally the %G+C switch from 46.2365591397849 to 46.2365591397849
   -> Change is ACCEPTED
At position 162, CCT is replaced by CCT : no change
At position 41, ACT is replaced by ACT : no change
At position 70, CCT is replaced by CCC
   At position 70, CCT is replaced by the weakly used CCC
   Globally the %G+C switch from 48.8288288288288 to 49.009009009009
   Locally the %G+C switch from 48.3870967741936 to 49.4623655913978
   -> Change is REJECTED
At position 44, GAT is replaced by GAT : no change
At position 56, CAG is replaced by CAG : no change
At position 124, CTT is replaced by TTG
   At position 124, CTT is replaced by the weakly used TTG
   Globally the %G+C switch from 48.8288288288288 to 48.8288288288288
   Locally the %G+C switch from 47.3118279569892 to 47.3118279569892
   -> Change is ACCEPTED
At position 117, GTG is replaced by GTG : no change
At position 7, GTG is replaced by GTG : no change
At position 177, GAA is replaced by GAA : no change
At position 29, ATA is replaced by ATA : no change
At position 155, CGT is replaced by CGT : no change
At position 111, GGG is replaced by GGG : no change
At position 170, GTG is replaced by GTG : no change
At position 30, GTG is replaced by GTG : no change
At position 97, GGT is replaced by GGT : no change
   At position 97, GGT is replaced by GGC
   At position 97, GGT is replaced by the weakly used GGC
   Globally the %G+C switch from 48.8288288288288 to 49.009009009009
   Locally the %G+C switch from 52.6881720430108 to 53.763440860215
   -> Change is REJECTED
At position 13, ACG is replaced by ACG : no change
At position 50, CTT is replaced by CTT : no change
At position 24, TTG is replaced by TTG : no change
At position 173, AGA is replaced by the weakly used AGG
   -> change is REJECTED : no more forbidden codons can be incorporated
At position 173, AGA is replaced by the weakly used AGA
   -> change is REJECTED : no more forbidden codons can be incorporated
At position 30, GTG is replaced by GTG : no change
At position 163, GAC is replaced by GAC : no change
At position 184, TGA is replaced by TGA : no change
At position 115, AAA is replaced by AAA : no change
At position 68, GGT is replaced by GGT : no change
At position 125, TTT is replaced by TTT : no change
At position 130, GTG is replaced by GTG : no change
At position 98, ATT is replaced by ATC
   At position 98, ATT is replaced by the weakly used ATC
   Globally the %G+C switch from 48.8288288288288 to 49.009009009009
   Locally the %G+C switch from 53.763440860215 to 54.8387096774194
   -> Change is REJECTED
At position 99, GAT is replaced by GAT : no change
At position 91, ACG is replaced by ACG : no change
At position 184, TGA is replaced by TGA : no change
At position 45, GTT is replaced by GTC
   At position 45, GTT is replaced by the weakly used GTC
   Globally the %G+C switch from 48.8288288288288 to 49.009009009009
   Locally the %G+C switch from 54.8387096774194 to 55.9139784946236
   -> Change is REJECTED
At position 2, AAT is replaced by AAT : no change
At position 2, AAT is replaced by AAT : no change
At position 121, GTG is replaced by GTG : no change
At position 159, GTT is replaced by GTT : no change
At position 35, GGT is replaced by GGT : no change
At position 22, GAA is replaced by GAA : no change
At position 105, GCT is replaced by GCT : no change
At position 113, GGT is replaced by GGC
   At position 113, GGT is replaced by the weakly used GGC
   Globally the %G+C switch from 48.8288288288288 to 49.009009009009
   Locally the %G+C switch from 44.0860215053763 to 45.1612903225806
   -> Change is REJECTED
At position 81, GGT is replaced by GGT : no change
At position 108, CTT is replaced by TTA
   At position 108, CTT is replaced by the weakly used TTA
   Globally the %G+C switch from 48.8288288288288 to 48.6486486486487
   Locally the %G+C switch from 46.2365591397849 to 45.1612903225806
   -> Change is REJECTED
At position 143, AGA is replaced by the weakly used AGG
   -> change is REJECTED : no more forbidden codons can be incorporated
At position 143, AGA is replaced by the weakly used AGA
   -> change is REJECTED : no more forbidden codons can be incorporated
At position 171, CAG is replaced by CAG : no change
At position 2, AAT is replaced by AAT : no change
At position 129, GAA is replaced by GAA : no change
At position 181, TCC is replaced by TCT
   At position 181, TCC is replaced by the weakly used TCT
   Globally the %G+C switch from 48.8288288288288 to 48.6486486486487
   Locally the %G+C switch from 43.859649122807 to 42.1052631578947
   -> Change is REJECTED
At position 80, TTG is replaced by TTG : no change
At position 58, AGT is replaced by AGT : no change
At position 73, TAC is replaced by TAC : no change
At position 129, GAA is replaced by GAA : no change
At position 41, ACT is replaced by ACG
   At position 41, ACT is replaced by the weakly used ACG
   Globally the %G+C switch from 48.8288288288288 to 49.009009009009
   Locally the %G+C switch from 53.763440860215 to 54.8387096774194
   -> Change is REJECTED
At position 149, GAA is replaced by GAA : no change
At position 172, ACT is replaced by ACC
   At position 172, ACT is replaced by the weakly used ACC
   Globally the %G+C switch from 48.8288288288288 to 49.009009009009
   Locally the %G+C switch from 52.3809523809524 to 53.5714285714286
   -> Change is REJECTED
At position 184, TGA is replaced by TGA : no change
At position 2, AAT is replaced by AAT : no change
At position 140, TCC is replaced by TCC : no change
At position 70, CCT is replaced by CCT : no change
At position 2, AAT is replaced by AAT : no change
At position 160, ACG is replaced by ACG : no change
At position 60, ACG is replaced by ACG : no change
At position 92, GAC is replaced by GAC : no change
At position 160, ACG is replaced by ACG : no change
At position 78, GTG is replaced by GTG : no change
At position 88, GAG is replaced by GAG : no change
At position 84, GAT is replaced by GAT : no change
At position 78, GTG is replaced by GTG : no change
At position 157, GGG is replaced by GGG : no change
At position 78, GTG is replaced by GTG : no change
At position 14, GAA is replaced by GAA : no change
At position 91, ACG is replaced by ACG : no change
At position 98, ATT is replaced by ATT : no change
At position 144, GCC is replaced by GCT
At position 144, GCC is replaced by the weakly used GCT
Globally the %G+C switch from 48.8288288288288 to 48.6486486486487
Locally the %G+C switch from 48.3870967741936 to 47.3118279569892
-> Change is REJECTED
At position 128, CCA is replaced by CCG
   At position 128, CCA is replaced by the weakly used CCG
   Globally the %G+C switch from 48.8288288288288 to 49.009009009009
   Locally the %G+C switch from 43.010752688172 to 44.0860215053763
   -> Change is REJECTED
At position 145, ATA is replaced by ATA : no change
At position 7, GTG is replaced by GTG : no change
At position 45, GTT is replaced by GTT : no change
At position 120, CTT is replaced by TTG
   At position 120, CTT is replaced by the weakly used TTG
   Globally the %G+C switch from 48.8288288288288 to 48.8288288288288
   Locally the %G+C switch from 45.1612903225806 to 45.1612903225806
   -> Change is ACCEPTED
At position 94, GGT is replaced by GGT : no change
At position 39, AGG is replaced by AGG : no change
At position 163, GAC is replaced by GAC : no change
At position 80, TTG is replaced by TTG : no change
At position 68, GGT is replaced by GGT : no change
   At position 63, ATA is replaced by ATA : no change
At position 50, CTT is replaced by CTC
   At position 50, CTT is replaced by the weakly used CTC
   Globally the %G+C switch from 48.8288288288288 to 49.009009009009
   Locally the %G+C switch from 50.5376344086022 to 51.6129032258064
   -> Change is REJECTED
At position 82, TCT is replaced by TCG
   At position 82, TCT is replaced by the weakly used TCG
   Globally the %G+C switch from 48.8288288288288 to 49.009009009009
   Locally the %G+C switch from 52.6881720430108 to 53.763440860215
   -> Change is REJECTED
At position 96, AGA is replaced by the weakly used AGG
   -> change is REJECTED : no more forbidden codons can be incorporated
At position 96, AGA is replaced by the weakly used AGA
   -> change is REJECTED : no more forbidden codons can be incorporated
   At position 80, TTG is replaced by TTG : no change
At position 161, ACG is replaced by ACG : no change
At position 68, GGT is replaced by GGC
   At position 68, GGT is replaced by the weakly used GGC
   Globally the %G+C switch from 48.8288288288288 to 49.009009009009
   Locally the %G+C switch from 49.4623655913978 to 50.5376344086022
   -> Change is REJECTED
At position 154, GAA is replaced by GAA : no change
At position 148, TAT is replaced by TAT : no change
At position 93, CCT is replaced by CCT : no change
At position 29, ATA is replaced by ATA : no change
At position 18, GCC is replaced by GCT
   At position 18, GCC is replaced by the weakly used GCT
   Globally the %G+C switch from 48.8288288288288 to 48.6486486486487
   Locally the %G+C switch from 47.3118279569892 to 46.2365591397849
   -> Change is REJECTED
At position 78, GTG is replaced by GTG : no change
At position 167, GTT is replaced by GTC
   At position 167, GTT is replaced by the weakly used GTC
   Globally the %G+C switch from 48.8288288288288 to 49.009009009009
   Locally the %G+C switch from 53.763440860215 to 54.8387096774194
   -> Change is REJECTED
At position 131, ACG is replaced by ACG : no change
At position 96, AGA is replaced by the weakly used AGG
   -> change is REJECTED : no more forbidden codons can be incorporated
At position 96, AGA is replaced by the weakly used AGA
   -> change is REJECTED : no more forbidden codons can be incorporated
At position 151, GCC is replaced by GCT
   At position 151, GCC is replaced by the weakly used GCT
   Globally the %G+C switch from 48.8288288288288 to 48.6486486486487
   Locally the %G+C switch from 56.989247311828 to 55.9139784946236
   -> Change is ACCEPTED
At position 98, ATT is replaced by ATT : no change
At position 10, AGG is replaced by AGG : no change
At position 5, GAC is replaced by GAC : no change
At position 133, ATA is replaced by ATA : no change
At position 61, CCT is replaced by CCC
   At position 61, CCT is replaced by the weakly used CCC
   Globally the %G+C switch from 48.6486486486487 to 48.8288288288288
   Locally the %G+C switch from 47.3118279569892 to 48.3870967741936
   -> Change is REJECTED
At position 61, CCT is replaced by CCT : no change
At position 67, AAC is replaced by AAC : no change At position 34, GGG is replaced by GGG : no change
At position 131, ACG is replaced by ACG : no change
At position 26, AGG is replaced by AGG : no change
At position 62, TAT is replaced by TAT : no change
At position 71, ATA is replaced by ATA : no change
At position 140, TCC is replaced by TCG
   At position 140, TCC is replaced by the weakly used TCG
   Globally the %G+C switch from 48.6486486486487 to 48.6486486486487
   Locally the %G+C switch from 44.0860215053763 to 44.0860215053763
   -> Change is ACCEPTED
At position 152, GGC is replaced by GGC : no change
At position 42, TTG is replaced by TTG : no change
At position 31, GAA is replaced by GAA : no change
At position 51, CCT is replaced by CCT : no change
At position 31, GAA is replaced by GAA : no change
At position 77, TAT is replaced by TAT : no change
At position 4, AAT is replaced by AAT : no change
At position 56, CAG is replaced by CAG : no change
At position 132, AAA is replaced by AAA : no change
At position 163, GAC is replaced by GAC : no change
At position 148, TAT is replaced by TAT : no change
At position 78, GTG is replaced by GTG : no change
At position 161, ACG is replaced by ACG : no change
At position 45, GTT is replaced by GTT : no change
At position 5, GAC is replaced by GAC : no change
At position 106, AGT is replaced by AGT : no change
At position 94, GGT is replaced by GGT : no change
At position 51, CCT is replaced by CCT : no change
At position 159, GTT is replaced by GTC
   At position 159, GTT is replaced by the weakly used GTC
   Globally the %G+C switch from 48.6486486486487 to 48.8288288288288
   Locally the %G+C switch from 52.6881720430108 to 53.763440860215
   -> Change is REJECTED
At position 93, CCT is replaced by CCT : no change
At position 17, TTG is replaced by TTG : no change
At position 74, GCA is replaced by GCA : no change
At position 117, GTG is replaced by GTG : no change
At position 161, ACG is replaced by ACG : no change
At position 148, TAT is replaced by TAT : no change
At position 83, GGT is replaced by GGT : no change
At position 7, GTG is replaced by GTG : no change
At position 9, TTG is replaced by TTA
   At position 9, TTG is replaced by the weakly used TTA
   Globally the %G+C switch from 48.6486486486487 to 48.4684684684685
   Locally the %G+C switch from 45.3333333333333 to 44
   -> Change is REJECTED
At position 151, GCT is replaced by GCC
   At position 151, GCT is replaced by the weakly used GCC
   Globally the %G+C switch from 48.6486486486487 to 48.8288288288288
   Locally the %G+C switch from 55.9139784946236 to 56.989247311828
   -> Change is REJECTED
At position 51, CCT is replaced by CCC
   At position 51, CCT is replaced by the weakly used CCC
   Globally the %G+C switch from 48.6486486486487 to 48.8288288288288
   Locally the %G+C switch from 49.4623655913978 to 50.5376344086022
   -> Change is REJECTED At position 1, ACG is replaced by ACG : no change
At position 68, GGT is replaced by GGC
   At position 68, GGT is replaced by the weakly used GGC
   Globally the %G+C switch from 48.6486486486487 to 48.8288288288288
   Locally the %G+C switch from 49.4623655913978 to 50.5376344086022
   -> Change is REJECTED
At position 104, AAC is replaced by AAC : no change
At position 156, CAG is replaced by CAG : no change
At position 13, ACG is replaced by ACG : no change
At position 13, ACG is replaced by ACG : no change
At position 72, GGC is replaced by GGC : no change
At position 26, AGG is replaced by AGG : no change
At position 91, ACG is replaced by ACG : no change
At position 10, AGG is replaced by AGG : no change
At position 4, AAT is replaced by AAT : no change
At position 74, GCA is replaced by GCG
   At position 74, GCA is replaced by the weakly used GCG
   Globally the %G+C switch from 48.6486486486487 to 48.8288288288288
   Locally the %G+C switch from 49.4623655913978 to 50.5376344086022
   -> Change is REJECTED
At position 158, ACG is replaced by ACG : no change
At position 179, ACT is replaced by ACT : no change
At position 104, AAC is replaced by AAC : no change
At position 56, CAG is replaced by CAG : no change
At position 107, CAG is replaced by CAG : no change
At position 17, TTG is replaced by TTG : no change
At position 2, AAT is replaced by AAT : no change
At position 104, AAC is replaced by AAC : no change
At position 112, CTT is replaced by the weakly used CTA
   -> change is REJECTED : no more forbidden codons can be incorporated
At position 112, CTT is replaced by the weakly used CTT
   -> change is REJECTED : no more forbidden codons can be incorporated
At position 112, CTT is replaced by the weakly used CTT
   -> change is REJECTED : no more forbidden codons can be incorporated
At position 151, GCT is replaced by GCT : no change
At position 128, CCA is replaced by CCG
   At position 128, CCA is replaced by the weakly used CCG
   Globally the %G+C switch from 48.6486486486487 to 48.8288288288288
   Locally the %G+C switch from 43.010752688172 to 44.0860215053763
   -> Change is REJECTED
At position 40, CCT is replaced by CCT : no change
At position 34, GGG is replaced by GGG : no change
At position 139, CCC is replaced by CCT
   At position 139, CCC is replaced by the weakly used CCT
   Globally the %G+C switch from 48.6486486486487 to 48.4684684684685
   Locally the %G+C switch from 43.010752688172 to 41.9354838709677
   -> Change is REJECTED
At position 177, GAA is replaced by GAA : no change
At position 100, CAA is replaced by CAA : no change
At position 165, CCT is replaced by CCC
   At position 165, CCT is replaced by the weakly used CCC
   Globally the %G+C switch from 48.6486486486487 to 48.8288288288288
   Locally the %G+C switch from 52.6881720430108 to 53.763440860215
   -> Change is REJECTED
At position 78, GTG is replaced by GTG : no change
At position 154, GAA is replaced by GAA : no change
At position 111, GGG is replaced by GGG : no change
At position 120, TTG is replaced by CTT
   At position 120, TTG is replaced by the weakly used CTT
   Globally the %G+C switch from 48.6486486486487 to 48.6486486486487
   Locally the %G+C switch from 45.1612903225806 to 45.1612903225806
   -> Change is ACCEPTED
At position 117, GTG is replaced by GTG : no change
At position 9, TTG is replaced by TTG : no change
At position 70, CCT is replaced by CCC
   At position 70, CCT is replaced by the weakly used CCC
   Globally the %G+C switch from 48.6486486486487 to 48.8288288288288
   Locally the %G+C switch from 48.3870967741936 to 49.4623655913978
   -> Change is REJECTED
At position 167, GTT is replaced by GTT : no change
At position 81, GGT is replaced by GGC
   At position 81, GGT is replaced by the weakly used GGC
   Globally the %G+C switch from 48.6486486486487 to 48.8288288288288
   Locally the %G+C switch from 51.6129032258064 to 52.6881720430108
   -> Change is REJECTED
At position 157, GGG is replaced by GGG : no change
At position 109, GGG is replaced by GGG : no change
At position 176, TTT is replaced by TTT : no change
At position 138, AGT is replaced by AGC
   At position 138, AGT is replaced by the weakly used AGC
   Globally the %G+C switch from 48.6486486486487 to 48.8288288288288
   Locally the %G+C switch from 43.010752688172 to 44.0860215053763
   -> Change is REJECTED
At position 69, GAA is replaced by GAA : no change
   At position 10, AGG is replaced by AGG : no change
At position 85, GGT is replaced by GGC
   At position 85, GGT is replaced by the weakly used GGC
   Globally the %G+C switch from 48.6486486486487 to 48.8288288288288
   Locally the %G+C switch from 50.5376344086022 to 51.6129032258064
   -> Change is REJECTED
At position 102, TTA is replaced by TTG
   At position 102, TTA is replaced by the weakly used TTG
   Globally the %G+C switch from 48.6486486486487 to 48.8288288288288.
   Locally the %G+C switch from 50.5376344086022 to 51.6129032258064
   -> Change is REJECTED
At position 107, CAG is replaced by CAG : no change
At position 152, GGC is replaced by GGT
   At position 152, GGC is replaced by the weakly used GGT
   Globally the %G+C switch from 48.6486486486487 to 48.4684684684685
   Locally the %G+C switch from 55.9139784946236 to 54.8387096774194
   -> Change is ACCEPTED
You can calculate statistics regarding AEP and REP by generating random synonymous sequences ?
   How many interations do you wish for these calculations ? (no more than 10000 is strongly recommended...)
   10000

| * | FINAL SEQUENCE | * |
|---|---|---|
| A synonymous sequence of maximum REP with respect to the initial sequence is : | | |
| ATGACGAATTCTAATGACAGCGTGACGTTGAGGTTGATGACGGAACACGATTTG GCCATGTTGTACGAATGGTTGAACAGGAGTCACATAGTGGAATGGTGGGGGG TGAGGAGGCTAGGCCTACTTTGGCAGATGTTCAAGAGCAATATCTTCCTTCTGTG CTGGCCCAGGAAAGTGTGACGCCTTATATAGCCATGCTTAACGGTGAACCTATA GGCTACGCACAAAGTTATGTGGCATTGGGTTCTGGTGATGGTTGGTGGGAGGA GGAGACGGACCCTGGTGTTAGAGGTATTGATCAACTGTTAGCTAACGCTAGTCA GCTTGGGAAGGGGCTTGGTACGAAATTAGTGAGAGCACTTGTGGAACTTTTGTT TAACGACCCAGAAGTGACGAAAATACAGACTGATCCCAGTCCCTCGAATCTTAG AGCCATAAGATGTTATGAAAAGGCTGGTTTCGAACGTCAGGGGACGGTTACGAC GCCTGACGGGCCTGCGGTTTATATGGTGCAGACTAGACAAGCTTTTGAAAGAAC TAGATCCGACGCATGA | | |

| * | FEATURES | * |
|---|---|---|
| %GC = 48.4684684684685 | | |
| Number of codon with different REP | : 119 | |
| Number of different best REP sequences | : 7.35251708795721 e+024 | |
| Similarity with the initial sequence | : 61.0810810810811 | |
| | | |
| initial sequence AEP | : 6.45901639344262 | |
| Alt. sequence AEP | : 6.54644808743169 | |
| Alt. sequence REP | : 1.6120218579235 | |
| | | |
| Stat. AEP | : 6.81420437158316 +/- 0.000327852458606665 | |
| Stat. REP | : 1.03825136612014 +/- 8.10462807976364e-014 | |
| Number of iterations | : 10000 | |

## Claims

1. A method for making a mutant polypeptide comprising :
- identifying an original nucleotide sequence which encodes a polypeptide;
- determining at least one synonymous nucleotide sequence encoding the same protein, which comprises at least one synonymous codon different from the corresponding codon in the original nucleotide sequence.
- synthesizing said synonymous polynucleotide sequence,
- transforming said synonymous polynucleotide sequence into a host cell,
- culturing said host cell under culture conditions specifically designed to positively link features of interest to cell fitness, e.g. in the presence of chemicals or antibiotics, thereby introducing at least one point mutation into said synonymous nucleotide sequence,
- isolating a mutant cell expressing a mutant polypeptide, and
- recovering said mutant polypeptide.

2. The method of claim 1, wherein at least one codon of the synonymous nucleotide sequence has a different evolutionary landscape from the corresponding codon in the original nucleotide sequence.

3. The method of claim 1 or 2, wherein at least one codon of the synonymous nucleotide sequence has a greater potential to mutate into a different amino acid by a single point mutation than the corresponding original codon.

4. The method of claim 1 or 2, wherein at least one codon of the synonymous nucleotide sequence has a lesser potential to mutate into a different amino acid by a single point mutation than the corresponding original codon.

5. The method of claim 1, comprising expressing the mutated synonymous nucleotide sequence and selecting a sequence encoding a polypeptide having a desired functional activity.

6. The method of claim 5, wherein said mutated synonymous nucleotide sequence is expressed in a host cell.

7. The method of claim 5 or 6, wherein a polypeptide having the functional activity of the polypeptide encoded by the original polynucleotide sequence is selected.

8. The method of claim 5 or 6, wherein a polypeptide having a lesser degree of the functional activity of the polypeptide encoded by the original polynucleotide is selected.

9. The method of claim 5 or 6, wherein a polypeptide having a greater degree of the functional activity of the polypeptide encoded by the original polynucleotide is selected.

10. The method of claims 5 to 9, wherein a polypeptide having a more stable functional activity than that of the polypeptide encoded by the original polynucleotide is selected.

11. The method of claims 1 to 10, which is a computer-implemented method.

12. The method of claims 1 to 11, which is performed using the Evolutionary Lanscape Painter program (ELP).

13. A computer-implemented method for selecting a nucleotide sequence which is synonymous to a known polynucleotide sequence, using the Evolutionary Lanscape Painter program (ELP), and comprising:
obtaining an original nucleotide sequence which encodes a polypeptide ;
determining synonymous nucleotides for each codon of the sequence;
determining the intrinsic evolutionary power of each synonymous codon;
selecting a synonymous nucleotide sequence having a higher or lower intrinsic evolutionary power than the original nucleotide sequence.

14. The method of claim 13, further comprising determining at least one alternative codon having a higher or lower GC content than the original codon.

15. The method of claim 13 or 14, further comprising the alternative sequences having the highest or lowest GC content.

16. A polynucleotide comprising the polynucleotide sequence of the dehydrofolate reductase gene dfrB1 as disclosed in figure 3, which has been modified to increase the intrinsic evolutionary power or relative evolutionary power, wherein said modified polynucleotide sequence has been determined by the Evolutionary Landscape Painter program and is dfr(G/C) as disclosed in figure 3.

17. A vector comprising the polynucleotide sequence of claim 16.

18. A host cell comprising the vector of claim 17.

## Patentansprüche

1. Verfahren zur Herstellung eines mutierten Polypeptids, welches umfasst:
- Identifizieren einer ursprünglichen Nukleotidsequenz, welche ein Polypeptid kodiert;
- Bestimmen mindestens einer synonymen Nukleotidsequenz, die das gleiche Protein kodiert, welche mindestens ein synonymes Codon umfasst, das sich von dem entsprechenden Codon in der ursprünglichen Nukleotidsequenz unterscheidet,
- Synthetisieren der genannten synonymen Polynukleotidsequenz,
- Einführen der genannten synonymen Polynukleotidsequenz in eine Wirtszelle,
- Kultivieren der genannten Wirtszelle unter Kulturbedingungen, welche speziell ausgelegt sind, um interessierende Merkmale mit Zellfitness positiv zu verknüpfen, z.B. angesichts Chemikalien oder Antibiotika, wodurch mindestens eine Punktmutation in die genannte synonyme Nukleotidsequenz eingeführt wird,
- Isolieren einer mutierten Zelle, welche ein mutiertes Polypeptid exprimiert, und
- Gewinnen des genannten mutierten Polypeptids.

2. Verfahren nach Anspruch 1, in dem mindestens ein Codon der synonymen Nukleotidsequenz eine unterschiedliche Evolutionslandschaft gegenüber dem entsprechenden Codon in der ursprünglichen Nukleotidsequenz aufweist.

3. Verfahren nach Anspruch 1 oder 2, in dem mindestens ein Codon der synonymen Nukleotidsequenz ein größeres Potential, zu einer unterschiedlichen Aminosäure zu mutieren, als das entsprechende ursprüngliche Codon, durch eine einzelne Punktmutation aufweist.

4. Verfahren nach Anspruch 1 oder 2, in dem mindestens ein Codon der synonymen Nukleotidsequenz ein geringeres Potential, zu einer unterschiedlichen Aminosäure zu mutieren, als das entsprechende ursprüngliche Codon, durch eine einzelne Punktmutation aufweist.

5. Verfahren nach Anspruch 1, welches das Exprimieren der mutierten synonymen Nukleotidsequenz und das Auswählen einer Sequenz umfasst, welche ein Polypeptid mit einer gewünschten funktionellen Aktivität kodiert.

6. Verfahren nach Anspruch 5, in dem die genannte mutierte synonyme Nukleotidsequenz in einer Wirtszelle exprimiert wird.

7. Verfahren nach Anspruch 5 oder 6, in dem ein Polypeptid mit der funktionellen Aktivität des Polypeptids, das durch die ursprüngliche Polynukleotidsequenz kodiert wird, ausgewählt wird.

8. Verfahren nach Anspruch 5 oder 6, in dem ein Polypeptid mit einem geringeren Ausmaß der funktionellen Aktivität als das Polypeptid, das durch das ursprüngliche Polynukleotid kodiert wird, ausgewählt wird.

9. Verfahren nach Anspruch 5 oder 6, in dem ein Polypeptid mit einem höheren Ausmaß der funktionellen Aktivität als das Polypeptid, das durch das ursprüngliche Polynukleotid kodiert wird, ausgewählt wird.

10. Verfahren nach den Ansprüchen 5 bis 9, in dem ein Polypeptid mit einer stabileren funktionellen Aktivität als das Polypeptid, das durch das ursprüngliche Polynukleotid kodiert wird, ausgewählt wird.

11. Verfahren nach den Ansprüchen 1 bis 10, welches ein Computer-implementiertes Verfahren ist.

12. Verfahren nach den Ansprüchen 1 bis 11, welches unter Verwendung des Evolutionary Landscape Painter-Programms (ELP) ausgeführt wird.

13. Computer-implementiertes Verfahren zum Auswählen einer Nukleotidsequenz, die zu einer bekannten Polynukleotidsequenz synonym ist, unter Verwendung des Evolutionary Landscape Painter-Programms (ELP) und umfassend:
Gewinnen einer ursprünglichen Nukleotidsequenz, welche ein Polypeptid kodiert;
Bestimmen der synonymen Nukleotiden für jedes Codon der Sequenz;
Bestimmen des intrinsischen Evolutionsvermögens jedes synonymen Codons;
Auswählen einer synonymen Nukleotidsequenz, die ein höheres oder geringeres intrinsisches Evolutionsvermögen als die ursprüngliche Nukleotidsequenz aufweist.

14. Verfahren nach Anspruch 13, welches ferner umfasst, mindestens ein alternatives Codon mit einem höheren oder geringeren GC-Gehalt als das ursprüngliche Codon zu bestimmen.

15. Verfahren nach Anspruch 13 oder 14, welches ferner die alternativen Sequenzen mit dem höchsten oder geringsten GC-Gehalt umfasst.

16. Polynukleotid, umfassend die Polynukleotidsequenz des Dehydrofolatreduktase-Gens dfrB1, wie in Figur 3 offenbart, welche modifiziert worden ist, um das intrinsische Evolutionsvermögen oder relative Evolutionsvermögen zu erhöhen, wobei die modifizierte Polynukleotidsequenz durch das Evolutionary Landscape Painter-Programm bestimmt worden ist und dfr(G/C), wie in Figur 3 offenbart, ist.

17. Vektor, welcher die Polynukleotidsequenz nach Anspruch 16 umfasst.

18. Wirtszelle, welche den Vektor nach Anspruch 17 umfasst.

## Revendications

1. Procédé pour obtenir un polypeptide mutant, comprenant :
- identifier une séquence de nucléotides d'origine qui code pour un polypeptide ;
- déterminer au moins une séquence de nucléotides synonyme codant pour la même protéine, qui comprend au moins un codon synonyme qui est différent du codon correspondant dans la séquence de nucléotides d'origine.
- synthétiser ladite séquence polynucléotidique synonyme,
- introduire ladite séquence polynucléotidique synonyme dans une cellule hôte,
- cultiver ladite cellule hôte dans des conditions de culture spécifiquement conçues pour positivement lier des caractéristiques d'intérêt à la vigueur cellulaire, par exemple en présence de produits chimiques ou d'antibiotiques, ce qui introduit ainsi au moins une mutation ponctuelle dans ladite séquence de nucléotides synonyme,
- isoler une cellule mutante exprimant un polypeptide mutant, et
- récupérer ledit polypeptide mutant.

2. Procédé selon la revendication 1, dans lequel au moins un codon de la séquence de nucléotides synonyme possède un schéma évolutionnaire différent de celui du codon correspondant dans la séquence de nucléotides d'origine.

3. Procédé selon la revendication 1 ou 2, dans lequel au moins un codon de la séquence de nucléotides synonyme a une plus grande chance de muter en un acide aminé différent, par une mutation ponctuelle unique, par rapport au codon d'origine correspondant.

4. Procédé selon la revendication 1 ou 2, dans lequel au moins un codon de la séquence de nucléotides synonyme a une chance plus faible de muter en un acide aminé différent, par une mutation ponctuelle unique, par rapport au codon d'origine correspondant.

5. Procédé selon la revendication 1, comprenant l'expression de la séquence de nucléotides synonyme mutée et la sélection d'une séquence codant pour un polypeptide ayant une activité fonctionnelle souhaitée.

6. Procédé selon la revendication 5, dans lequel ladite séquence de nucléotides synonyme mutée est exprimée dans une cellule hôte.

7. Procédé selon la revendication 5 ou 6, dans lequel un polypeptide ayant l'activité fonctionnelle du polypeptide codé par la séquence polynucléotidique d'origine est sélectionné.

8. Procédé selon la revendication 5 ou 6, dans lequel un polypeptide ayant un degré d'activité fonctionnelle moindre par rapport au polypeptide codé par le polynucléotide d'origine est sélectionné.

9. Procédé selon la revendication 5 ou 6, dans lequel un polypeptide ayant un degré d'activité fonctionnelle plus élevé par rapport au polypeptide codé par le polynucléotide d'origine est sélectionné.

10. Procédé selon les revendications 5 à 9, dans lequel un polypeptide ayant une activité fonctionnelle plus stable que celle du polypeptide codé par le polynucléotide d'origine est sélectionné.

11. Procédé selon les revendications 1 à 10, qui est un procédé implémenté par ordinateur.

12. Procédé selon les revendications 1 à 11, qui est réalisé en utilisant le programme Evolutionary Landscape Painter (ELP).

13. Procédé implémenté par ordinateur pour sélectionner une séquence de nucléotides qui est synonyme d'une séquence polynucléotidique connue, en utilisant le programme Evolutionary Landscape Painter (ELP), et comprenant :
obtenir une séquence de nucléotides d'origine qui code pour un polypeptide ;
déterminer des nucléotides synonymes pour chaque codon de la séquence ;
déterminer le pouvoir évolutionnaire intrinsèque de chaque codon synonyme ;
sélectionner une séquence de nucléotides synonyme ayant un pouvoir évolutionnaire intrinsèque plus élevé ou plus faible que celui de la séquence de nucléotides d'origine.

14. Procédé selon la revendication 13, comprenant en outre la détermination d'au moins un codon alternatif ayant une teneur en GC plus élevée ou plus faible par rapport au codon d'origine.

15. Procédé selon la revendication 13 ou 14, comprenant en outre les séquences alternatives ayant la teneur en GC la plus élevée ou la plus faible.

16. Polynucléotide comprenant la séquence polynucléotidique du gène de la dihydrofolate réductase dfrB1, tel que décrit sur la Figure 3, qui a été modifiée pour augmenter le pouvoir évolutionnaire intrinsèque ou le pouvoir évolutionnaire relatif, dans lequel ladite séquence polynucléotidique modifiée a été déterminée par le programme Evolutionary Landscape Painter et est dfr(G/C), telle que décrite sur la Figure 3.

17. Vecteur comprenant la séquence polynucléotidique de la revendication 16.

18. Cellule hôte comprenant le vecteur de la revendication 17.
